# EUROPEAN PATENT APPLICATION

(11) **EP 4 361 643 A1**
(43) Date of publication of application: **01.05.2024**
(21) Application number: 22828831.2
(22) Date of filing: 24.06.2022
(51) Int. Cl.: G01N 35/02, G01N 35/10, C12Q 1/6806, C12Q 1/686, G01N 35/00, G01N 35/04

(54) **AUTOMATED ANALYSIS SYSTEM USING INDIVIDUALLY OPERATED BIOLOGICAL DEVICES, ANALYSIS METHOD AND STORAGE MEDIUM**

(30) Priority: 24.06.2021 KR 20210082324; 21.07.2021 KR 20210096081; 21.07.2021 KR 20210096088; 30.08.2021 KR 20210115009
(71) Applicant: Seegene, Inc., Seoul 05548 (KR)
(72) Inventor: YOON, Su Mi, Seongnam-si Gyeonggi-do 13313 (KR); KIM, Jae Young, Seoul 07703 (KR); PARK, Sang Jong, Seoul 05059 (KR)
(74) Representative: Viering, Jentschura & Partner mbB Patent- und Rechtsanwälte
(86) International application number: PCT/KR2022/009053
(87) International publication number: WO 2022/270984

(57) **Abstract**

The present disclosure relates to a technology related to an automated analysis system using individually operated biological devices, an analysis method, and a storage medium, wherein the system and the method enable an analysis sample to be prepared and analyzed by operating together with an automated analysis system through the operative connection of stand-alone devices.

## Description

### Technical field

The present disclosure relates to an automated analysis system that operates like a full automation system through the operative connection of stand-alone devices and, more particularly, to an automated analysis system including a device for preparing analysis samples and a device for analyzing the samples which are individually operated, an analysis method, and a storage medium.

### Background technology of the invention

The PCR test for diagnosis of a disease performs a test process such as nucleic acid extraction, PCR setup, amplification reaction, and reaction analysis, and in this case, various devices for performing each step are used.

For example, nucleic acid extraction equipment for extracting nucleic acid from a specimen, a liquid dispensing device for preparing an analysis sample, a Real-time PCR device for performing amplification reaction and reaction analysis thereon, and the like may be used.

These various devices are used in a stand-alone form, and the user installs containers for reagents, specimens, and/or analysis samples for each test step in each device or moving containers from one device to another.

External contamination may occur when the container of the specimen, reagent, and/or analysis sample is mounted on each device or moved to another device by the user. Also, a human test error may occur by the user.

Various devices have been developed to solve this problem. One embodiment is a Full Automation System.

Currently, various full automation systems are used in large medical institutions and large inspection institutions. Representatively, there are Cobas 6800/Cobas 8800 of Roche, Panther/Panther Fusion of Hologic, Alinity of Abbott, and QlAsymphony of Qiagen.

These devices may operate some or all of various processes for the diagnostic test in an integrated device as one step to provide a result.

However, since the conventional full automation system is implemented as one system from the time when the device is developed, it is not possible to individually perform nucleic acid extraction, PCR setup, amplification reaction, reaction analysis, and the like.

In addition, the conventional full automation system is manufactured in a considerably large size in order to implement various processes for a diagnostic test in one system, and has a high purchase cost. Therefore, there is also an issue where it is difficult to develop various diagnostic reagents due to the problem that it cannot be introduced easily by small and medium-sized hospitals and small and medium-sized inspection institutions.

Since the conventional system has low equipment applicability, it is not easy to apply an additionally developed diagnostic reagent. In addition, even when a partial error occurs in the equipment, it may be difficult to use the entire equipment.

Further, in an unusual situation in which a large-scale diagnostic test occurs, it is necessary to operate each testing device individually for rapid testing, but the conventional system cannot use each part separately. Even if it could be used separately, it was impossible to remove and use the device separately.

When a diagnosis kit development companies and research institutes having conventional systems develop diagnosis reagents, development and experiments are performed using the entire system, so it is not easy for small-scale diagnosis kit development companies and research institutes to develop a reagent.

Even if proceeded in large-scale diagnostic kit development companies and research institutes, if a large number of devices are not installed, there is a problem that the development period of diagnostic reagents cannot be achieved in a short period of time.

### Summary of the invention

### Problems to be solved

An object of the present disclosure is to combine individual stand-alone biological devices for molecular diagnostic testing or research into a modular type and integrate the modular type into an automated analysis system.

Another object of the present disclosure is to integrate and operate individually operated biological devices as an integrated system, and easily and quickly disassemble the biological devices if necessary to be individually operated again.

Another object of the present disclosure is to integrate and operate devices as an automated analysis system while minimizing structural changes of biological devices.

Another object of the present disclosure is to allow biological devices that are integrally operated by an automated analysis system to use reagents of molecular diagnostic tests that have been used conventionally without any changes.

Another object of the present disclosure is to integrate and operate an automated analysis system through a modular combination of individually operated biological devices, and to enclose some or all of the devices, thereby improving user convenience and reducing use space.

Another object of the present disclosure is to provide an environment control means inside an enclosure so that a change in an environment in which a device is operated does not occur when at least one of biological devices that are operated individually enclosed.

Another object of the present disclosure is to form a defined passage through which a reaction vessel commonly used by biological devices is transported, so that the reaction vessel can be easily transported from one or more devices.

Another object of the present disclosure is to provide a robotic module for transporting a reaction vessel so that the reaction vessel can be easily transported through a defined passage between biological devices.

Another object of the present disclosure is to provide a combination device capable of combining two or more biological devices that are individually operated so that they can be integrated and operated into an automated analysis system.

Another object of the present disclosure is to perform a test using an existing diagnostic reagent without changing the diagnostic reagent even when individually operated biological devices are modularly combined and integrated into an automated analysis system.

Another object of the present disclosure is to combine the biological devices individually licensed with an automated analysis system so that the biological devices can be integrated and operated, and to operate the biological devices without requiring a new license for each device.

### Means for solving problems

To achieve these objects and other advantages and in accordance with the purpose of the disclosure, as embodied and broadly described herein, there is provided an automated analysis system including: a preparation device for preparing an analysis sample in a reaction vessel, the preparation device being a stand-alone device; an analysis device for analyzing the analysis sample prepared in the reaction vessel, the analysis device being a stand-alone device; a transport device for transporting the reaction vessel; and an enclosure, wherein at least one device selected from the group consisting of the analysis device and the transport device is located within the enclosure; wherein the preparation device and the enclosure comprise a defined passage through which the reaction vessel is transported, and the transport device transports the reaction vessel through the defined passage; wherein the stand-alone device can operate independently when separated from the automated analysis system.

In another aspect of the present disclosure, provided is an analysis method using an automated analysis system, the automated analysis system including a preparation device, an analysis device, a transport device, a control module and an enclosure, wherein the analysis method includes: controlling, by the control module, the transport device such that a reaction vessel containing an analysis sample is transported from the preparation device to the analysis device; the preparation device and the analysis device being stand-alone devices; controlling, by the control module, the analysis device such that the analysis sample is analyzed in the analysis device; and controlling, by the control module, the transport device such that the reaction vessel in which analysis of the analysis sample is completed is removed from the analysis device, wherein at least one device selected from the group consisting of the analysis device and the transport device is located inside an enclosure, the preparation device and the enclosure each include a defined passage through which the reaction vessel is transported, and the transport device transports the reaction vessel through the defined passage.

In accordance with another aspect of the present disclosure to achieve the object, there is provided an automated analysis system including: a memory; at least one processor configured to access the memory; and at least one program stored in the memory and configured to be executed by the processor, the automated analysis system including: a preparation device; an analysis device; a transport device; a control module; and an enclosure, the at least one program including instructions that, when executed by the at least one processor, cause the following steps to be performed: controlling, by the control module, the transport device such that a reaction vessel containing an analysis sample is transported from the preparation device to the analysis device; the preparation device and the analysis device being stand-alone devices; controlling, by the control module, the analysis device such that the analysis sample is analyzed in the analysis device; controlling, by the control module, the transport device such that the reaction vessel in which the analysis of the analysis sample is completed is removed from the analysis device; wherein at least one device selected from the group consisting of the analysis device and the transport device is located inside the enclosure, the preparation device and the enclosure each including a defined passage through which the reaction vessel is transported, the at least one program including instructions that cause the transport device to transport the reaction vessel through the defined passage.

In order to achieve another object of the present disclosure, there is provided a non-transitory computer-readable storage medium having instructions that, when executed by one or more processors, cause an analysis method using an automated analysis system to be performed, the automated analysis system including a preparation device, an analysis device, a transport device, a control module and an enclosure, the method including: controlling, by the control module, the transport device such that a reaction vessel containing an analysis sample is transported from the preparation device to the analysis device; the preparation device and the analysis device being stand-alone devices; controlling, by the control module, the analysis device such that the analysis sample is analyzed in the analysis device; and controlling, by the control module, the transport device such that the reaction vessel in which the analysis of the analysis sample is completed is removed from the analysis device, wherein at least one device selected from the group consisting of the analysis device and the transport device is located inside the enclosure, the preparation device and the enclosure each include a defined passage through which the reaction vessel is transported, and the instructions cause the transport device to transport the reaction vessel through the defined passage.

According to another aspect of the present disclosure, there is provided a method of controlling a fan module in an automated analysis system, wherein the automated analysis system includes: a preparation device for preparing an analysis sample in a reaction vessel; an analysis device for analyzing the analysis sample prepared in the reaction vessel; a transport device for transporting the reaction vessel; an enclosure; a gate part for opening and closing a defined passage; and a fan module that operates to discharge air out from an internal space of the enclosure; wherein at least one device selected from the group consisting of the analysis device and the transport device is located inside the enclosure, wherein the preparation unit and the enclosure each include a defined passage through which the reaction vessel is transported, and wherein the method of controlling the fan module includes: opening the defined passage by the gate part; and stopping an operation of the fan module while the defined passage is opened by the gate part.

To further achieve these and other advantages and in accordance with the purpose of the present disclosure, there is provided a method of manufacturing an assembly of a molecular diagnostic device, the assembly including a stand-alone analysis device, a transport device and a stand-alone preparation device, wherein the transport device transporting a reaction vessel; the stand-alone preparation device providing a reaction vessel including a sample analyzable by the stand-alone analysis device; the stand-alone analysis device analyzing the sample contained in the reaction vessel; the method including the following steps: (a) providing the stand-alone analysis device and the stand-alone preparation device to the transport device; and (b) aligning the stand-alone analysis device, the transport device and the stand-alone preparation device, wherein the aligning forms a movement pathway for the reaction vessel between the stand-alone preparation device and the stand-alone analysis device.

### Effects of the invention

The features and advantages of the present disclosure are summarized as follows.
(1) The automated analysis system, analysis method, and storage medium of the present disclosure may facilitate an operational connection between two devices by providing a transport device capable of providing a reaction vessel between a preparation device and an analysis device used individually for analysis of an analysis sample.
(2) The automated analysis system, analysis method, and storage medium according to the present disclosure are advantageous in that they can provide convenience of use wherein conventional stand-alone biological devices may be operatively connected to be used as an automated analysis system.
(3) The automated analysis system, analysis method, and storage medium of the present disclosure may be used by being integrated into an automated analysis system by operatively connecting a plurality of devices that have been used independently, and if necessary, may be disassembled again to be used independently.
(4) The automated analysis system, analysis method, and storage medium according to the present disclosure may combine biological devices separately used, but combine them without any change in the internal structure of the device, thus enabling the combined device to be used without requiring additional authorization or licensing.
(5) The automated analysis system, analysis method, and storage medium according to the present disclosure may be combined so as not to require additional approval or licensing for biological devices integrated into the automated analysis system, thus enabling each device to use a reagent without changing the conventional reagent.
(6) The automated analysis system, analysis method, and storage medium according to the present disclosure are advantageous in that reaction vessels used by biological devices combined with the automated analysis system can be provided to each device through a transport device, thereby preventing human test errors.
(7) The automated analysis system, analysis method, and storage medium of the present disclosure combine biological devices into an automated analysis system, and two or more devices are enclosed, thereby protecting analysis samples from contamination of the external environment.
(8) The automated analysis system, analysis method, and storage medium of the present disclosure may prevent the reaction vessel from being brought into contact with external contamination by enclosing at least one of the analysis device and the transport device.

### Brief description of drawings

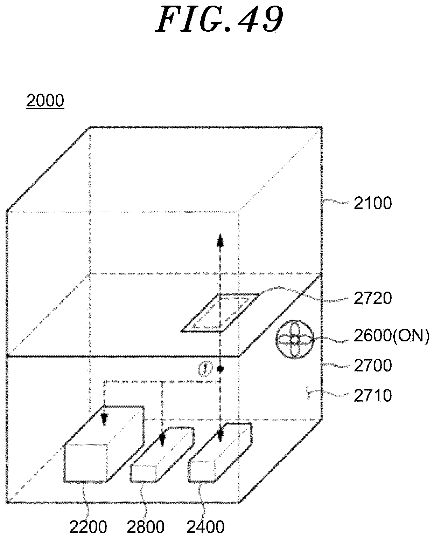

FIG. 1 is a front view illustrating an automated analysis system according to an embodiment of the present disclosure.
FIG. 2 is a right-side view illustrating an automated analysis system according to an embodiment of the present disclosure.
FIG. 3 is an exemplary diagram illustrating an operational connection of the automated analysis system according to an embodiment of the present disclosure.
FIG. 4 is an internal front view illustrating an automated analysis system according to an embodiment of the present disclosure.
FIG. 5 is a perspective view illustrating a stand-alone preparation device according to an embodiment of the present disclosure.
FIG. 6 is a perspective view illustrating an enclosure according to an embodiment of the present disclosure.
FIG. 7 is an exemplary diagram for illustrating an operating state of a defined passage of an enclosure according to an embodiment of the present disclosure.
FIG. 8 is an internal perspective view illustrating an enclosure of the automated analysis system according to an embodiment of the present disclosure.
FIG. 9 is a perspective view illustrating a transport device according to an embodiment of the present disclosure.
FIG. 10 is a perspective view illustrating a lift module of the transport device according to an embodiment of the present disclosure.
FIG. 11 is a perspective view illustrating a crane module of the transport device according to an embodiment of the present disclosure.
FIG. 12 is a perspective view illustrating a rotation component of a crane module according to an embodiment of the present disclosure.
FIG. 13 is a perspective view illustrating a stand-alone analysis device according to an embodiment of the present disclosure.
FIG. 14 is a perspective view illustrating an automatic sealer according to an embodiment if the present disclosure.
FIG. 15 is an exemplary diagram illustrating a conveyor for recovering a reaction vessel according to an embodiment of the present disclosure.
FIGS. 16A and 16B are perspective views illustrating a solution collection bin according to an embodiment of the present disclosure.
FIG. 17 is a first exemplary view illustrating a horizontal extension movement of a lift module in an enclosure according to an embodiment of the present disclosure.
FIG. 18 is a second exemplary view illustrating a horizontal extension movement of a lift module in an enclosure according to an embodiment of the present disclosure.
FIG. 19 is a first exemplary view illustrating transport of a reaction vessel of a lift module and a crane module according to an embodiment of the present disclosure.
FIG. 20 is a second exemplary view illustrating transport of a reaction vessel of a lift module and a crane module according to an embodiment of the present disclosure.
FIG. 21 is an exemplary diagram illustrating an operation of mounting a reaction vessel on an automatic sealer according to an embodiment of the present disclosure.
FIG. 22 is an exemplary diagram illustrating an operation of mounting a reaction vessel on an analysis device according to an embodiment of the present disclosure.
FIG. 23 is an exemplary diagram illustrating an operation of a lift module according to an embodiment of the present disclosure.
FIG. 24 is a perspective view illustrating a horizontal extension movement of a lift module in a preparation device according to an embodiment of the present disclosure.
FIG. 25 is an exemplary view illustrating a crane module according to an embodiment of the present disclosure transporting a reaction vessel in the lift module.
FIG. 26 is an exemplary view illustrating a crane module according to an embodiment of the present disclosure mounting a reaction vessel on an automatic sealer.
FIG. 27 is an exemplary view illustrating a crane module according to an embodiment of the present disclosure mounting a reaction vessel on an analysis device.
FIG. 28 is a first exemplary view illustrating an operation of recovering a reaction vessel using a conveyor according to an embodiment of the present disclosure.
FIG. 29 is a second exemplary view illustrating an operation of recovering a reaction vessel using a conveyor according to an embodiment of the present disclosure.
FIG. 30 is an internal plan view illustrating the inside of an enclosure according to an embodiment of the present disclosure.
FIG. 31 is an internal perspective view illustrating the inside of an enclosure according to an embodiment of the present disclosure.
FIG. 32 is an exemplary diagram illustrating an analysis device being mounted in an enclosure using a positioning means according to an embodiment of the present disclosure.
FIG. 33 is a first exemplary diagram illustrating a positioning means according to an embodiment of the present disclosure.
FIG. 34 is a second exemplary diagram illustrating a positioning means according to an embodiment of the present disclosure.
FIG. 35 illustrates a fan module which is an environment control means inside an enclosure according to an embodiment of the present disclosure.
FIG. 36A illustrates a stand-alone preparation device according to an embodiment of the present disclosure provided in a transport unit, FIG. 36B is a view for describing the stand-alone preparation device being aligned with the transport unit, and FIG. 36C is a view illustrating the internal structure of a housing of the stand-alone preparation device.
FIG. 37A illustrates a stand-alone analysis device according to an embodiment of the present disclosure. FIG. 37B illustrates the stand-alone analysis device according to an embodiment of the present disclosure provided inside an enclosure of a transport unit.
FIG. 38 is a layout diagram illustrating components of a preparation device according to an embodiment of the present disclosure located on a deck.
FIG. 39 is a flowchart for illustrating an operating method of an automated analysis system according to an embodiment of the present disclosure.
FIG. 40 is a conceptual diagram conceptually illustrating a configuration of an automated analysis system according to an embodiment of the present disclosure.
FIG. 41 is a block diagram conceptually illustrating a configuration of a HW part of a preparation device.
FIG. 42 is a block diagram conceptually illustrating a configuration of a preparation device managing unit included in a preparation device.
FIG. 43 is a block diagram conceptually illustrating a configuration of a HW part of an analysis device.
FIG. 44 is a block diagram conceptually illustrating a configuration of an analysis device managing unit included in an analysis device.
FIG. 45 exemplarily illustrates a path through which a reaction vessel is transported by a transport device in an automated analysis system according to an embodiment.
FIG. 46 is a block diagram conceptually illustrating a configuration of a transport module.
FIG. 47 is a block diagram conceptually illustrating a configuration of an automatic sealer.
FIG. 48 is a view exemplarily illustrating a movement direction of a transport device in an automated analysis system according to an embodiment.
In each of FIGS. 49 through 56, a concept of an operation of a fan module being controlled according to a position or a moving direction of a transport device, or according to whether the passthrough cavity is opened or closed in an automated analysis system according to an embodiment, is exemplarily illustrated.

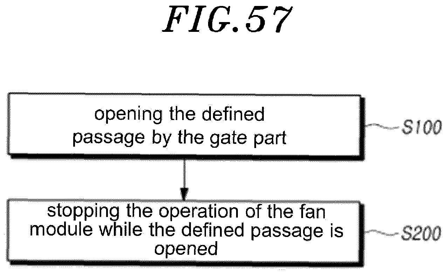

FIG. 57 is a flowchart of a fan module control method that may be performed by an automation analysis system according to an embodiment.

### Detailed description of the Invention

Hereinafter, the present disclosure will be described in more detail with reference to the embodiments of the present disclosure. These embodiments are provided only to describe the present disclosure in more detail, and it will be apparent to those skilled in the art that the scope of the present disclosure is not limited by these embodiments according to the gist of the present disclosure.

In addition, terms such as first, second, A, B, (a), (b), (i), and (ii) may be used in describing the elements of the present disclosure. The term is used only to distinguish the elements from other elements, and the nature, sequence, or order of the corresponding elements is not limited by the term. When an element is described as being "connected", "coupled", or "joined" to another element, the element may be directly connected or joined to the other element, but it should be understood that other elements may be "connected", "coupled", or "joined" between the respective elements.

### Automated analysis system

The present inventors have made intensive efforts to develop a system, a method, and the like, which intend to interwork and use various devices performing a processor for detecting a target nucleic acid by amplifying the target nucleic acid. As a result, the present inventors have configured such that a preparation device and an analysis device, which are individually operated stand-alone biological devices used to detect a target nucleic acid, can be operatively connected and used. That is, an automated analysis system configured to provide reaction vessels used in a preparation device and an analysis device, which were operated independently, from the preparation device to the analysis device using a transport device was developed.

As used herein, the term "automated analysis system" includes a preparation device for preparing an analysis sample including or presumed to include an analyte, and an analysis device for amplifying a nucleic acid having a specific nucleotide sequence in the prepared analysis sample and detecting the amplified nucleic acid. The preparation device and the analysis device are stand-alone devices that are individually operated. The preparation device and the analysis device can be applied to the automated analysis system in a state licensed as a stand-alone device. Alternatively, for application to an automated analysis system, some housings may be changed, and partial licensing may be performed.

Therefore, the preparation device and the analysis device are used in the stand-alone device, and the approved and licensed reagent may be used as it is.

The automated analysis system includes one or more defined passages and transport devices for operatively connecting the preparation device and analysis device, which are each operative. The defined passage may be a passthrough cavity.

As used herein, the term "sample" refers to a material including or presumed to include an analyte.

A "sample" includes biological samples (e.g., cells, tissues, and body fluids from biological sources) and non-biological samples (e.g., food, water, and soil).

The biological sample may include, but is not limited to, viruses, bacteria, tissues, cells, blood (including whole blood, plasma, and serum), lymph, bone marrow fluid, sputum, swab, aspiration, bronchial lavage fluid, bronchial alveolar lavage fluid, nasal lavage fluid, milk, urine, feces, ocular fluid, saliva, semen, brain extract, cerebrospinal fluid (SCF), arthroid fluid, appendage, spleen and tonsil tissue extract, amniotic fluid, and ascites.

The sample may also include naturally occurring nucleic acid molecules and synthetic nucleic acid molecules isolated from a biological source.

In one embodiment, the term "sample" may include a substance used for the preservation, processing, detection, and the like of the sample. The "sample" may include an amplification reagent, a detection reagent, a preservative, water, deionized water, a saline solution, a pH buffer, an acidic solution, and an additional material such as a basic solution, but is not limited thereto.

The term "raw sample" used herein may be used to refer to a sample before it is used in a sample processing device for analysis.

The term "raw sample" used herein may be used to indicate a sample before being processed in a preparation device for sample preparation.

The term "analysis sample" used herein may be used to refer to intermediates including analytes prepared in the course of several steps of processing the sample for analysis.

It may be used to indicate samples prepared in the process of being processed in a preparation device and an analysis device for sample preparation. In particular, it can be used to represent a sample analyzed in an analysis device.

As used herein, the term "specimen" may refer to an object to be analyzed that is collected from food, soil, air, water, or living organisms. In general, a specimen includes a sputum, blood, urine, stool, etc. A specimen container including a specimen may include a specimen collection composition for collecting a specimen and/or a specimen transport medium. The medium for transporting a sample performs a deactivation function due to lysis of an infectious pathogen and a stabilization function of nucleic acid materials released from the disrupted pathogen.

The term "specimen" may be interchangeably used with the term "sample". In particular, in one embodiment, the analyte is an antigen, antibody, enzyme or nucleic acid.

In a specific embodiment, the analyte is a nucleic acid. When the analyte to be analyzed herein is a nucleic acid molecule, the nucleic acid may be extracted from a specimen through a nucleic acid extraction process known in the art (See: Sambrook, J. et al., Molecular Cloning. A Laboratory Manual, 3rd ed. Cold Spring Harbor Press (2001)). The nucleic acid extraction process may vary depending on the type of specimen. In addition, when the extracted nucleic acid is RNA, a reverse transcription process for synthesizing cDNA may be additionally performed (See: Sambrook, J. et al., Molecular Cloning. A Laboratory Manual, 3rd ed. Cold Spring Harbor Press (2001)).

One or more types of analytes may be included in the sample, and a plurality of analysis samples for detection may be prepared for detection thereof.

As used herein, the term "preparation device" refers to a device used to prepare an analysis sample. The preparation device is a stand-alone device that is individually operated.

In one embodiment of the disclosure, the preparation device includes a housing, and the preparation device may be located within a closed enclosure in the form of a separate hexahedron.

The preparation device may provide the reaction vessel therein to the analysis device.

In one embodiment of the present disclosure, the preparation device may provide an internal reaction vessel to the analysis device through one or more passthrough cavity formed in the housing.

In another embodiment of the present disclosure, the preparation device may form one or more defined passages (first defined passage) in the housing in order to provide the reaction vessel therein to the analysis device. The defined passage may be a passthrough cavity.

In another embodiment of the present disclosure, when the preparation device is located inside the enclosure and the analysis device is located outside the enclosure, the enclosure may be formed with one or more defined passages to provide the reaction vessel of the preparation device to the analysis device.

As used herein, the term "preparation device" refers to a device for preparing an analysis sample that includes or is presumed to include an analyte.

The preparation device automatically performs a sample preparation process used in detecting an analyte (for example, a target nucleotide sequence) using a micro robot, wherein the sample preparation process in the present disclosure includes a process of extracting nucleic acids from a specimen, preparing a reaction mixture for amplification (for example, a reaction solution for polymerase chain reaction (PCR)), and preparing a detection sample in which the reaction mixture and the extracted nucleic acid are mixed.

When the preparation device does not include a nucleic acid extraction module, nucleic acid extraction from the specimen may be performed using a separate device.

Nucleic acid extraction may be performed using a nucleic acid extraction module to extract nucleic acid from a specimen when the analyte to be analyzed herein is a nucleic acid.

That is, when the preparation work to be performed in the preparation device is the preparation of a nucleic acid extract, actions that consequently lead to collecting separated nucleic acids should be performed through a series of actions for separation and purification of nucleic acids, starting from actions such as fractionating a specimen in a container in which the specimen is accommodated, dispensing a solution for cell lysis into the fractionated specimen, heating, and the like. The preparation of the nucleic acid extract may be performed through a nucleic acid extraction module included in the preparation device.

According to an embodiment of the present disclosure, a magnetic bead-based method using a magnetic bead capable of binding to a nucleic acid and eluting the bound nucleic acid is frequently used in a process of extracting nucleic acids from a specimen. The magnetic bead-based automated nucleic acid extraction method may be divided into a liquid transfer method and a bead transfer method according to the type of a process of eluting nucleic acids bound to magnetic beads.

In addition, the preparation device may perform a task of preparing a reaction mixture for nucleic acid amplification. In one embodiment of the present disclosure, the preparation device may simultaneously perform a preparation operation of a nucleic acid extract and a preparation operation of a reaction mixture for nucleic acid amplification.

The preparation device performs a sample preparation process, including the nucleic acid extraction from a specimen, the preparation of a reaction for amplification, and the preparation of a reaction micture in which they are mixed. The preparation process of the sample in the preparation device is implemented through a control device (not shown) for controlling the preparation device, and an operation of the preparation process of each sample is performed by the control device performing control of each component.

The control device may be configured to be embedded in the preparation device, and may be provided as a separate device and connected to the preparation device through a network.

The control device according to the present disclosure is a software control type. The control method of the preparation device may be controlled by software. Methods implemented as software or algorithms may be stored on a computer-readable recording medium as computer-readable codes or program instructions executable on a processor.

Examples of the computer-readable recording medium include a magnetic storage medium (e.g., read-only memory (ROM), random access memory (RAM), floppy disk, hard disk, etc.) and an optical reading medium (e.g., CD-ROM, Digital Versatile Disc (DVD), etc.). The computer-readable recording medium may be distributed over network coupled computer systems so that the computer-readable code is stored and executed in a distributed fashion. The medium may be readable by a computer, stored in a memory, and executed by a processor.

The preparation device according to the disclosure is an automated liquid handling device. The automated liquid handling device can automatically and programmatically aspirate and/or dispense a desired amount of reagent, sample, or other liquid from a designated container for automation of a chemical or biochemical laboratory. Various components of automated liquid handling devices are known to skilled people in the art.

All components of the preparation device are designed as an integrated device and located within the system housing.

In an embodiment of the present disclosure, the preparation device may use products such as "Microlab VANTAGE", "Microlab STAR", "Microlab NIMBUS", or "Microlab Prep" of Hamilton Company (see https://www.hamiltoncompany.com/automated-liquid-handling/platforms).

As used herein, the term "analysis device" refers to a device used for qualitative or quantitative analysis of an analyte.

Sample analysis includes detecting the presence of an analyte and measuring the content.

An analysis device may refer to a device that amplifies a nucleic acid having a specific nucleotide sequence and detects the amplified nucleic acid.

The analysis device may include an optical device including a light source and a photodetector.

The analysis device may include a device capable of heating or cooling the analysis sample through temperature control.

The analysis device may include a nucleic acid amplifier performing a nucleic acid amplification reaction through temperature control. Nucleic acid amplifiers typically include thermal cyclers.

In an analysis device, nucleic acid may be amplified in various ways. It may be performed by ligase chain reaction (LCR) (see Wiedmann M, et al., "Ligase chain reaction (LCR)- overview and applications." PCR Methods and Applications 1994 Feb;3(4):S51-64), gap filling LCR (GLCR) (see WO 90/01069, EP 439182 and WO 93/00447), Qbeta replicase amplification (Q-beta) (see Cahill P, et al., Clin Chem., 37(9): 1482-5(1991), U.S Pat. No. 5,556,751), strand displacement amplification (SDA) (See G T Walker et al., Nucleic Acids Res. 20(7):16911696(1992), EP 497272), nucleic acid sequence-based amplification (NASBA) (See Compton, J. Nature 350(6313):912(1991)), transcription-mediated amplification (TMA) (See Hofmann WP et al., J Clin Virol. 32(4):289-93(2005); U.S Pat. No. 5,888,779), and rolling circle amplification (RCA) (See Hutchison C.A. et al., Proc. Natl Acad. Sci. USA. 102:1733217336(2005)).

A thermal cycler, which is a nucleic acid amplification device included in the analysis device of the present disclosure, is usefully used in a polymerase chain reaction (PCR)-based nucleic acid amplification reaction. Various nucleic acid amplification methods based on polymerase chain reaction (PCR) are known. For example, quantitative PCR (quantitative PCR), digital PCR, asymmetric PCR, reverse transcription PCR (RT-PCR), Differential Display PCR (DDPCR), nested PCR, optional priming PCR (AP-PCR), multiplex PCR, SNP genome typing PCR, etc. are included.

For example, a thermal cycler which is the nucleic acid amplification device of the present disclosure, may perform a denaturing step, an annealing step, and an extension (or amplification) step in order to amplify deoxyribonucleic acid (DNA) having a specific nucleotide sequence.

The denaturation step is a step of separating the double-stranded DNA into single-stranded DNA by heating a solution containing a reagent and a sample containing the double-stranded DNA which is a template nucleic acid to a specific temperature, for example, about 95°C. The annealing step is a step of providing an oligonucleotide primer having a nucleotide sequence complementary to a nucleotide sequence of a nucleic acid to be amplified, cooling the isolated single-stranded DNA to a specific temperature, for example, 60°C., and binding the primer to a specific nucleotide sequence of the single-stranded DNA to form a partial DNA-primer complex. The extension step carries out a step of maintaining the solution at a specific temperature, for example 72°C., after the annealing step to form double-stranded DNA based on the primers of the partial DNA-primer complex by DNA polymerase.

In one embodiment, the analysis device of the present disclosure may exponentially amplify the DNA having the specific nucleotide sequence by repeating the above-described three steps, for example, 10 to 50 times.

In another embodiment, the analysis device of the present disclosure may simultaneously perform the annealing step and the extension step. In this case, the analysis device may complete the first circulation by performing two steps consisting of a denaturation step and an annealing/extension step.

Meanwhile, a nucleic acid detecting device included in the analysis device of the present disclosure is a device for detecting a target nucleic acid in a sample on which polymerase chain reaction (PCR) is performed through a nucleic acid amplification device, and includes an optical module for detecting emission light emitted from a fluorescent material in the target nucleic acid.

The optical module is an optics mechanism that analyzes (or monitors) in real time the amplification reactions performed in the nucleic acid amplification device. As an embodiment, the optical module may include a plurality of components such as a light source, an optical filter, a convex lens, a beam splitter, and a photodetector, and may detect fluorescence generated in a nucleic acid amplification reaction performed in the optical module in real time.

According to an embodiment of the present disclosure, the analysis device is a real-time detection device.

According to an embodiment of the present disclosure, the analysis device is a real-time nucleic acid detection device.

According to an embodiment of the present disclosure, the analysis device is a real-time PCR device.

The term "analysis device" used herein refers to a device for analyzing an analysis sample. The analysis device is a stand-alone device that is individually operated.

In one embodiment of the disclosure, the analysis device includes a housing for the analysis device, and the analysis device may be located in a closed enclosure in the form of a separate hexahedron.

The analysis device may receive a reaction vessel including an analysis sample from the preparation device.

The analysis device does not receive the reaction vessel directly from the preparation device, but may receive the reaction vessel transported from the transport device.

As used herein, the term "enclosure" is a structure in the form of a housing formed from one or more closed spaces that can be environmentally/spatially separated from the outside.

In one embodiment of the present disclosure, the enclosure may have a hexahedron shape.

In another embodiment of the present disclosure, the enclosure may have a shape in which a plurality of hexahedrons is connected. In this case, the inside may be connected to one space or may be separated into a plurality of spaces.

In an embodiment of the present disclosure, when the preparation device is located outside the enclosure and the analysis device is located inside, the transport device may be located inside the enclosure.

In another embodiment of the present disclosure, when the analysis device is located outside the enclosure and the preparation device is located inside, the transport device may be located outside the enclosure.

In another embodiment of the present disclosure, when the preparation device and the analysis device are located inside the enclosure, the transport device may be located inside the enclosure.

An automatic sealer may be positioned in the enclosure.

When the preparation device and/or the analysis device are located inside the enclosure, an environment control means may be included therein to implement an environment in which each device operates as a stand-alone device.

The environment control means is for controlling the temperature, humidity, contamination, etc. inside the enclosure, and may include a heating device, a cooling device, a humidity control device, a fan module, a filter, etc.

As used herein, the term "defined passage" is a configuration for connecting an environmentally/spatially separated preparation device and an analysis device. The defined passage is a passage through which a reaction vessel prepared by the preparation device is moved to the analysis device or the analysis device in the enclosure so as to spatially connect the preparation device and the analysis device which are located adjacent to each other.

In one embodiment of the present disclosure, the defined passage may be a passthrough cavity.

In an embodiment of the present disclosure, the defined passage may include a first defined passage formed in the preparation device and a second defined passage included in the enclosure.

In another embodiment of the disclosure, the first defined passage and/or the second defined passage may include a door device capable of blocking spatially connected states. The door device may be included in the first defined passage or may be included in the second defined passage. Alternatively, it may be included in both the first defined passage and the second defined passage.

The defined passage may be opened when the reaction vessel prepared in the preparation device is moved to the analysis device or the analysis device in the enclosure, and may be operated to be closed after the movement is completed.

The first defined passage is formed in the lower portion of the preparation device, and preferably may be formed in a deck of the preparation device. The first defined passage formed in the deck of the preparation device is located at the lower portion to provide the reaction vessel to the analysis device or the analysis device located in the enclosure.

The second defined passage may be formed on an upper surface, a side surface, or a bottom surface of the enclosure so that the reaction vessel moving from the first defined passage of the preparation device can enter.

In addition, the enclosure may include a transport device for transporting the reaction vessel from the first defined passage to the second defined passage.

As used herein, the term "transport device" refers to a device that can transport a reaction vessel used in an automated analysis system from a preparation device to an analysis device or an analysis device in an enclosure, and transport and mount the reaction vessel to each component in the enclosure.

In an embodiment of the present disclosure, the transport device for moving the reaction vessel from the preparation device to the analysis device may include at least one robotic module.

In one embodiment of the present disclosure, the robotic module includes a lift module. The lift module is a robotic module for moving the reaction vessel up and down, and may move the reaction vessel of the preparation device into the enclosure.

In another embodiment of the disclosure, the robotic module includes a crane module. The crane module can transport and mount the reaction vessel moved into the enclosure to the components within the enclosure.

In another embodiment of the disclosure, the robotic module includes robot arms. The robot arms may move the reaction vessel to a desired position via one or more joint motions.

The robotic module may be operated in up/down, front/rear, left/right directions, but in an embodiment of the present disclosure, the lift module may be operated in up/down and left/right directions, and the crane module may be operated in up/down, front/rear, left/right directions, and rotate.

As used herein, the term "vessel" refers to a space for accommodating a material used in a preparation device and an analysis device. The material generally includes a solution. The vessel may be used as a "sample vessel" or "reaction vessel" containing the analysis sample. In addition, in the present specification, a space accommodating a material used in the preparation device and the analysis device may be used as "container" or "carrier". "Vessel", "container", and "carrier" are not specifically distinguished. However, it may be selectively used depending on the device, shape, or internal receiving material used.

Also, the container refers to a container used for nucleic acid extraction, amplification reaction solution composition, and amplification reaction setup (for example, PCR setup) performed in a preparation device. That is, the specimen, the at least one extraction reagent, the at least one composition for the reaction solution, the master mix of the extracted nucleic acid and the reaction solution, and the like may be accommodated in the container, and the analysis sample in which the reaction is to be performed through the analysis device may be dispensed into the reaction vessel and accommodated therein. In one embodiment of the disclosure the container includes a tube, a tube strip and the like. In another embodiment of the disclosure the container may include a cartridge, a well plate, or the like.

The container may have various sizes according to the materials to be accommodated therein, and various means for storing or containing the container may be prepared according to containers having various sizes. The means for containing the containers may include a carrier, a rack, an adapter, and the like, and one or more containers may be inserted and stored in each means.

In one embodiment of the disclosure, the container may include a cap. In another embodiment of the present disclosure, the container may be sealed using a film or the like.

As used herein, the term "reaction vessel" is a sample vessel that can be received in a sample holder of an analysis device. The reaction vessel may contain a predetermined volume of an analysis sample containing a target nucleic acid or a predetermined volume of an analysis sample not containing a target nucleic acid, and be contained in a sample holder to be used for reaction (e.g., amplification) or detection (e.g., fluorescence signal).

The reaction vessel described in the present specification describes a tube capable of accommodating an analysis sample as an example, but reaction vessels of various materials and shapes may be used according to the shape of the reaction region. The reaction vessel is inserted into a well formed in the reaction region so that a reaction cycle of heating and cooling can be performed. That is, the "reaction vessel" refers to a closed space in which the reaction is performed.

The term reaction vessel may be understood to include one or two or more reaction vessels. A reaction vessel refers to a unit capable of receiving an analysis sample (e.g., an analyte or reaction mixture). Each of the test tube, amplification tube, strip tube, well plate, multi well PCR plate is one embodiment of a reaction vessel comprising one or two or more.

In one embodiment of the disclosure, one or more reaction vessels may be mounted on the sample holder.

In another embodiment of the present disclosure, one or more reaction vessels are placed in a multi-well plate (hereinafter referred to as a "well plate"). A well plate containing one or more reaction vessels may be mounted on the sample holder.

In another embodiment of the present disclosure, the reaction vessel is a well plate capable of receiving the analysis sample in one or more wells. A well plate that received the analysis sample in one or more wells may be mounted on the sample holder.

The embodiments of the reaction vessel describe some embodiments among preferred embodiments to be implemented in the present disclosure. Therefore, it is obvious that the reaction vessel may be variously implemented according to other embodiments.

The term "reaction mixture" used herein may refer to a solution mixed with an analyte to facilitate detection of the analyte. The reaction mixture may include one or more reaction reagents for amplification.

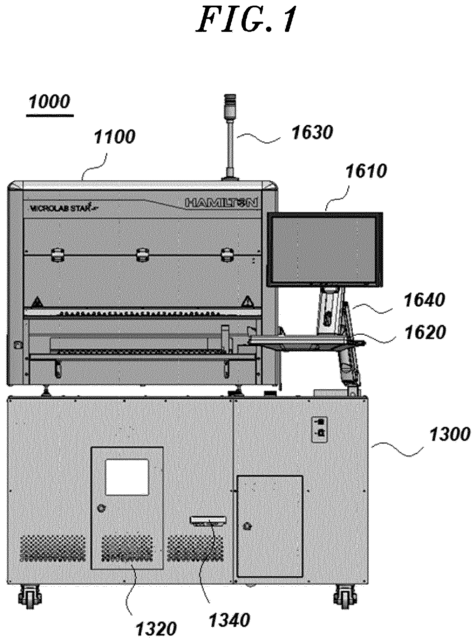

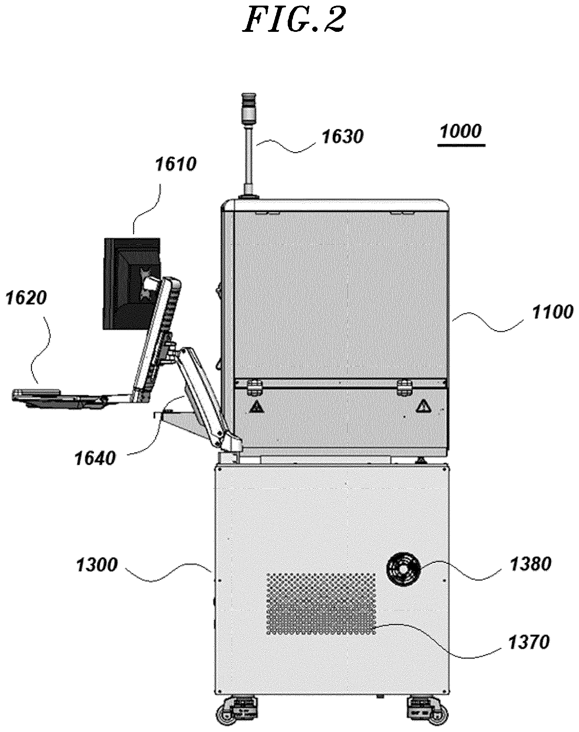

FIG. 1 is a front view illustrating an automated analysis system according to the present disclosure, and FIG. 2 is a right-side view illustrating an automated analysis system according to the present disclosure. As shown in FIGS. 1 and 2, in one embodiment of the present disclosure, an automated analysis system 1000 includes a preparation device 1100 and an enclosure 1300.

In an embodiment of the present disclosure, the preparation device 1100 may be configured to be located on top of the enclosure 1300.

In another embodiment of the present disclosure, the preparation device 1100 may be configured to be located on the side of the enclosure 1300.

In another embodiment of the present disclosure, the preparation device 1100 may be configured to be located on the rear surface of the enclosure 1300.

In another embodiment of the present disclosure, the preparation device 1100 may be configured to be located on the front surface of the enclosure 1300.

In another embodiment of the present disclosure, the preparation device 1100 may be configured to be located inside the enclosure 1300.

In another embodiment of the present disclosure, the preparation device 1100 may be configured to be located under the enclosure 1300.

The enclosure 1300 is a space having a closed hexahedron shape.

The enclosure 1300 may accommodate at least one of the preparation device 1100, the analysis device 1200, and the transport device 1400.

In an embodiment of the disclosure, the enclosure 1300 may accommodate one or more of the analysis device 1200 and the transport device 1400.

In another embodiment of the present disclosure, the enclosure 1300 may accommodate one or more analysis devices 1200.

In another embodiment of the present disclosure, the enclosure 1300 may accommodate the transport device 1400.

In yet another embodiment of the present disclosure, the enclosure 1300 may accommodate the preparation device 1100 and the transport device 1400.

In another embodiment of the present disclosure, the enclosure 1300 may accommodate the preparation device 1100.

The enclosure 1300 may provide an operative connection between one or more devices accommodated therein and devices located externally.

In an embodiment of the disclosure, the enclosure 1300 encloses a single space. When the enclosure 1300 encloses a single space, at least one of the preparation device 1100, the analysis device 1200, and the transport device 1400 may be located in a single space.

In another embodiment of the present disclosure, an enclosure 1300 encloses a plurality of spaces. When the enclosure 1300 encloses a plurality of spaces, at least one of the preparation device 1100, the analysis device 1200, and the transport device 1400 may be located in any one of the plurality of spaces or in at least two of the plurality of spaces.

For example, in the enclosure 1300 having a single space, the analysis device 1200 and the transport device 1400 may be located in the same space.

For example, in the enclosure 1300 having a plurality of spaces, the analysis device 1200 and the transport device 1400 may be located in different spaces.

In an embodiment of the present disclosure, the analysis device 1200 and the transport device 1400 may be located inside the enclosure 1300. In this case, the preparation device 1100 is located outside the enclosure 1300.

The enclosure 1300 is provided with a second passthrough cavity (2nd passthrough cavity) 1310, which is a defined passage through which the transport device 1400 for transporting the reaction vessel 1500 to be provided from the preparation device 1100 to the analysis device 1200 is moved.

The second passthrough cavity 1310 of the enclosure 1300 is a defined passage through which the reaction vessel 1500 is transported, and the transport device 1400 may provide the reaction vessel 1500 from the first passthrough cavity 1130 of the preparation device 1100 to the analysis device 1200 through the second passthrough cavity 1310 of the enclosure 1300.

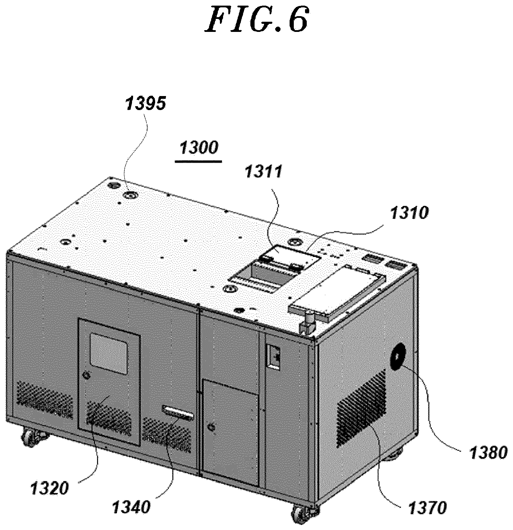

The position of the second passthrough cavity 1310 of the enclosure 1300 may be located on any one of the outer surfaces of the enclosure 1300. As illustrated in FIG. 6, when the enclosure 1300 has a hexahedron, the second passthrough cavity 1310 may be formed in an upper surface of the enclosure 1300, but is not limited thereto.

The transport device 1400 may be programmed to transport the reaction vessel 1500 through a path formed from the first passthrough cavity (1st passthrough cavity) 1130 of the preparation device 1100 to the second passthrough cavity 1310 of the enclosure 1300.

Therefore, it is preferable that each defined passage is located at a distance within a range in which the transport device 1400 can move the reaction vessel 1500.

The enclosure 1300 may be implemented in a hexahedron such as a cabinet, a locker, a box/case, etc. The enclosure 1300 is configured such that front/rear/left/right/top/bottom surfaces thereof are closed, and at least one door 1320 is provided. The enclosure door 1320 is installed on the front/rear, left/right, etc. so that the user may access the devices and components located therein.

The enclosure 1300 is not sealed, and an air vent 1370 may be formed.

The enclosure 1300 may include at least one selected from the group consisting of the preparation device 1100, the analysis device 1200, and the transport device 1400.

In addition, the enclosure 1300 may be provided with an automatic sealer 1700 for sealing the inlet of the upper surface of the reaction vessel 1500.

In addition, the enclosure 1300 may be provided with a liquid waste collection bin 1330 for recovering various solutions used to prepare the analysis sample in the preparation device 1100.

In addition, the enclosure 1300 may be provided with a reaction vessel reaction vessel retrieval container 1360 for recovering the reaction vessel 1500 in which the analysis is completed in the analysis device 1200.

In an embodiment of the present disclosure, the reaction vessel retrieval container 1360 may be located inside the enclosure 1300.

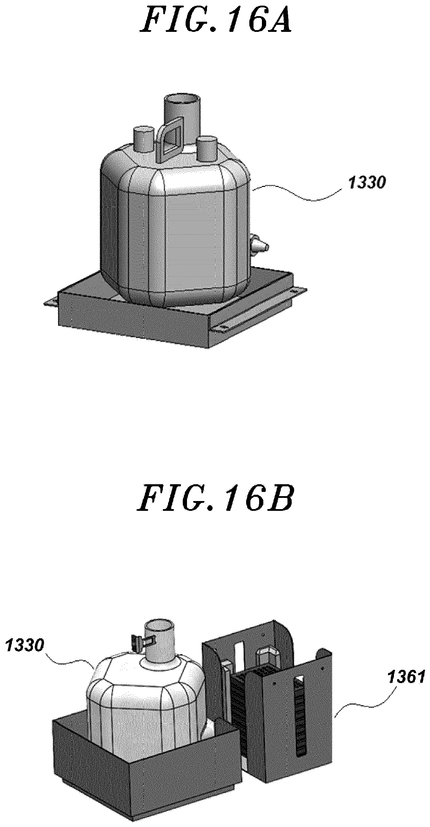

Referring to FIG. 16B, when the reaction vessel retrieval container 1361 is located inside the enclosure 1300, the reaction vessel 1500 moved by the crane module 1430 may be accommodated. The reaction vessel retrieval container 1361 located therein may include a sensor for sensing at least one of the quantity and weight of the reaction vessel 1500 accommodated therein.

In another embodiment of the present disclosure, the reaction vessel retrieval container 1360 may be located outside the enclosure 1300.

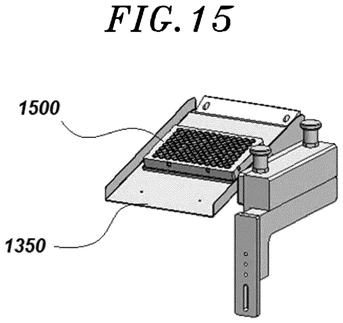

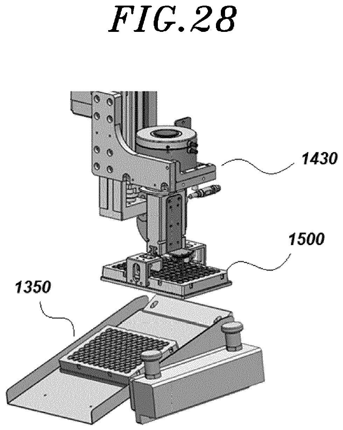

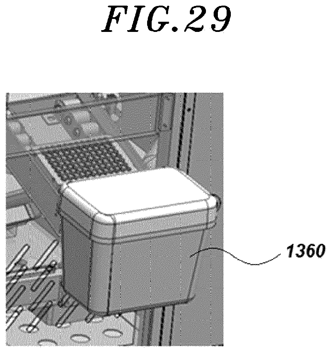

Referring to FIGS. 15, 28, and 29, the reaction vessel retrieval container 1360 located at the outside may recover the reaction vessel 1500 while the reaction vessel 1500 is transported from the inside to the outside through the recovery opening retrieval passthrough cavity 1340 connecting the inside and the outside of the enclosure 1300.

A conveyor 1350 may be installed in the retrieval passthrough cavity 1340 connecting the inside and the outside of the enclosure 1300 for the movement of the reaction vessel 1500 to be connected to each other. When the transport device 1400 puts the reaction vessel 1500 down on the inner conveyor 1350 of the enclosure 1300, the reaction vessel 1500 may be moved by the conveyor 1350 and may be accommodated in the reaction vessel retrieval container 1360 located outside the enclosure 1300.

In one embodiment of the present disclosure, the conveyor 1350 forms an inclined surface having an outer portion positioned lower than an inner portion. The conveyor 1350 may discharge the reaction vessel 1500 to the outside of the enclosure 1300 by having the rollers on the inclined surface. The reaction vessel 1500 discharged to the outside may be accommodated in the reaction vessel retrieval container 1360.

In another embodiment of the present disclosure, the conveyor 1350 may be driven by power. The power may rotate a belt included in the conveyor 1350 to discharge the reaction vessel 1500 placed on the conveyor 1350 to the outside of the enclosure 1300. The reaction vessel 1500 discharged to the outside may be accommodated in the reaction vessel retrieval container 1360.

The one or more reaction vessels 1500 accommodated therein may be emptied from the reaction vessel retrieval container 1360 by the user.

The retrieval passthrough cavity 1340 in which the inside and the outside of the enclosure 1300 are connected by the conveyor 1350 may include an open/close module (not shown). The open/close module may be opened when the reaction vessel 1500 is moved to the reaction vessel retrieval container 1360, and may be closed in other cases. The open/close module may protect the inside of the enclosure 1300 from external contamination.

In an embodiment of the present disclosure, the enclosure 1300 is not completely sealed and is implemented to be ventilated. To this end, the enclosure 1300 includes an environment control means, and the environment control means may include an air vent, an exhaust vent, a fan, a temperature control means, a humidity control means, an air filter, and the like.

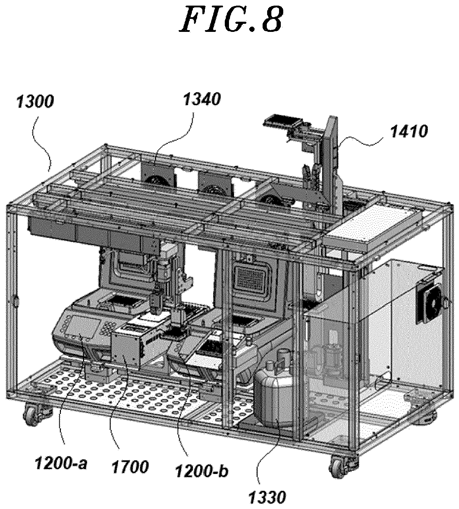

In FIGS. 6 and 8 of the present disclosure, at least one air vent 1370 or exhaust vent 1370 for air circulation in the enclosure 1300 is formed, and/or at least one fan 1380 for discharging internal air is formed.

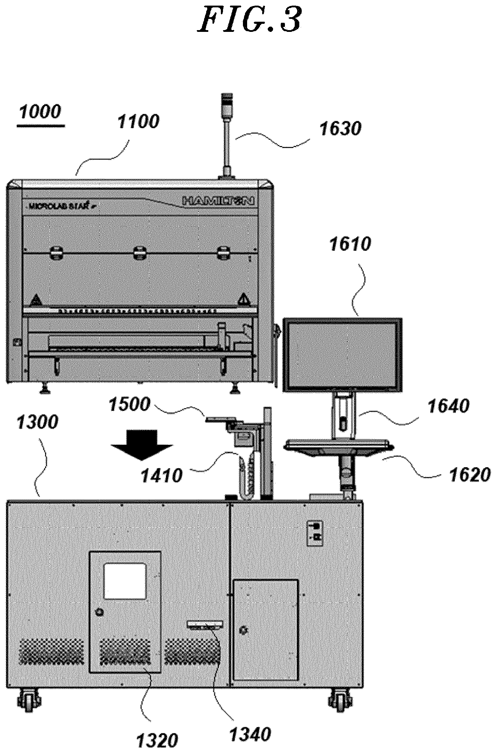

FIG. 3 is an exemplary diagram illustrating the operational connection of the automated analysis system according to an embodiment of the present disclosure. As illustrated in FIG. 3, the preparation device 1100 is located above the enclosure 1300.

The preparation device 1100 may be installed and operated alone for preparation of an analysis sample, and may be used as the automated analysis system 1000 in operative connection with the enclosure 1300, according to an embodiment of the present disclosure.

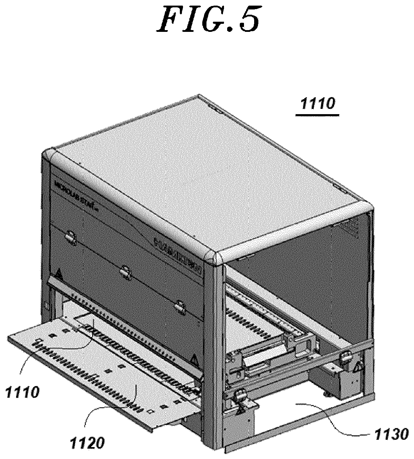

According to an embodiment of the present disclosure, when the preparation device 1100 is combined with the enclosure 1300 and used as the automated analysis system 1000, the preparation device 1100 may form a first passthrough cavity 1130 so that the lift module 1410 provided in the enclosure 1300 may move inside (see FIG. 5). The first passthrough cavity 1130 is an empty space and is a defined passage through which the reaction vessel 1500 is moved by the lift module 1410.

When the lift module 1410 is moved into the preparation device 1100, the preparation device 1100 mounts a reaction vessel 1500 for receiving the analysis sample or a plate (not shown) for receiving the reaction vessel 1500 on the lift module 1410. The lift module 1410 moves the mounted reaction vessel 1500 or plate to the inside of the enclosure 1300.

According to an embodiment of the present disclosure, when the preparation device 1100 is positioned on the enclosure 1300, the preparation device 1100 and the enclosure 1300 may be coupled to each other through a coupling mechanism (not shown).

In an embodiment of the present disclosure, when at least one of the preparation device 1100 and the analysis device 1200 is operatively connected to the enclosure 1300 to be used as the automated analysis system 1000, the preparation device 1100 and the analysis device 1200 use the power module used as the stand-alone device as it is.

In one embodiment of the present disclosure, the preparation device and the analysis device may be supplied with power by separate power sources, respectively.

In an embodiment of the present disclosure, when at least one of the stand-alone-type preparation device 1100 and the stand-alone-type analysis device 1200 is operatively connected to the enclosure 1300 to be used as the automated analysis system 1000, the stand-alone-type preparation device 1100 and the stand-alone-type analysis device 1200 are devices that have been already commercialized and/or licensed as stand-alone-type devices.

Accordingly, even when being operatively connected to the enclosure 1300 and used as the automated analysis system 1000, a separate license may not be required or there may be partial modification.

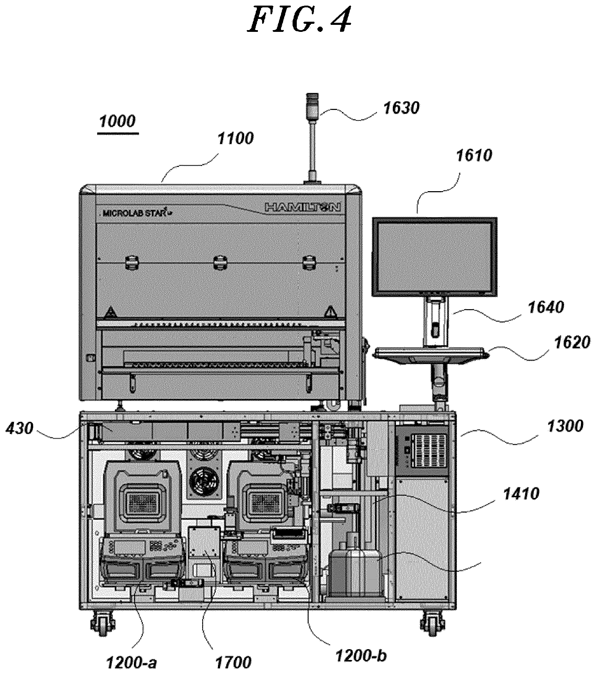

FIG. 4 is an internal front view illustrating the automation analysis system of the present disclosure. As shown in FIG. 4, the analysis devices 1200-a and 1200-b are located under the enclosure 1300.

The enclosure 1300 will be described with reference to FIGS. 8, 30, and 31.

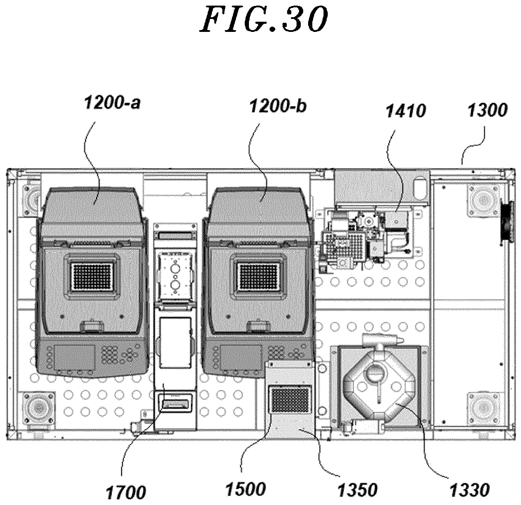

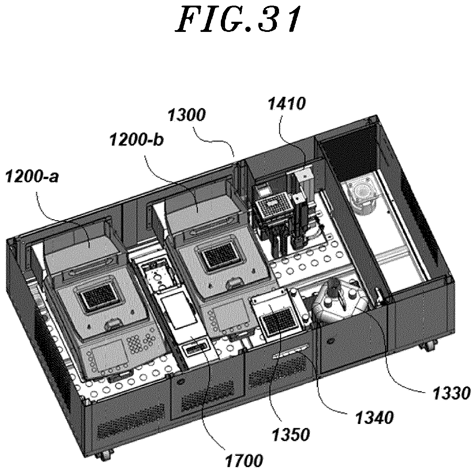

FIG. 8 is an internal perspective view illustrating an enclosure of the automation analysis system of the automation analysis system according to an embodiment of the present disclosure. FIG. 30 is an internal plan view illustrating the interior of an enclosure according to an embodiment of the present disclosure. FIG. 31 is an internal perspective view illustrating the interior of an enclosure according to an embodiment of the present disclosure.

In one embodiment of the present disclosure, the enclosure 1300 comprises a transport device 1400 for providing the reaction vessel 1500 prepared in the preparation device 1100 to the analysis devices 1200-a, 1200-b.

The transport device 1400 is a robotic module, and particularly, includes a lift module 1410 and a crane module 1430.

In an embodiment of the present disclosure, the transport device 1400 includes a lift module 1410 and a crane module 1430.

In another embodiment of the present disclosure, the transport device 1400 includes a lift module 1410.

In another embodiment of the present disclosure, the transport device 1400 includes a crane module 1430.

In another embodiment of the present disclosure, the transport device 1400 includes a robot arm (not shown).

In another embodiment of the present disclosure, the transport device 1400 may include a mechanical device capable of transporting the reaction vessel 1500.

In an embodiment of the present disclosure, the lift module 1410 and the crane module 1430 may be located inside the enclosure 1300.

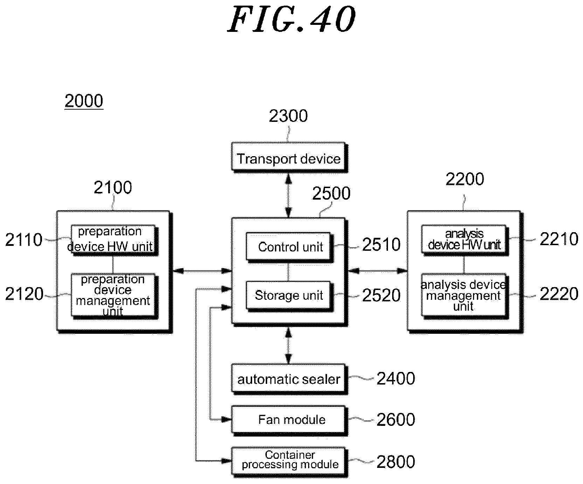

The lift module 1410 and the crane module 1430 included in the enclosure 1300 are robotic modules. The robotic module moves the reaction vessel 1500 under the control of the control module 2500 (see FIG. 40) included in the automation analysis system 1000.

An automatic sealer 1700 for sealing an inlet of the reaction vessel 1500 may be disposed in the enclosure 1300.

The enclosure 1300 may be provided with at least one analysis device, 1200-a, 1200-b for analyzing the analysis sample received in the reaction vessel 1500.

In the enclosure 1300, a liquid waste collection bin 1330 for recovering various solutions used to prepare the analysis sample in the preparation device 1100 may be positioned.

A reaction vessel retrieval container 1360 for collecting the reaction vessel 1500, which has been completely analyzed by the analysis devices 1200-a and 1200-b, may be located in the enclosure 1300.

The enclosure 1300 includes a control module 2500 (see FIG. 40) that transmits and/or receives data from a plurality of devices operatively connected to the automated analysis system 1000.

In an embodiment of the present disclosure, the control module may be operatively connected to at least one of the preparation device 1100, the analysis device 1200, the transport device 1400, and/or the automatic sealer 1700 a through a communication channel. For example, the communication channel may be connected wirelessly and/or by wire.

In one embodiment of the present disclosure, the control module may control the preparation device, the analysis device, and the transport device to operate in a timely manner. The control module controls the preparation device, the analysis device, and the transport device so that the preparation of the analysis sample using the preparation device and the analysis device of the present disclosure, which are the stand-alone devices, and the analysis thereof may be automatically performed. Specifically, when the preparation of the analysis sample is completed in the preparation device, the control module controls the transport device to transport the analysis sample to the analysis device. In addition, in order to mount the analysis sample on the analysis device, a signal, which is to be provided to the analysis device, is provided to mount the analysis sample thereon, and the analysis device starts an analysis.

Further, in an embodiment of the present disclosure, the control module may provide an external signal required for the operation of the stand-alone device. The external signal requiring the operation may be, for example, a signal allowing the analysis device to be in a state capable of receiving an analysis sample, a signal allowing the analysis device to start analysis, and a signal allowing the automatic sealer to start a sealing operation.

As described above, the control module not only transfers signals between the stand-alone devices to allow the analysis sample to be moved from the preparation device to the analysis device, but also provides signals that should be input manually by a user or the like in the related art in a timely manner in order for the stand-alone devices to independently perform unit work, thereby implementing a full automation system.

In an embodiment of the present disclosure, the control module may receive a signal in which the preparation of the analysis sample is completed in the preparation device 1100 via a communication channel.

Also, a control signal for moving the reaction vessel 1500 of the preparation device 1100 to the analysis device 1200 may be provided to the transport device 1400 via a communication channel.

In addition, the control module may receive a signal in which the analysis of the analysis sample is completed in the analysis device 1200 via a communication channel.

In addition, the control module may provide a control signal for moving the reaction vessel 1500 of the analysis device 1200 to the reaction vessel retrieval container 1360 to the transport device 1400 via a communication channel.

The enclosure 1300 has a second defined passage 1310 formed on an upper surface thereof through which the lift module 1410 moving to the preparation device 1100 to receive the reaction vessel 1500 passes.

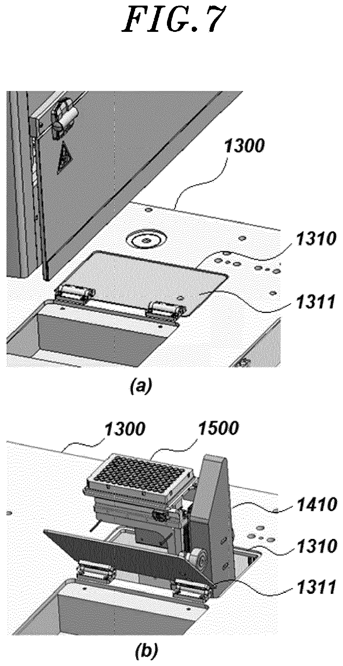

According to an embodiment of the present disclosure, the second defined passage 1310 may be described as follows with reference to FIGS. 6 and 7. FIG. 6 is a perspective view illustrating an enclosure according to an embodiment of the present disclosure. FIG. 7 is an exemplary diagram for illustrating an operation state of a second passthrough cavity of an enclosure according to an embodiment of the present disclosure. As shown in FIGS. 6 and 7, a second defining passage 1310 is formed in the upper surface of the enclosure 1300.

The second defined passage 1310 is formed to have a size through which a vertical motion guide 1413 and an analytical sample vessel rack 1416 included in the lift module 1410 may pass.

The second defined passage 1310 is formed to vertically connect with the first defined passage 1130 formed in the deck 1110 of the preparation device 1100.

In one embodiment of the present disclosure, the second defined passage 1310 is formed on the right side of the top surface of the enclosure 1300, and the first defined passage 1130 is formed on the right side in the plane of the deck 1110.

In another embodiment of the present disclosure, the second defined passage 1310 may be formed on the left side of the top surface of the enclosure 1300, and the first defined passage 1130 may be formed on the left side in the plane of the deck 1110.

In yet another embodiment of the present disclosure, the second defined passage 1310 may be formed on the upper side of the top surface of the enclosure 1300, and the first defined passage 1130 may be formed on the upper side in the plane of the deck 1110.

In yet another embodiment of the present disclosure, the second defined passage 1310 may be formed on the underside of the top surface of the enclosure 1300, and the first defined passage 1130 may be formed on the underside in the plane of the deck 1110.

In an embodiment of the present disclosure, the second defined passage 1310 is a passage for moving a part of the lift module 1410 located inside the enclosure 1300 into the preparation device 1100. (See FIG. 6)

In another embodiment of the present disclosure, the second defined passage 1310 is an opened passage for moving a part of the lift module 1410 inside the enclosure 1300 into the preparation device 1100, and when the part of the lift module 1410 does not move into the preparation device 1100, the second defined passage 1310 opened through the gate part 1311 provided in the second defined passage 1310 may be closed. (See FIG. 7)

The gate part 1311 is provided to block contaminants outside the enclosure 1300.

In an embodiment of the present disclosure, the gate part 1311 may be implemented in a hinge manner to be opened/closed to an upper side or a lower side of the upper surface of the enclosure 1300.

In another embodiment of the present disclosure, the gate part 1311 may be implemented in a sliding manner to be opened/closed in a manner of being moved on the upper surface of the enclosure 1300.

In another embodiment of the present disclosure, the gate part 1311 may be any type in which the second defined passage 1310 may be opened and closed by a method other than the hinge method or the sliding method.

As shown in FIG. 7(a), when the lift module 1410 is located inside the enclosure 1300, the gate part 1311 of the second defined passage 1310 is closed.

As shown in FIG. 7(b), when the lift module 1410 moves to the preparation device 1100, the gate part 1311 of the second defined passage 1310 is opened. The gate part 1311 of the second defined passage 1310 is closed after the lift module 1410 moves into the enclosure 1300.

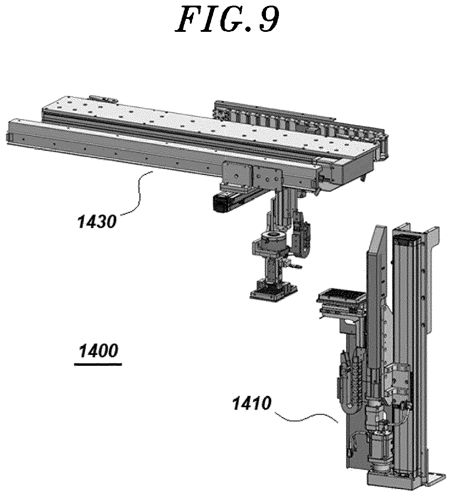

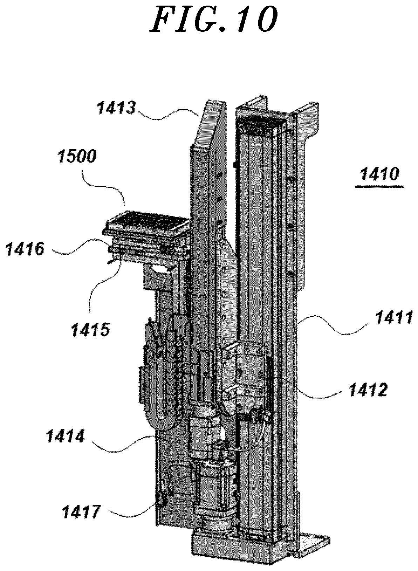

According to an embodiment of the present disclosure, the lift module 1410 may be described as follows with reference to FIGS. 9 and 10. FIG. 9 is a perspective view illustrating a transport device according to an embodiment of the present disclosure. FIG. 10 is a perspective view illustrating the lift module of the transport device according to an embodiment of the present disclosure.

The lift module 1410 is a component capable of receiving the reaction vessel 1500 in the preparation device 1100. The lift module 1410 is representative of an operational form of an elevator that can be elevated to the preparation device 1100 on top of the enclosure 1300 to receive an analysis sample from the preparation device 1100.

The lift module 1410 includes a vertical fixed guide 1411 in the form of a pillar fixed inside the enclosure 1300. The vertical fixed guide 1411 includes a fixed guide connector 1412 that moves up/down. The lift module 1410 includes a vertical motion guide 1413 coupled to the fixed guide connector 1412 of the vertical fixed guide 1411.

The vertical motion guide 1413 includes a motion guide connector 1414 that is moved up/down. The vertical motion guide 1413 includes a rack guide 1415 coupled to the motion guide connector 1414.

The lift module 1410 includes a reaction vessel analytical sample vessel rack 1416 receiving a reaction vessel above the rack guide 1415.

The lift module 1410 includes an actuator 1417 configured to provide power for moving the vertical motion guide 1413 upward/downward.

The vertical fixed guide 1411 is coupled and fixed to at least one of the upper portion, the lower portion, and/or the side surface of the inside of the enclosure 1300. The vertical fixed guide 1411 is coupled to the fixed guide connector 1412. The vertical fixed guide 1414 may move the coupled fixed guide connector 1412 in the up/down direction.

The vertical fixed guide 1411 may use power provided from the actuator 1417 to move the fixed guide connector 1412 upward/downward.

The size of the vertical fixed guide 1411 is equal to or smaller than the inner height of the enclosure 1300. As the vertical fixed guide 1411 moves the fixed guide connector 1412 upward, the vertical motion guide 1413 coupled to the fixed guide connector 1412 may be moved to the preparation device 1100 via the second defined passage 1310.

The fixed guide connector 1412 coupled to the vertical fixed guide 1411 couples the vertical motion guide 1413, and the fixed guide connector 1412 moves the vertical motion guide 1413 according to the up/down movement provided by the vertical fixed guide 1411.

The vertical motion guide 1413 may be moved in the vertical direction according to the driving of the vertical fixed guide 1411.

In one implementation of the disclosure, vertical motion guide 1413 is coupled with motion guide connector 1414. The vertical motion guide 1413 may move the coupled operation guide connector 1414 in the up/down direction.

The vertical motion guide 1413 may use power provided from the actuator 1417 to move the motion guide connector 1414 in the up/down direction.

In another embodiment of the disclosure, the vertical motion guide 1413 may be coupled in a fixed component without moving the motion guide connector 1414.

In an exemplary embodiment of the present disclosure, the operation guide connector 1414 coupled to the vertical motion guide 1413 to receive the vertical movement may be coupled to the rack guide 1415.

A rack guide 1415 is coupled to an upper portion of the operation guide connector 1414, and when the vertical fixed guide 1411 moves the vertical motion guide 1413 upward and the vertical motion guide 1413 moves the rack guide 1415 upward, the rack guide 1415 is moved into the preparation device 1100.

In another embodiment of the present disclosure, the motion guide connector 1414 may be coupled with the rack guide 1415.

When the vertical fixed guide 1411 moves the fixed guide connector 1412 upward, the rack guide 1415 may be moved into the preparation device 1100 by the movement of the vertical motion guide 1413 and the operation guide connector 1414.

The vertical motion guide 1413 uses power provided from the actuator 1417 or a separate actuator (not shown) to move the rack guide 1415 in the up/down direction.

The rack guide 1415 may be coupled to the operation guide connector 1414, and an analytical sample vessel rack 1416 on which the reaction vessel 1500 may be placed may be positioned at an upper portion.

The analytical sample vessel rack 1416 is a space in which the reaction vessel 1500 containing the analysis sample is located in the preparation device 1100. The analytical sample vessel rack 1416 may be referred to as a pedestal, a cradle, a holder, or the like.

The analytical sample vessel rack 1416 may be moved into the preparation device 1100 by movement of the operation guide connector 1414 together with the rack guide 1415, and may receive the reaction vessel 1500.

The rack guide 1415 performs an extension movement in the horizontal direction of the analytical sample vessel rack 1416 connected thereto.

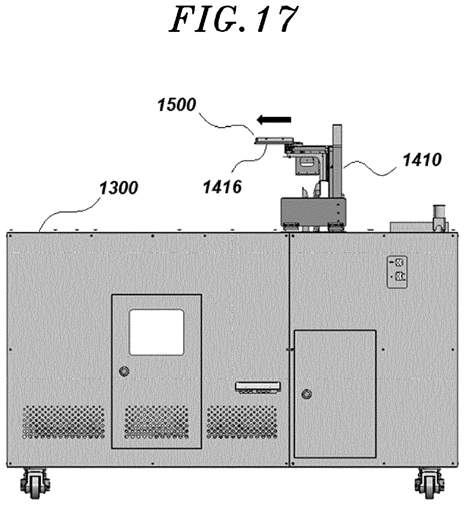

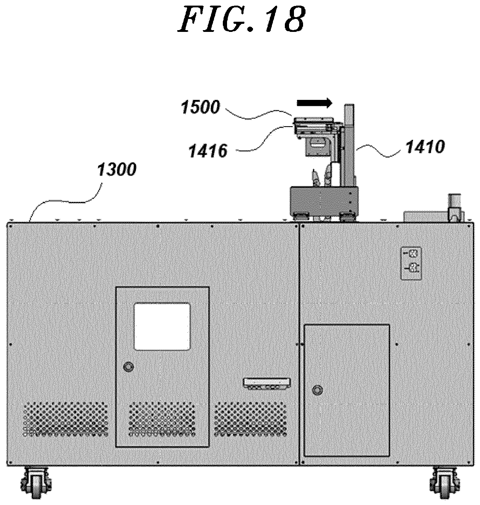

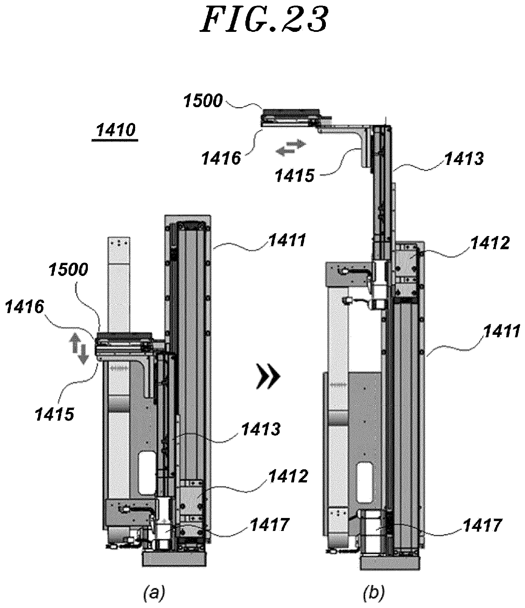

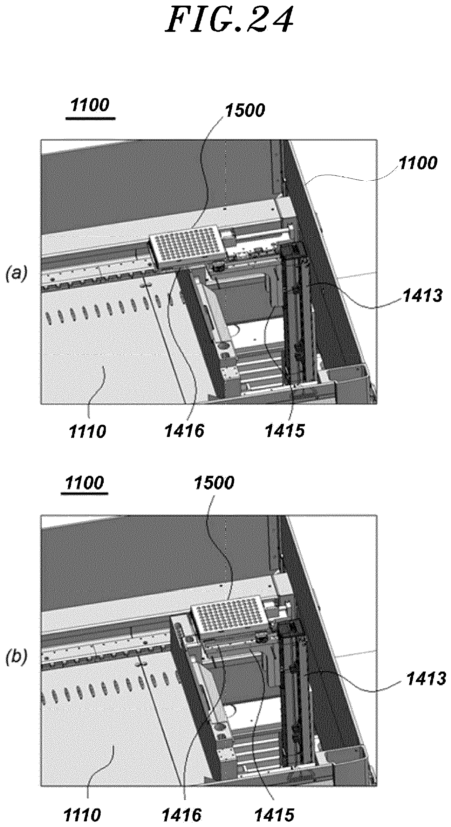

The extended movement of the analytical sample vessel rack 1416 may be described with reference to FIGS. 17, 18, 23 and 24. FIG. 17 is a first exemplary view illustrating the horizontal extension movement of the lift module in the enclosure according to an embodiment of the present disclosure. FIG. 18 is a second exemplary view illustrating a horizontal extension movement of the lift module in the enclosure according to an embodiment of the present disclosure. FIG. 24 is a perspective view illustrating a horizontal extension movement of the lift module in the preparation device according to an embodiment of the present disclosure.

As shown in FIGS. 17 and 18, the vertical fixed guide 1411 of the lift module 1410 moves the fixed guide connector 1412 in the vertical direction using the power provided from the actuator 1417, and a part of the fixed guide connector 1412 is coupled to the vertical fixed guide 1411 and the other part is coupled to the vertical motion guide 1413.

The vertical motion guide 1413 may move into the preparation device 1100 according to the upward movement of the coupled fixed guide connector 1412. In addition, the vertical motion guide 1413 may move the operation guide connector 1414 coupled to the other side in the up/down direction. The operation guide connector 1414 moving upward by the operation of the vertical motion guide 1413 may move the rack guide 1415 connected to the upper portion thereof to the inside of the preparation device 1100.

When the rack guide 1415 is moved to the inside of the preparation device 1100, the rack guide 1415 may perform horizontal extension movement on the analytical sample vessel rack 1416 located at the upper portion by a predetermined distance.

The transfer module (not shown) provided in the preparation device 1100 picks up and transports the prepared reaction vessel 1500 and places the reaction vessel on the horizontally extended-moved analytical sample vessel rack 1416, thereby allowing the reaction vessel 1500 to be moved to the enclosure 1300.

When the reaction vessel 1500 is mounted on the analytical sample vessel rack 1416, the rack guide 1415 horizontally moves the analytical sample vessel rack 1416, which has been horizontally extended-moved, back to its original position.

The vertical fixed guide 1411 and/or the vertical motion guide 1413 move the coupled fixed guide connector 1412 and/or motion guide connector 1414 downward when the reaction vessel 1500 is ready to be moved to the enclosure 1300. The reaction vessel 1500 is moved into the enclosure 1300.

The analytical sample vessel rack 1416 includes a coupling guide (not shown) corresponding to the reaction vessel 1500 mounted thereon. The coupling guide prevents the reaction vessel 1500 from being separated from the analytical sample vessel rack 1416 while moving.

In one embodiment of the disclosure, the actuator 1417 may provide power for horizontal movement of the analytical sample vessel rack 1416.

In another embodiment of the disclosure, the rack guide 1415 may be powered by another actuator for horizontal movement of the analytical sample vessel rack 1416.

The actuator 1417 may provide power for moving the lift module 1410 in the enclosure 1300. One or more actuators 1417 may be provided, and may be located at one or more places.

In one embodiment of the present disclosure, the actuator 1417 may use a hydraulic motor.

In another embodiment of the present disclosure, the actuator 1417 may use an electric motor.

In another embodiment of the present disclosure, the actuator 1417 may be used by mixing a hydraulic motor and an electric motor.

In another embodiment of the present disclosure, the actuator 1417 may use a device capable of generating power except for the hydraulic motor and the electric motor.

In another embodiment of the present disclosure, the actuator 1417 may use a hydraulic motor, an electric motor and a device capable of generating power.

One or more actuators 1417 may be provided, and may provide power to at least one of the lift module 1410, the crane module 1430, and/or the analytical sample vessel rack 1416 in the enclosure 1300.

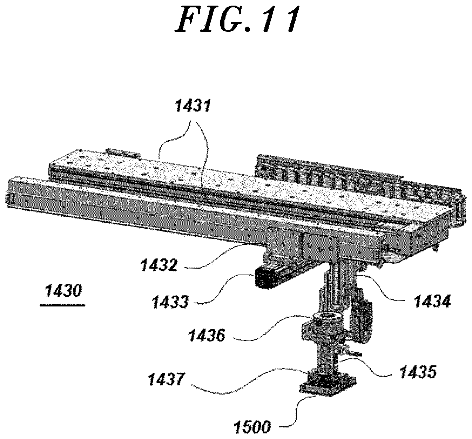

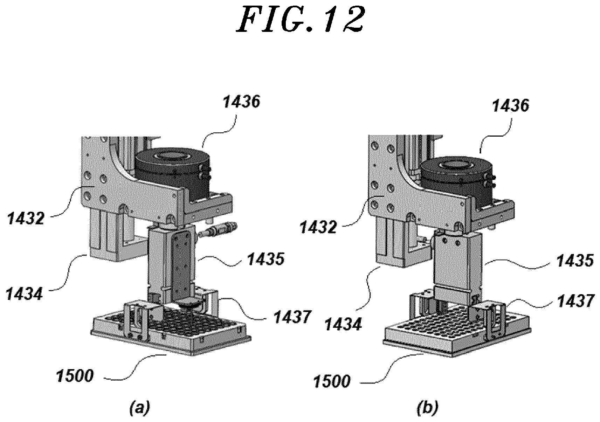

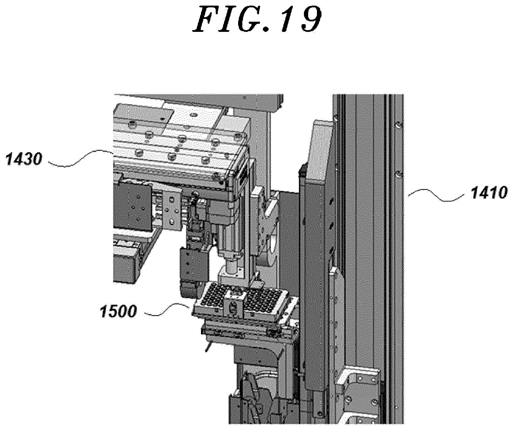

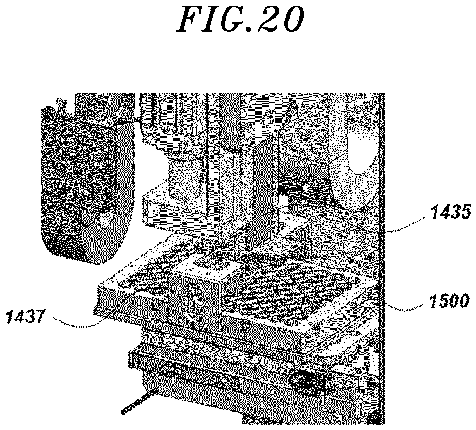

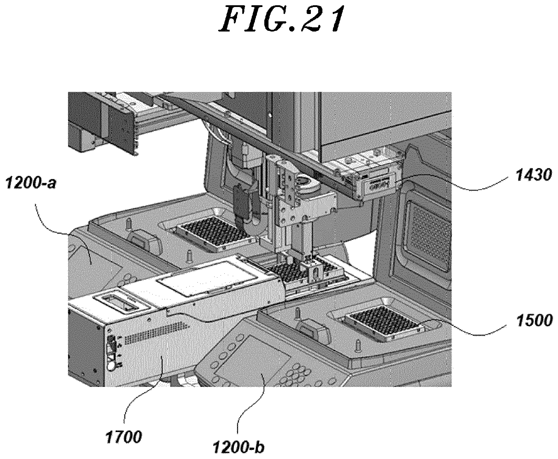

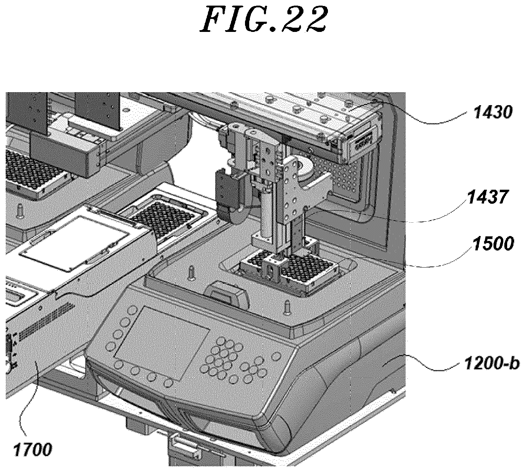

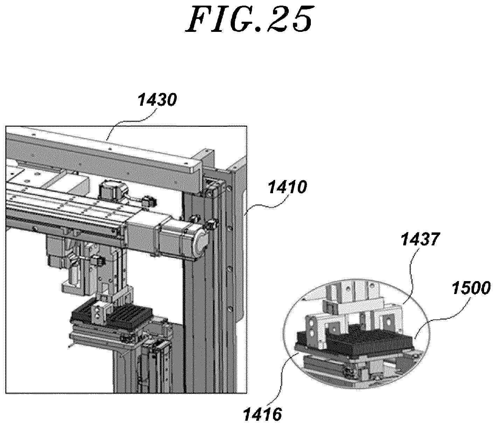

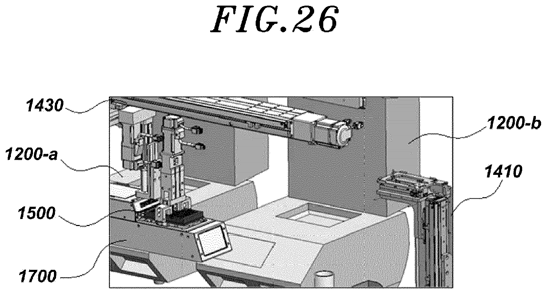

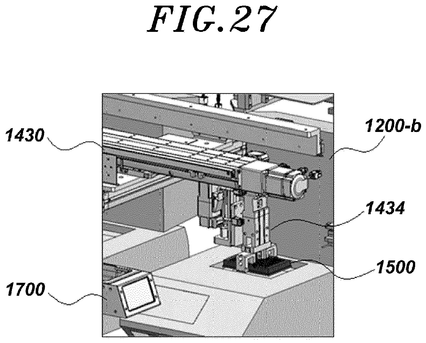

According to an embodiment of the present disclosure, the crane module 1430 may be described as follows with reference to FIGS. 9, 11 to 12, and 19 to 22. FIG. 11 is a perspective view illustrating the crane module of the transport device, according to an embodiment of the present disclosure. FIG. 12 is a perspective view illustrating a rotation component of the crane module according to an embodiment of the present disclosure. FIG. 19 is a first exemplary view illustrating the transport of the reaction vessel of the lift module and the crane module according to an embodiment of the present disclosure. FIG. 20 is a second exemplary view illustrating transport of the reaction vessel of the lift module and the crane module according to an embodiment of the present disclosure. FIG. 21 is an exemplary diagram illustrating the operation of mounting a reaction vessel on an automatic plate sealer according to an embodiment of the present disclosure. FIG. 22 is an exemplary diagram illustrating the operation of mounting a reaction vessel on the analysis device according to an embodiment of the present disclosure. FIG. 25 is an exemplary view illustrating that the crane module according to an embodiment of the present disclosure transports a reaction vessel in the lift module. FIG. 26 is an exemplary view illustrating that a crane module according to an embodiment of the present disclosure mounts a reaction vessel to an automatic sealer. FIG. 27 is an exemplary view illustrating that the crane module according to an embodiment of the present disclosure mounts the reaction vessel to the analysis device.

As shown in FIGS. 9, 11 to 12, and 19 to 22, the crane module 1430 performs an operation for moving the reaction vessel 1500 received by the lift module 1410 from the preparation device 1100 to each component of the enclosure 1300.

In an embodiment of the present disclosure, the crane module 1430 includes a horizontal fixed guide 1431, a horizontal motion guide 1433, a gripper lift 1434, a gripper rotation module 1436, and a gripper 1437 at an upper portion within the enclosure 1300, and the gripper 1437 may move and rotate in X, Y, and Z axis directions by the respective guides 1431, 1433 and the gripper rotation module 1436.

In another embodiment of the present disclosure, the crane module 1430 includes a horizontal fixed guide 1431, a guide connector 1432, a horizontal motion guide 1433, a gripper lift 1434, a gripper motion guide 1435, and a gripper 1437 on top of the enclosure 1300, wherein the gripper 1437 can be moved in the X, Y, Z axes by the respective guides 1431, 1433 and the gripper lift 1434.

The horizontal fixed guide 1431 is coupled to the horizontal motion guide 1433 by a fixed guide connector 1432.

The horizontal fixed guide 1431 may be provided in the form of one or more movable rails. The horizontal motion guide 1433 is coupled to the rail-shaped horizontal fixed guide 1431 via the fixed guide connector 1432 and moves in the X-axis direction.

The horizontal fixed guide 1431 may be provided in the form of two rails to stably move the horizontal motion guide 1433, and the horizontal motion guide 1433 may be connected to the two rails to be moved in the X-axis direction. One of the two rails of the horizontal fixed guide 1431 may move the combined horizontal motion guide 1433 in the X-axis direction.

In an embodiment of the present disclosure, the actuator 1417 may provide power for the horizontal fixed guide 1431 to move the horizontal motion guide 1433.

In another embodiment of the present disclosure, the power that the horizontal fixed guide 1431 moves the horizontal motion guide 1433 may be provided from an actuator (not shown) that is separately provided.

The horizontal motion guide 1433 is coupled to the gripper lift 1434.

The horizontal motion guide 1433 is provided in the form of a rail. The gripper lift 1434 is coupled to the rail-shaped horizontal motion guide 1433 to move in the Y-axis direction. The horizontal motion guide 1433 providing the rail-shaped motion may move the coupled gripper lift 1434 in the Y-axis direction.

In an embodiment of the present disclosure, the power that the horizontal motion guide 1433 moves the gripper lift 1434 may be provided by the actuator 1247.

In another embodiment of the present disclosure, the power that the horizontal motion guide 1433 moves the gripper lift 1434 may be provided from an actuator (not shown) that is separately provided.

The gripper lift 1434 is connected to a gripper motion guide 1435 and is coupled to the gripper 1437. The gripper movement guide 1435 is coupled to the gripper lift 1434 that is moved upward/downward to be moved in the Z-axis direction. The gripper list 1243 may move the combined gripper 1437 in the Z-axis direction.

The gripper lift 1434 is formed by combining two modules.

One is a fixing module coupled to the horizontal motion guide 1433 and moved in the Y-axis. The other is a moving module which is combined with the gripper movement guide 1435 and moves the gripper 1437 in the up/down direction. (See FIG. 12) The gripper movement guide 1435 is combined with the gripper 1437, and the gripper 1437 is moved in the Z-axis direction by the movement provided in the up/down direction of the gripper lift 1434.

The gripper 1437 may be moved to the position of the reaction vessel 1500 by the operation of the gripper lift 1434 to pick up the reaction vessel 1500. The gripper 1437 may sense a pressure for gripping the reaction vessel 1500 using a pressure sensor or the like to hold the container so that the reaction vessel 1500 is not damaged.

In one embodiment of the disclosure, the gripper 1437 is capable of rotating the picked-up reaction vessel 1500.

As shown in FIG. 12, the gripper movement guide 1435 coupled to the gripper lift 1434 is coupled to the gripper 1437 and the gripper rotation module 1436. The gripper rotation module 1436 may rotate the gripper 1437 as shown in (a) to (b) of FIG. 12. The gripper rotation module 1436 is made of a rotary motor.

In an embodiment of the disclosure, the gripper rotation module 1436 may rotate the gripper 1437 at a rotation angle of 90 degrees.

In another embodiment of the present disclosure, the gripper rotation module 1436 may rotate the gripper 1437 at every rotation angle.

One or more actuators used in the lift module 1410 and/or the crane module 1430 may be operated using various driving forces.

In one embodiment of the present disclosure, at least one or more of the actuators may use a hydraulic motor.

In another embodiment of the present disclosure, at least one of the actuators may use an electric motor.

In another embodiment of the present disclosure, at least one or more of the actuators may be used by mixing a hydraulic motor and an electric motor.

In another embodiment of the present disclosure, at least one of the actuators may use a device capable of generating power except for the hydraulic motor and the electric motor.

In another embodiment of the present disclosure, at least one of the actuators may use a hydraulic motor, an electric motor, and a device capable of generating power.

As shown in FIGS. 19 and 20, the crane module 1430 may move the gripper 1437 to a position above the analytical sample vessel rack 1416 of the lift module 1410 to move the reaction vessel 1500. The crane module 1430 may pick up the reaction vessel 1500 after the gripper 1437 located on top of the analytical sample vessel rack 1416 is lowered.

In one embodiment of the present disclosure, the action of the crane module 1430 moving the gripper 1437 to the top of the analytical sample vessel rack 1416 is moved by pre-stored position coordinates.

The automated analysis system 1000 stores all the locations to which the crane module 1430 can move within the enclosure 1300 as coordinate information. The crane module 1430 may perform the movement according to coordinate information of a location to be moved.

In another embodiment of the present disclosure, the action of the crane module 1430 moving the gripper 1437 over the analytical sample vessel rack 1416 is moved by pre-stored position coordinates, and can further be stopped in position by a position sensor module (not shown) provided in the enclosure 1300. The position sensor module allows the gripper 1437 to stop at a predetermined position by transmitting and receiving signals between the gripper 1437 and the analytical sample vessel rack 1416 based on the optical signal.

The position sensor module may be provided on the analytical sample vessel rack 1416, the automatic sealer 1700, the analysis devices 1200-a and 1200-b, the reaction vessel retrieval container 1360, or the conveyor 1350, which are components by which the gripper 1437 moves the reaction vessel 1500.

The lift module 1410 and the crane module 1430 of the transport device 1400 included in the enclosure 1300 receive power for moving the reaction vessel 1500.

The lift module 1410 receives power from the vertical fixed guide 1411, the vertical motion guide 1413, and/or the rack guide 1415.

The crane module 1430 receives power from the horizontal fixed guide 1431, the horizontal motion guide 1433, the gripper lift 1434, and/or the gripper 1437.

Each component receiving power from the lift module 1410 and the crane module 1430 may perform an operation through the following driving method.

In an embodiment of the present disclosure, the lift module 1410 and/or the crane module 1430 may provide a belt type of movement to move the reaction vessel 1500.

In another embodiment of the present disclosure, the lift module 1410 and/or the crane module 1430 may provide chain type movement to move the reaction vessel 1500.

In another embodiment of the present disclosure, the lift module 1410 and/or the crane module 1430 may provide a screw-type or jack-screw-type motion to move the reaction vessel 1500.

In another embodiment of the present disclosure, the lift module 1410 and/or the crane module 1430 may provide a cylinder type of movement to move the reaction vessel 1500.

In another embodiment of the present disclosure, the lift module 1410 and/or the crane module 1430 may provide a hoist type of movement to move the reaction vessel 1500.

In another embodiment of the present disclosure, the lift module 1410 and/or the crane module 1430 may move the reaction vessel 1500 through a driving scheme other than the scheme described above.

As shown in FIG. 21, the crane module 1430 can move the reaction vessel 1500 picked up from the analytical sample vessel rack 1416 to be mounted on the automated sealer 1700. The automatic sealer 1700 is a device for automatically sealing the upper surface of the reaction vessel 1500.

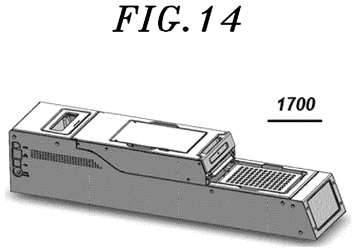

The automatic sealer 1700 will be described below with reference to FIG. 14.

FIG. 14 is a perspective view illustrating an automatic sealer according to an embodiment. As shown in FIG. 14, an automatic sealer 1700 may seal an inlet of a reaction vessel 1500 in which an analysis sample is accommodated, and may be implemented by the following embodiment.

In an embodiment of the present disclosure, the reaction vessel 1500 is a multi-well plate, and an analysis sample is received in each of the multi-well plates in which a plurality of wells having closed lower portions are formed.

The automatic sealer 1700 may seal an upper surface of the reaction vessel 1500, which is a multi-well plate, to prevent mixing of the analysis sample and contamination from the outside.

In another embodiment of the present disclosure, the reaction vessel 1500 is a tube-shaped vessel inserted into each well of a multi-well plate. A plurality of connected or individually separated tubes may be inserted into each well of the multi-well plate.

The automatic sealer 1700 may seal a top surface of at least one reaction vessel 1500 inserted into each well of the multi-well plate to prevent mixing of the analysis sample and contamination from the outside.

The automatic sealer 1700 may thermally adhere the injection port of the reaction vessel 1500 using a transparent film. Alternatively, the adhesive may be used.

In an embodiment of the present disclosure, the automatic sealer 1700 may use Plate Sealer product of Hamilton Inc. (see https://www.hamiltoncompany.com/automated-liquid-handling/small-devices/hamilton-plate-sealer).

The automatic sealer 1700 may be variously positioned within the enclosure 1300.

In one embodiment of the present disclosure, the automatic sealer 1700 may be disposed as shown in FIG. 30. As shown in FIG. 30, the automatic sealer 1700 may be located between the first analysis device 1200-a and the second analysis device 1200-b.

When the automatic sealer 1700 is positioned between the first analysis device 1200-a and the second analysis device 1200-b, the reaction vessel 1500 may be horizontally rotated by 90 degrees and mounted on the automatic sealer 1700.

A 90-degree horizontal rotation of the reaction vessel 1500 may be performed by the gripper rotation module 1436 of FIG. 12.

The crane module 1430 horizontally rotates the reaction vessel 1500 by 90 degrees using the gripper rotation module 1436 to mount the reaction vessel 1500 to the automated sealer 1700.

In another embodiment of the present disclosure, the automatic sealer 1700 may be implemented to be located at other locations inside the enclosure 1300.

In another embodiment of the present disclosure, the automatic sealer 1700 may be implemented to be located in the preparation device 1100.

As shown in FIG. 22, the crane module 1430 may move the reaction vessel 1500 sealed by the automatic sealer 1700 to be mounted on any one of the plurality of analysis devices 1200-a and 1200-b. The analysis devices 1200-a, 1200-b are devices for automatically analyzing one or more analysis samples contained within the reaction vessel 1500.

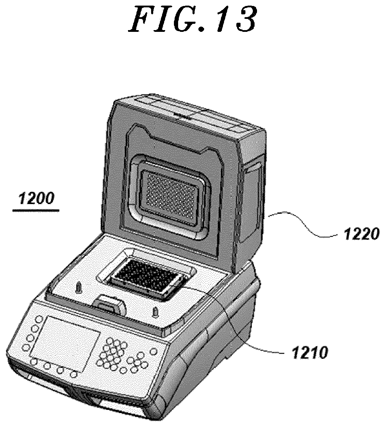

The analysis device 1200 according to an embodiment of the present disclosure may be described as follows with reference to FIG. 13. FIG. 13 is a perspective view illustrating a stand-alone analysis device according to an embodiment of the present disclosure. As shown in FIG. 13, the analysis device 1200 is a stand-alone device. That is, the analysis device 1200 may be installed and operated alone for analysis of the analysis sample.

The analysis device 1200 is a device for automatically analyzing one or more analysis samples contained in the reaction vessel 1500.

According to one embodiment of the disclosure, the analysis device 1200 may be operatively connected to the preparation device 1100 and/or the enclosure 1300 and used as the automated analysis system 1000.

The analysis device 1200 may include a nucleic acid amplifier for amplifying a nucleic acid and/or an optical module for detecting the amplified nucleic acid.

In one embodiment of the present disclosure, the analysis device 1200 includes a nucleic acid amplifier and an optical module.

In another embodiment of the present disclosure, the analysis device 1200 includes a nucleic acid amplifier.

In another embodiment of the present disclosure, the analysis device 1200 includes an optical module.

In an embodiment of the present disclosure, one analysis device 1200 may be operatively connected and applied to the automated analysis system 1000.

In another embodiment of the present disclosure, a plurality of analysis devices 1200 may be operatively connected and applied to the automated analysis system 1000.

A reaction vessel 1500 for receiving the analysis sample prepared in the preparation device 1100 may be mounted on the analysis device 1200.

In an embodiment of the present disclosure, the analysis device 1200 may be mounted with the reaction vessel 1500 whose upper surface is sealed by the automatic sealer 1700. To this end, the reaction vessel 1500 may be moved from the preparation device 1100 to the automatic sealer 1700, and may be mounted on the analysis device 1200 after the sealing of the upper surface is completed.

The analysis device 1200 is provided with a sample holder 1210 in which the reaction vessel 1500 is accommodated.

The analysis device 1200 may be provided with a sample holder and a lid 1220 for protecting the reaction vessel 1500 accommodated in the sample holder. The analysis device 1200 is provided with the lid 1220 open prior to receiving the reaction vessel 1500. The lid 1220 of the analysis device 1200 is closed after the reaction vessel 1500 is received.

When the analysis device 1200 operates as a stand-alone device, the lid 1220 may be opened or closed by a user's command input.

When operatively connected to the automated analysis system 1000, the analysis device 1200 may be configured such that the lid 1220 is opened or closed by the control module of the automated analysis system 1000.

In an embodiment of the present disclosure, the reaction vessel 1500 may be provided in the form of an amplification multi-well plate including a plurality of analysis samples to be analyzed in each of a plurality of wells. In this case, the sample holder may accommodate one amplification multi-well plate. If necessary, the well plate includes n X m wells (n and m are natural numbers of 2 or more). The well plate may have a rectangular shape in which n X m wells are arranged in rows and columns. For example, 16 wells of 4 by 4 are shown. A well plate having n X m wells may be mounted in the sample holder.

Various examples of well plates are as follows.

The well plate may include 4 wells of 2 X 2, 9 wells of 3 X 3, 16 wells of 4 X 4, 25 wells of 5 X 5, 36 wells of 6 X 6, 49 wells of 7 X 7, or 64 wells of 8 X 8, etc. Also, the well plate may include 8 wells of 2 X 4, 18 wells of 3 X 6, 32 wells of 4 X 8, 50 wells of 5 X 10, 72 wells of 6 X 12, 98 wells of 7 X 14, or 128 wells of 8 X 16, and the like. Also, the well plate may include 12 wells of 2 X 6, 27 wells of 3 X 9, 4 X 12, 48 wells of 5 X 15, 75 wells of 6 X 18, 108 wells of 7 X 21, 147 wells of 8 X 24, or the like. Also, the well plate may include 16 wells of 2 X 8, 36 wells of 3 X 12, 64 wells of 4 X 16, 100 wells of 5 X 20, 144 wells of 6 X 24, 196 wells of 7 X 28, or 256 wells of 8 X 32, and the like. In addition, the well plate may include 96 wells of 8 X 12, 192 wells of 12 X 16, or 384 wells of 16 X 24, etc.

In another embodiment of the present disclosure, the reaction vessel may be provided with one or more independent reaction vessels. In this case, the sample holder may receive one or more of each reaction vessel.

In another embodiment of the present disclosure, the reaction vessel may be provided in the form of a strip tube to which two or more sample vessels are connected. In this case, the sample holder may accommodate at least one reaction vessel in the form of a strip tube.

In an embodiment of the present disclosure, at least one analysis device 1200 receiving the sealed reaction vessel 1500 in the automatic sealer 1700 may be provided in the enclosure 1300. That is, referring to FIG. 4, two analysis devices 1200-a and 1200-b may be provided inside the enclosure 1300.

In an embodiment of the present disclosure, when two analysis devices are provided in the enclosure 1300, the preparation device 1100 sequentially prepares analysis samples for analysis. When the first reaction vessel is prepared in the preparation device 1100, the first half vessel is moved so that any one analysis device in the enclosure 1300 performs analysis of the first reaction vessel.

When the second reaction vessel is prepared in the preparation device 1100, another analysis device in the enclosure 1300 moves the second reaction vessel to perform analysis of the second reaction vessel.

In another embodiment of the present disclosure, when two analysis devices are provided in the enclosure 1300, the preparation device 1100 simultaneously or sequentially prepares analysis samples for analysis. When the first reaction vessel and the second reaction vessel are prepared in the preparation device 1100, each reaction vessel 1500 is sequentially moved to the enclosure 1300, and when the first reaction vessel is mounted on any one analysis device 1200-a, the second reaction vessel is mounted on the other analysis device 1200-b.

FIG. 5 is a perspective view illustrating a stand-alone preparation device according to an embodiment of the present disclosure. As shown in FIG. 5, the preparation device 1100 is a stand-alone device. That is, the preparation device 1100 may be installed and operated alone to prepare the analysis sample.

According to one embodiment of the present disclosure, the preparation device 1100 may be operatively connected to the analysis device 1200 and/or the enclosure 1300 and used as the automated analysis system 1000.

The preparation device 1100 may include a nucleic acid extraction module and/or a liquid handling module for extracting nucleic acid from the analyte.

In one embodiment of the present disclosure, the preparation device 1100 includes a nucleic acid extraction module and a liquid handling module.

In another embodiment of the present disclosure, the preparation device 1100 includes a nucleic acid extraction module.

In another embodiment of the present disclosure, the preparation device 1100 includes a liquid handling module.

In the present disclosure, the nucleic acid extraction module and/or the liquid handling module included in the stand-alone preparation device 1100 may be operatively connected to the automated analysis system 1000 without being deformed.

In addition, when the stand-alone preparation device 1100 is operatively connected to the automated analysis system 1000, the preparation device 1100 may use a reagent container in the form of tube that is previously used.

In an embodiment of the present disclosure, when the preparation device 1100 is used as the automated analysis system 1000, the first passthrough cavity 1130 formed in the preparation device 1100 may be used as a defined passage through which the reaction vessel 1500 is transported by the transport device 1400. Therefore, in the present specification, the first passthrough cavity and the first defined passage may be mixed.

As shown in FIG. 5, the first defined passage 1130 is formed on the bottom surface of the preparation device 1100, but the first defined passage 1130 may be formed on the side surface (including the front, rear, left, and right) or may be previously formed on the upper surface according to the type of the preparation device 1100.

In another embodiment of the present disclosure, when the preparation device 1100 is used as the automated analysis system 1000, the preparation device 1100 may form a first defined passage 1130, which is a defined passage through which the reaction vessel may be transported by the transport device 1400.

The first defined passage of the preparation device 1100 may be formed on any one of an upper surface, a lower surface, and a side surface (including front/rear/left/right).

The first defined passage 1130 is formed to have a size to allow the reaction vessel 1500 and the transport device 1400 for transporting the reaction vessel to move.

In an embodiment of the present disclosure, when one analysis device 1200 is provided as the analysis device used in the automated analysis system 1000, the preparation device 1100 may sequentially prepare the analysis samples one by one and provide the prepared analysis samples after the analysis is completed in the analysis device 1200.

In another embodiment of the present disclosure, when two or more analysis devices 1200 used in the automated analysis system 1000 are provided, the preparation device 1100 sequentially prepares each analysis sample to be provided to each analysis device and provides the prepared analysis sample to any one analysis device. Thereafter, the next analysis sample prepared in the preparation device may be provided to another analysis device.

In another embodiment of the present disclosure, when two or more analysis devices 1200 used in the automated analysis system 1000 are provided, the preparation device 1100 prepares each analysis sample to be provided to each analysis device to be equal to or less than the number of analysis devices. Thereafter, a plurality of prepared analysis samples may be provided to each corresponding analysis device.

The preparation device 1100 includes a deck 1110 that can hold various kinds of instruments and containers for preparing the analytical sample. The deck 1110 is formed in a form in which components included in the preparation device 1100 may be mounted and fixed.

In one embodiment of the present disclosure, the deck 1110 provides a guide for the components of the preparation device 1100 to be inserted into the interior of the preparation device 1100 in a sliding manner and positioned on top of the deck 1110.

The guide is an embodiment of the deck 1110 and may be provided in the form of another embodiment.

In one embodiment of the disclosure, one or more components located on the deck 1110 may be secured during operation of the preparation device 1100 by projections and/or grooves formed on the underside of each component, etc.

The preparation device 1100 may include a loading tray 1120 in a plane extending from the deck 1110. The loading tray 1120 is installed to extend from the deck 1110 so that components mounted in the preparation device 1100 can be easily moved into the preparation device 1100. The loading tray 1120 may include a guide extending or connected to the guide of the deck 1110. The components mounted on the preparation device 1100 may be easily moved and mounted by the guide of the deck 1110 and the guide of the loading tray 1120.

In one embodiment of the present disclosure, the preparation device 1100 is provided with a pipette module (not shown) comprising pipette arms for dispensing liquid and one or more pipetting channels connected to the pipette arms. The pipette module is provided at an upper portion inside the preparation device 1100.

In addition, a transfer module (not shown) for transporting various containers used for sample preparation including a reaction vessel in the preparation device 1100 is provided at one side of the inside of the preparation device 1100.

In another embodiment of the present disclosure, the transfer module is configured on the upper side of the interior of the preparation device 1100 together with the pipette module.

In another embodiment of the disclosure, the transfer module is implemented by a pipetting channel of the pipetting module and a gripper (not shown) coupled to the pipetting channel.

Each component located in the deck 1110 of the preparation device 1100 is positioned at a predetermined location for preparation of the analysis sample.

A first passthrough cavity 1130 is formed in the deck 1110. The first passthrough cavity 1130 is a space in which the transport device 1400 for receiving the reaction vessel 1500 prepared in the preparation device 1100 is moved. The first passthrough cavity 1300 is sized to allow the transport device 1400 to move by receiving the reaction vessel 1500.

In an embodiment of the present disclosure, each component located in the deck 1110 may be described as follows. The components described below are generally included in the preparation device 1100 and used to prepare an analysis sample, but any one or more components may not be included according to a type of an individually operated stand-alone device. Alternatively, it may be used as a separate device.

All components of the preparation device 1100 are designed as an integrated device. The preparation device 1100 includes a nucleic acid extraction module for extracting nucleic acid of a sample and various components for amplification reaction setup (e.g., PCR setup).

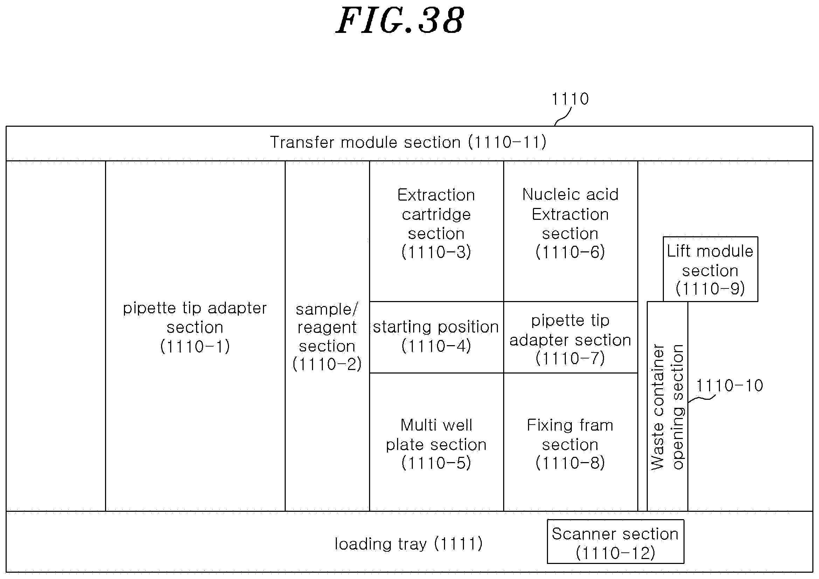

The inside of the preparation device 1100 according to one embodiment of the present disclosure may include a pipette tip adapter, a container carrier, a nucleic acid extraction module, a multi-well plate adapter, a scanner, a waste liquid inlet, a transfer module, a pipette module, and the like. FIG. 38 is a layout diagram illustrating that the components of the preparation device according to an embodiment of the present disclosure are located on the deck.
1) A pipette tip adapter holds one or more pipette tips coupled to a pipetting channel. The pipette tip may be coupled to the pipetting channel to aspirate and dispense a solution, such as a specimen or reagent, contained in the container.

The one or more pipette tips accommodated in the pipette tip adapter may be provided to have different sizes and dispensing amounts according to a preparation operation environment such as a size of a container and a volume of a dispensed solution.

In one embodiment of the present disclosure, the pipette tip adapter of the device may be provided in a plurality to accommodate tips of various capacities such as 1mℓ, 500µℓ, 300µℓ, 250µℓ, 200pP, 150µℓ, 100µℓ, and/or 50. Further, any one or more of the tips of various capacities may be piercing tip.

Each pipette tip adapter can receive one or more pipette tips, and the pipette module positions the pipetting channel over the pipette tip adapter and then moves it in the direction of the pipette tip so that the pipetting channel can engage the pipette tip.

The number of pipette tip adapters and each capacity and size of a pipette tip accommodated in the pipette tip adapter may be modified or changed according to various embodiments of the present disclosure.

2) The container carrier includes various containers for containing various kinds of solutions used in the preparation device. The preparation device may prepare an analysis sample including the extracted nucleic acid using a nucleic acid extraction module. Various types of containers are used for the operation of the preparation device, and the container carrier may be inserted with containers except for the well plate.

The container carrier may be provided in various forms to allow each container to be easily inserted and fixed according to a capacity and/or a size of a container to be inserted.

In one embodiment of the present disclosure, the inserted container includes a container for accommodating the specimen, a container for accommodating the extraction reagent, and a container for accommodating the reaction reagent. The container carrier may insert containers in a row or in parallel.

The container carrier may have a passthrough cavity in the side to allow the identification code printed or attached to the container to be exposed. Accordingly, the scanner located in the deck 1110 may recognize the exposed identification code.

3) The nucleic acid extraction module automatically performs a detection sample preparation process used for detecting a target nucleotide sequence in a preparation device.

In the present disclosure, the sample preparation process for detection includes a process of extracting nucleic acid from a specimen, preparing a reaction mixture for amplification, and preparing a detection sample in which the reaction mixture and the extracted nucleic acids are mixed.

If a nucleic acid extraction module is not included in the preparation device, the sample may be a nucleic acid obtained through a nucleic acid extraction process in advance.

In another embodiment, the nucleic acid extraction is frequently performed by a magnetic bead-based method using magnetic beads that bind to a nucleic acid and are capable of eluting the bound nucleic acid. The magnetic bead-based automated nucleic acid extraction method may use a liquid transfer method or a bead transfer method according to the type of the process of eluting the nucleic acid bound to the magnetic bead.

4) The multi-well plate adapter is a structure in which a reaction vessel for accommodating a specimen to be detected may be located, and the reaction vessel may be mounted on a sample holder of an analysis device.

The multi-well plate adapter may load a reaction vessel (multi-well plate), and a sample for detection may be dispensed to the reaction vessel (multi-well plate) positioned in the multi-well plate adapter. In the multi-well plate adapter, two or more multi-well plates used in the preparation device may be stacked. In an embodiment of the present disclosure, the term "multi-well plate" may be used as a reaction vessel adapter.

At this time, any one of the plurality of multi-well plates provided in the multi-well plate adapter may be moved to the starting position and used in an operation for preparing an analysis sample. The multi well plate is moved to the starting position by the transfer module.

5) Various containers may be mounted on the fixing frame directly or through an adapter or the like. The container may contain an analysis sample or extraction reagent. The container is equipped with a cap, which can be a pierceable cap by a pipette tip. The penetrating portion of the cap may be made of a material such as silver rubber, silicon, plastic, or the like.

The pipette tip perforates the upper portion of the cap by a descending operation, performs aspirating or dispensing of the solution, and then moves upward again. When the pipette tip moves upward from the container, the container needs to be fixed because the container can be lifted together upward by the pipette tip inserted into the perforated part of the cap. The fixing frame can fix the container so that the container using pierceable cap is not moved by the pipette tip.

6) The first passthrough cavity 1130 is a defined passage through which the transport device 1400 is transported to the preparation device 1100 to receive the reaction vessel 1500.

In an embodiment of the present disclosure, the first passthrough cavity 1130 is an empty space. The transport device 1400 is moved from a lower portion of the preparation device 1100 to an inside of the preparation device 1100. Therefore, the first passthrough cavity 1130, which is an empty space, may be formed in the deck 1110, which is the lower surface of the preparation device 1100, or may be formed.

In another embodiment of the present disclosure, the first passthrough cavity 1130 includes an open/close module (not shown). The open/close module is provided to block an opened space, which is the first passthrough cavity 1130, into which the transport device 1400 enters. The transport device 1400 is moved to the preparation device 1100 to receive the reaction vessel prepared in the preparation device 1100. The preparation device 1100 may block the first passthrough cavity 1130 by using an open/close module for closing the opened space at a time when the transport device 1400 does not move.

7) The waste part comprises a waste liquid inlet and/or a waste pipette tip collecting unit. The recovery solution inlet can be collected such that the solution used for preparation of the analysis sample is discarded, and the pipette tip recovery can be collected such that the pipette tip used for preparation of the analysis sample is discarded.

In one embodiment of the present disclosure, the waste liquid inlet is connected to a separately positioned liquid waste collection bin (not shown). The waste solution of the preparation device 1100 is moved to be accommodated in the liquid waste collection bin through the retrieve solution inlet.

In addition, the pipette tip collected through the waste pipette tip collecting unit may be moved to the waste container and stored. In one embodiment of the present disclosure, the waste container may be located in the deck 1110 of the preparation device 1100. In another embodiment of the present disclosure, the waste container may be located on the bottom surface of the preparation device 1100. In another embodiment of the present disclosure, the waste container may be located outside the preparation device 1100.

When the waste container is located in the deck 1110, the waste container may be separated into an area where the analysis sample is prepared, a partition, and the like.

8) The transfer module is a mechanical device in the form of a gripper for moving the reaction vessel or the like in the preparation device 1100. The transfer module is operated by a control device of the preparation device 1100.

In one embodiment of the present disclosure, the transfer module is located on the inner rear side of the preparation device 1100. The transfer module is configured to move the reaction vessel up and down, left and right, back and forth, and rotation.

In another embodiment of the present disclosure, the transfer module is located at an inner upper portion of the preparation device 1100. The transfer module is configured to move the reaction vessel up and down, left and right, forward and backward, and rotate through an operation type such as a pipette module.

In another embodiment of the present disclosure, the transfer module is configured to move the reaction vessel up and down, left and right, back and forth, and rotation using a gripper coupled to at least two pipetting channels of the pipetting channels of the pipetting module.

The transfer module may move components necessary for preparation of an analysis sample, such as a reaction vessel, a reagent container, an adapter, a cartridge, a multi-well plate, and the like, within the preparation device 1100.

In an embodiment of the present disclosure, the transfer module may move the reaction vessel in which the setup of the analysis sample is completed to the transport device 1400. The transport device 1400 is moved through the first passthrough cavity 1130 of the preparation device 1100 to receive a reaction vessel of which preparation is completed. The transfer module may move the reaction vessel to the transport device 1400, and the transport device 1400 may move the reaction vessel to the outside of the preparation device 1100 through the first passthrough cavity 1130.

9) The scanner may read an identifying code displayed on a specimen, a reagent, a reaction mixture, etc. The identification code is a display including information such as a barcode and a matrix code. The scanner may recognize the identification code to receive information such as the type and capacity of the solution contained in the container.

In one embodiment of the present disclosure, a plurality of scanners may be provided and may be composed of any one scanner as necessary. Further, the scanner may be configured as a barcode scanner and/or a 2D scanner. It is preferable that such a configuration is provided so as to be able to recognize different types of identification codes marked on a reaction vessel or the like.

In one implementation of the disclosure, the scanner may recognize 1D and/or 2D barcodes. The scanner may recognize an identification code printed or attached to the side of the container to obtain information such as the type and/or capacity of the solution contained in the container. The container includes a reaction vessel, a reagent container, and a specimen container used in a preparation device. The scanner may recognize the identification code of the container inserted into the deck 1110. The scanner may sequentially recognize at least one container identification code inserted into the deck 1110. The scanner may move to a position where the container or the carrier receiving the container is coupled to the deck 1110 to recognize the identification code of the container side.

In another implementation of the disclosure, the scanner is a 2D barcode scanner (not shown). Matrix (two-dimensional) codes can be recognized, and identification codes printed or attached to the bottom surface of the container can be recognized. The container includes a container used in a preparation device such as a reaction vessel, a reagent container, and a specimen container.

Accordingly, when a plate formed to have a passthrough cavity in all or a part of the bottom surface of each well is mounted on the scanner, the scanner can recognize an identification code printed or attached to the bottom surface of the container inserted into the plate. The scanner may recognize the codes of a plurality of containers inserted into the plate at once.

In one embodiment of the present disclosure, the plane on which the container is mounted in the scanner is made of a transparent material. The scanner may recognize the identification code of the bottom of the container using an optical signal that transmits a transparent material. In addition, the scanner may recognize the identification code of the container by photographing the lower portion of the container. The scanner has a form in which the multi-well plate may be mounted so that the identification code located on the bottom surface of the container inserted into the multi-well plate may be recognized.

The 2D scanner according to an embodiment of the present disclosure may use "easyCode Carrier" product of Hamilton (see https://www.hamiltoncompany.com/automated-liquid-handling/small-devices/ea sycode-ca rrier).

10) Although not shown in the drawings, the pipette module is positioned at an inner upper portion of the preparation device 1100. A pipette module, which is a solution fractionator, includes a pipette arm and a pipetting channel, and the pipetting channel may be automatically moved up and down, left and right, and front and rear by a control device.

The pipette arm may include one or more pipetting channels that move independently or dependently. In one embodiment, a pipette tip or needle is coupled to the end of the pipetting channel and can be used for aspirate and dispense of the solution.

In another embodiment, a gripper may be coupled to an end of the pipetting channel, and may be used as a transfer module capable of moving a vessel (including a reaction vessel), or the like, used in the preparation device 1100 for the reaction vessel, or the like, by the gripper.

The pipette arm may perform an operation of moving one or more pipetting channels to a fractionating pipette tip, an operation of fixing the fractionating pipette tip to the pipetting channel, an operation of moving the pipetting tip to which the pipetting channel is fixed to a predetermined place, an operation of inserting the fractionating pipette tip into a container at a predetermined depth, or the like.

The pipette arm is placed inside the preparation device 1100 and the pipetting channel is operated within the preparation device 1100 by the pipette arm. The one or more pipetting channels couple the pipette tip inserted into the pipette tip adapter to an end of the pipetting channel.

The pipette arm may move the pipetting channel to be located above the container in which the solution to be dispensed is contained. The pipetting channel is lowered in the direction of the container in the moved position to accommodate the solution in the pipette tip and is positioned to rise again. The pipetting channel can end the dispensing by moving to the top of another container to be dispensed by the pipette arm, and descending to dispense the solution received in the pipette tip and then rising again.

The plurality of pipetting channels may be simultaneously operated, and the number of containers that can be simultaneously dispensed may be determined according to the number of pipetting channels.

The pipette arm and pipetting channel may remove the pipette tip coupled to the end after the dispensing is complete. The bonded pipette tips are removed from the waste pipette tip collecting unit located in the waste unit and can be discarded in the waste container.

In an embodiment of the present disclosure, each device must be accurately positioned so that at least one of the preparation device 1100, the analysis device 1200, and/or the transport device 1400 is operatively connected to the enclosure 1300. To this end, the enclosure 1300 and each device include a positioning means.

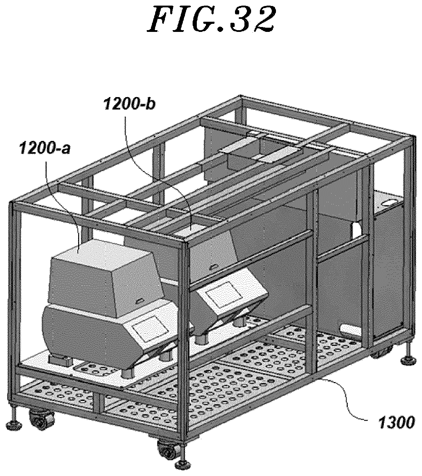

Since the preparation device 1100, the analysis device 1200 and/or the transport device 1400 should not be externally deformed and should be coupled to the enclosure 1300, it is preferable that the positioning means of each device use a component previously formed in each device. This is described with reference to FIGS. 32 to 34.

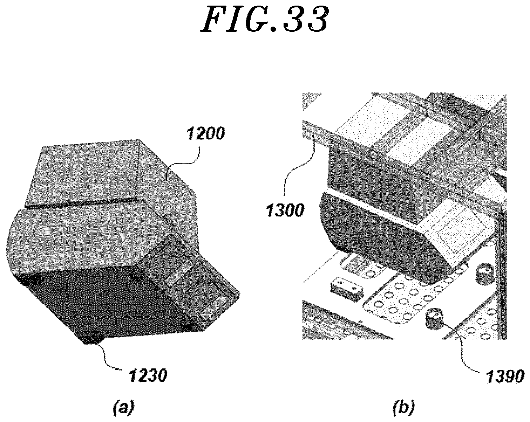

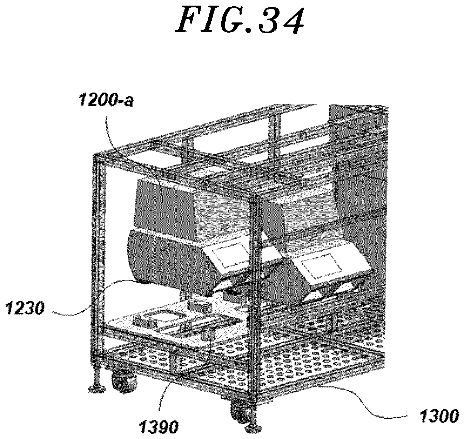

FIG. 32 is an exemplary diagram illustrating that an analysis device is mounted in an enclosure using a positioning means according to an embodiment of the present disclosure. FIG. 33 is a first exemplary diagram illustrating a positioning means according to an embodiment of the present disclosure. FIG. 34 is a second exemplary diagram illustrating a positioning means according to an embodiment of the present disclosure. As shown in FIGS. 32 to 34, at least one of the preparation device 1100, the analysis device 1200, and/or the transport device 1400 is spatially closed, and the enclosure 1300 is used as a configuration for spatially closing.

At least one of the preparation device 1100, the analysis device 1200, and/or the transport device 1400 is configured to be positioned within the enclosure 1300 in order to operatively connect the at least one or more.

FIG. 32 shows that the analysis device 1200-a, 1200-b are located in an enclosure 1300, according to one embodiment of the present disclosure. In this case, in the analysis devices 1200-a and 1200-b, the reaction vessel 1500 in which the analysis sample is accommodated in the preparation device 1100 located outside the enclosure 1300 may be moved through the transport device 1400.

In particular, the crane module 1430 of the transport device 1400 provides the reaction vessel 1500 to the analysis devices 1200-a and 1200-b. The crane module 1430 may move to a designated position as a robotic module to lift the reaction vessel 1500, and may put the reaction vessel 1500 down at a designated place.

If the positions of the analysis devices 1200-a and 1200-b deviate from the predetermined position even by a little, the reaction vessel 1500 is not accurately positioned, and thus an appropriate analysis cannot be performed. Thus, the analysis devices 1200-a, 1200-b must be accurately positioned or coupled at a predetermined location within the enclosure 1300. To this end, the enclosure 1300 provides a positioning means.

As shown in FIGS. 33 and 34, the positioning means may be located at the analysis device 1200 and the enclosure 1300.

In one embodiment of the present disclosure, the first positioning means 1230 of the analysis device 1200 may be a fixing means located at the lower portion. Also, the second positioning means 1390 of the enclosure 1300 may be a structure formed at a place where the analysis device 1200 is located.

The first positioning means 1230 and the second positioning means 1390 may be formed in a shape capable of being coupled to each other.

According to an embodiment of the present disclosure, even if the first positioning means 1230 and the second positioning means 1390 do not have separate fastening means, the analysis device 1200 may be fixed at an accurate position of the enclosure 1300.

According to another embodiment of the present disclosure, the first positioning means 1230 and the second positioning means 1390 are provided with mutual fastening means (not shown) to fasten the respective positioning means with the fastening means when the analysis device 1200 is fixed to the correct position of the enclosure 1300.

Although FIGS. 32 to 34 illustrate the positioning means between the analysis device 1200 and the enclosure 1300, it is preferable that the positioning means matching the enclosure is formed in each device located inside the enclosure 1300 such as the transport device 1400 and the automatic sealer 1700 according to an embodiment of the present disclosure.

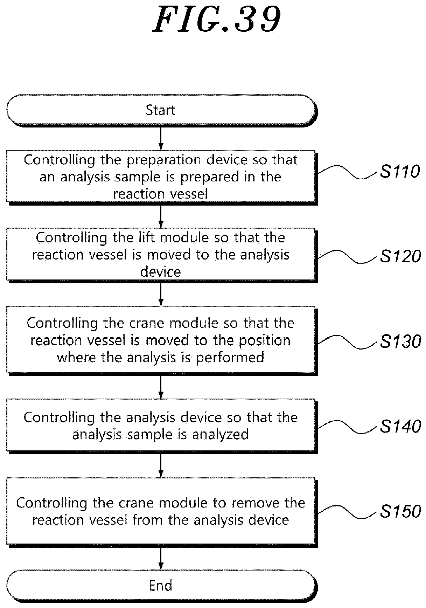

FIG. 39 is a flowchart illustrating an operating method of the automated analysis system according to an embodiment of the present disclosure. As shown in FIG. 39, the automated analysis system of the present disclosure consists of an enclosure that includes a preparation device, an analysis device and/or a transport device. Each of the preparation device and the analysis device included in the automated analysis system may be operated as a stand-alone device.

The automated analysis system may position at least one device selected from the group consisting of a preparation device, an analysis device, and a transport device inside the spatially enclosure.

As in one embodiment of the present disclosure, the automated analysis system is configured such that an analysis device is provided inside an enclosure and a preparation device is positioned on the enclosure.

The automated analysis system includes a control module. The control module controls the preparation device to prepare an analysis sample. The control module controls the enclosure to analyze the prepared analysis sample.

A method in which the control module of the automated analysis system controls the preparation device and the enclosure to prepare and analyze the analysis sample is as follows.

The control module controls the preparation device so that an analysis sample is prepared in the reaction vessel provided in the preparation device (S110).

The preparation device prepares a solution such as a specimen, a nucleic acid extraction reagent, an amplification reaction reagent, and the like so as to prepare an analysis sample, and prepares the analysis sample using internal components.

In step S110, a preparation device may be used as a control module to prepare an analysis sample.

The preparation device may perform at least one preparation of: preparing the analysis sample by performing a division of at least one of a specimen and a reagent; receiving the analysis sample in the reaction vessel; or extracting nucleic acid from the specimen expected to contain a pathogen.

The control module controls the lift module in the enclosure so that the reaction vessel prepared in the preparation device is moved to the enclosure (S120).

In step S120, the lift module moves into the preparation device through the second passthrough cavity formed in the enclosure and the first passthrough cavity formed in the deck of the preparation device. The preparation device mounts the reaction vessel to the moved lift module.

When the reaction vessel is mounted on the lift module, the control module controls the lift module to move to the enclosure.

The first passthrough cavity is a defined passage through which the reaction vessel can be conveyed.

In one embodiment of the present disclosure, the enclosure may include a plurality of analysis devices for analyzing an analysis sample.

The control module controls the preparation device so that analysis samples to be analyzed can be prepared simultaneously or sequentially using a plurality of analysis devices.

The control module controls the lift module so that the lift module can move the reaction vessel accommodating the analysis sample which is simultaneously or sequentially prepared in the preparation device to the enclosure.

In step S120, when the lift module is moved to the preparation device to receive the reaction vessel, the control module controls the lift module to perform an extension movement in a horizontal direction to easily receive the reaction vessel.

The control module controls the crane module such that the reaction vessel moved to the enclosure is moved to the position where the analysis is performed (S130).

The crane module may perform a movement operation for moving the reaction vessel moved to the enclosure in upward and downward, left and right directions, and forward and backward directions. The crane module may perform a rotation operation for horizontally rotating the reaction vessel.

In one embodiment of the present disclosure, the automatic sealer is included within the enclosure.

When the automatic sealer is provided in the enclosure, the control module controls the crane module to move the reaction vessel moved from the preparation device to the automatic sealer. The automatic sealer may seal the upper surface of the reaction vessel.

The control module controls the crane module so that the reaction vessel sealed in the automatic sealer is moved to a position where the analysis of the analysis sample is performed. A location where the analysis is performed is an analysis device.

In another embodiment of the present disclosure, the automatic sealer may be included in the preparation device.

When an automatic sealer is provided in the preparation device, the control module moves a reaction vessel prepared in the preparation device to the automatic sealer. The control module controls the automatic sealer to seal the upper surface of the moved reaction vessel.

When the automatic sealer is provided in the preparation device, in step S120, the lift module may receive a reaction vessel in which sealing is completed.

In an embodiment of the present disclosure, when a plurality of analysis devices is provided and a plurality of reaction vessels are sequentially received in the preparation device, the control module controls the crane module to move each of the sequentially moved analysis sample containers to the analysis device.

The control module controls the enclosure so that the analysis sample accommodated in the reaction vessel in the enclosure is analyzed (S140).

In step S140, the reaction vessel may be moved to the analysis device by the crane module. The analysis device analyzes an analysis sample of the moved reaction vessel and generates a result.

In step S140, the enclosure in which the control module controls the enclosure such that analysis of the analysis sample is performed is an analysis device.

The analysis device may perform at least one of a process of performing a polymerase chain reaction and a process of performing analysis on a reaction result.

The analysis device is provided at a position where the analysis is performed.

The analysis device includes a thermal cycler and an optics module. A process of performing the polymerase chain reaction in the analysis device uses a thermal cycler, and a process of measuring a reaction result uses an optical module.

The analysis device may be provided with a cover for analysis of the analysis sample. The control module may provide a control signal to the analysis device so that a cover of the analysis device to which the reaction vessel is to be moved is opened. Thereafter, when the crane module provides the reaction vessel to the analysis device, the control module may provide a control signal to close the lid of the analysis device.

The control module controls the crane module to remove the reaction vessel of which the analysis is completed from the position where the analysis is performed (S150).

In step S150, the control module controls the crane module so that the reaction vessel of which the analysis has been completed can be moved to a reaction vessel retrieval container.

In accordance with one aspect of the present disclosure, the present disclosure provides an automated analysis system including a memory, at least one processor configured to access the memory, and one or more programs stored in the memory and configured to be executed by the processor. The automated analysis system includes a preparation device, an analysis device, a transport device, a control module and an enclosure, the one or more programs including instructions that, when executed by the one or more processors, cause the one or more programs to perform the following steps: the control module controls the transport device to transport a reaction vessel containing an analysis sample from the preparation device to the analysis device; the preparation device and the analysis device being stand-alone devices; the control module controls the analysis device to analyze the analysis sample in the analysis device; the control module controls the transport device to remove the reaction vessel from which analysis of the analysis sample is completed from the analysis device; at least one device selected from the group consisting of the analysis device and the transport device is located inside an enclosure, the preparation device and the enclosure each including a defined passage through which the reaction vessel is transported, the one or more programs including instructions that cause the transport device to carry out the transport of the reaction vessel through the defined passage.

Each component described in the exemplary embodiment of the present disclosure is the same as the automated analysis system described in FIGS. 1 to 39, and thus a description thereof will be omitted.

According to one aspect of the present disclosure, there is provided a non-transitory computer readable storage medium having instructions, when executed by one or more processors, that cause the automated analysis system to perform an analysis method using an automated analysis system, the automated analysis system including a preparation device, an analysis device, a transport device, a control module and an enclosure, the instructions including instructions that cause the control module to control the transport device to transport a reaction vessel containing an analysis sample from the preparation device to the analysis device, the preparation device and the analysis device being stand-alone devices, the control module controlling the analysis device to analyze the analysis sample in the analysis device, and the control module controlling the transport device to remove the reaction vessel from the analysis device after analysis of the analysis sample is completed, at least one device selected from the group consisting of the analysis device and the transport device being located inside an enclosure, the preparation device and the enclosure each including a defined passage through which the reaction vessel is transported, and the instructions cause the transport device to perform the transport of the reaction vessel through the defined passage.

Each component described in the exemplary embodiment of the present disclosure is the same as the automated analysis system described in FIGS. 1 to 39, and thus a description thereof will be omitted.

### Fan module control and signal transmission

A RT-PCR device is disposed inside the automated analysis system, and heat is generated in the RT-PCR device during operation, and the heat needs to be discharged out from the inside of the automated analysis system. Accordingly, a technology for discharging heat out from the automated analysis system, for example, a temperature control technology, is also included in the present problem.

In addition, when the above-described temperature control technology is performed, if the target nucleic acid in the sample floats in the air in the above-described automated analysis system, another sample may be contaminated due to the floating. Accordingly, a technique for preventing contamination between samples when performing such a temperature control technique is also included in the present subject.

An automated analysis system according to an embodiment includes: a preparation device for preparing an analysis sample in a reaction vessel; an analysis device for analyzing the analysis sample prepared in the reaction vessel; a transport device for transporting the reaction vessel; an enclosure; an gate part for opening and closing the defined passage; a control module; and a fan module operated to discharge air out from an internal space of the enclosure, wherein at least one device selected from the group consisting of the analysis device and the transport device is located inside the enclosure, and each of the preparation device and the enclosure includes a defined passage through which the reaction vessel is transported.

The control module may include a control unit and a storage unit.

The defined passage may be opened while the transport device passes through the defined passage.

In addition, the defined passage may be opened while the transport device is located in the preparation device.

The automated analysis system may further include a transport device configured to receive a predetermined unsealed reaction vessel from the preparation device and transport the vessel to the analysis device, wherein the control unit may operate or stop the fan module according to a position or a moving direction of the transport device.

In addition, the control unit may stop an operation of the fan module while the transport device passes through the defined passage.

The control unit may stop an operation of the fan module while the transport device is located in the preparation device.

In addition, the control unit may stop the operation of the fan module when the transport device in the internal space starts to move toward the defined passage.

The control unit may stop an operation of the fan module while the transport device moves toward the analysis device.

In addition, the control unit may resume the operation of the fan module after the transport device has arrived at the analysis device and has completed the delivery of the container to the analysis device.

Further, the automated analysis system may further include an automatic sealer performing a sealing operation on the unsealed container, wherein the transport device may receive the unsealed container from the preparation device and transport the unsealed container to the automatic sealer, and receive the container on which the sealing operation is completed from the automatic sealer and transport the container to the analysis device, and the period in which the control unit stops the operation of the fan module may include a period from when the transport device receives the unsealed container from the preparation device to when the transport device transports the container on which the sealing operation is completed to the analysis device.

The automated analysis system may further include a plurality of fan modules, the automated analysis system may further include a temperature measurement unit configured to measure a temperature of the internal space, and the control unit may operate only some of the plurality of fan modules when the temperature measured by the temperature measurement unit is equal to or lower than a predetermined temperature, stop remaining fan modules, and operate all of the plurality of fan modules when the measured temperature exceeds the predetermined temperature.

The automated analysis system may further include a temperature measurement unit configured to measure a temperature of the internal space, and the control unit may control a rotation speed of a fan included in the fan module according to the temperature measured by the temperature measurement unit when the fan module is operating.

The preparation device may include a preparation device management unit configured to manage performance of the preparation job, and the analysis device may include an analysis device management unit configured to control performance of the detection job.

According to an embodiment of the present disclosure, a method for controlling a fan module performed in an automated analysis system includes: a preparation device for preparing an analysis sample in a reaction vessel; an analysis device for analyzing the analysis sample prepared in the reaction vessel; a transport device for transporting the reaction vessel; an enclosure; a gate part for opening and closing a defined passage; and a fan module operated to discharge air out from an internal space of the enclosure, wherein at least one device selected from the group consisting of the analysis device and the transport device is located inside the enclosure, wherein the preparation device and the enclosure each include a defined passage through which the reaction vessel is transported, and wherein the method for controlling the fan module includes: a step of opening the defined passage by the gate part; and a step of stopping an operation of the fan module while the defined passage is opened by the gate part.

In the opening step, the defined passage may be opened while the transport device passes through the defined passage.

In addition, in the opening step, while the transport device is located in the preparation device, the defined passage may be opened.

The automated analysis system may further include a transport device configured to receive a predetermined unsealed reaction vessel from the preparation device and transport the vessel to the analysis device, wherein the stopping step may include operating or stopping the fan module according to a position or a moving direction of the transport device.

Also, in the stopping step, the operation of the fan module may be stopped while the transport device passes through the defined passage.

The stopping step may include stopping an operation of the fan module while the transport device is located in the preparation device.

In the stopping step, when the transport device in the internal space starts to move toward the defined passage, the operation of the fan module may be stopped.

In the stopping step, the operation of the fan module may be stopped while the transport device is moved to the analysis device.

The method may further include resuming the operation of the fan module after the transport device completes the delivery of the container to the analysis device after arriving at the analysis device.

Further, the automated analysis system may further include an automatic sealer performing a sealing operation on the unsealed container, wherein the transport device may receive the unsealed container from the preparation device and transport the unsealed container to the automatic sealer, and receive the container on which the sealing operation is completed from the automatic sealer and transport the container to the analysis device, and the period in which the operation of the fan module is stopped may include a period from a time when the transport device receives the unsealed container from the preparation device to a time when the transport device transports the container on which the sealing operation is completed to the analysis device.

The automated analysis system may further include a plurality of fan modules, the automated analysis system may further include a temperature measurement unit configured to measure a temperature of the internal space, and the method of controlling the fan module may operate only some of the plurality of fan modules and stop the remaining fan modules when the temperature measured by the temperature measurement unit is equal to or lower than a predetermined temperature, and operate all of the plurality of fan modules when the measured temperature is higher than the predetermined temperature.

The method may further include: measuring a temperature of the internal space; and when the fan module is operating, controlling a rotation speed of a fan included in the fan module according to the temperature measured by the temperature measuring unit
According to an embodiment, a computer-readable recording medium includes a computer program, and the computer program may be programmed to perform each step included in the above-described method.

A computer program according to an embodiment may be stored in a computer-readable recording medium, and the computer program may be programmed to include each step included in the above-described method.

According to an embodiment of the method for controlling a pen module of the present disclosure, the risk of the pathogens that may be included in the reaction vessel being leaked and dispersed into the air by the operation of the fan module may be reduced.

Also, the possibility of mixing the air inside a preparation device for carrying out preparation for detecting the target nucleic acid and the air around an analysis device for carrying out detection of the target nucleic acid can be reduced.

FIG. 40 is a block diagram conceptually illustrating a configuration of an automated analysis system 2000 according to an embodiment. Referring to FIG. 40, the automated analysis system 2000 includes a preparation device 2100, an analysis device 2200, a transport device 2300, an automatic sealer 2400, a control module 2500, a fan module 2600, and a container processing module 2800. However, the block diagram illustrated in FIG. 40 is merely exemplary.

Here, the preparation device 2100 and the analysis device 2200 may be devices that can be stand-alone, that is, devices in the form of a stand-alone. Further, each of the devices 2100 and 2200 includes an application programming interface (API). The API may be used to control or monitor each of the devices 2100 and 2200 from the outside, or may be used by each of the devices 2100 and 2200 to monitor the external situation or to control or monitor the external device.

Here, since these devices 2100 and 2200 are equipment for diagnosis or examination, they may be used only when a license is obtained in a specific country. In addition, even after the license is obtained, if a major function, structure, or the like is changed or a feature is added, a situation in which the license needs to be obtained again may occur.

In this regard, since the API corresponds to a main function of the devices 2100 and 2200, when the API is changed or added, a situation in which a license needs to be obtained again may occur.

Meanwhile, the automated analysis system 2000 includes not only the preparation device 2100 and the analysis device 2200 in the form of the stand-alone described above, but also a transport device 2300, an automatic sealer 2400, a control module 2500, a fan module 2600, and a container processing module 2800 for transporting or processing a reaction vessel or transmitting and receiving data therebetween. In addition, the device 2100, 2200 or 2300 and the modules 2400 to 2600 and 2800 may be coupled to each other through assembly, and thus a molecular diagnostic system may be completed, and an automated analysis system according to an embodiment may be referred to as an "assembly type".

Meanwhile, according to an embodiment, the automated analysis system 2000 may further include a configuration not illustrated in FIG. 40 or may not include at least one of the configurations illustrated in FIG. 40. Also, each configuration of the automated analysis system 2000 may be connected differently from that shown in FIG. 40. Hereinafter, each configuration will be described in detail.

First, the preparation device 2100 is used in a preparation operation for an analysis sample. In this regard, the preparation device 2100 may be referred to as a sample preparation device.

The preparation of the analysis sample performed by the preparation device 2100 includes a nucleic acid extraction operation and a reaction mixture preparation operation for nucleic acid amplification, but is not limited thereto. For example, a nucleic acid extraction operation may not be included in the preparation operation of the analysis sample performed by the preparation device 2100 according to an embodiment. In this case, the above-described nucleic acid extraction operation may be performed by another configuration not shown in FIG. 40, rather than the preparation device 2100. However, the following description will be made on the premise that the nucleic acid extraction work is included in the preparation work of the analysis sample performed by the preparation device 2100.

Meanwhile, since the above-described nucleic acid extraction operation and the preparing operation for the reaction mixture are known in the art, a detailed description thereof will be omitted.

Referring to FIG. 40, the preparation device 2100 includes a preparation device hardware (HW) unit 2110 and a preparation device management unit 2120.

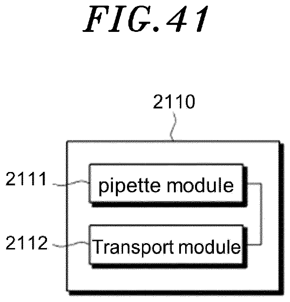

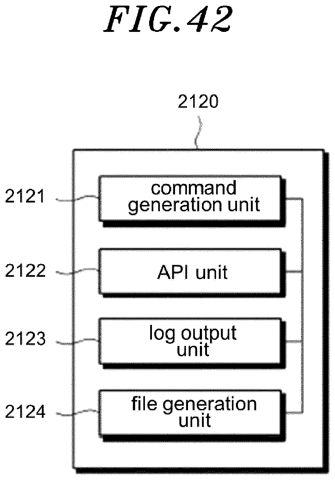

Here, the preparation device HW unit 2110 refers to a physically implemented equipment or structure. In addition, the preparation device management unit 2120 may generate a command for driving the preparation device HW unit 2110, drive the preparation device HW unit 2110 based on the generated command, output various messages or information, such as a driving result, as a log, and provide a predetermined application programming interface (API). The preparation device HW unit 2110 and the preparation device management unit 2120 themselves will be described below with reference to FIGS. 41 and 42.

FIG. 41 is a block diagram conceptually illustrating a configuration of the preparation device HW unit 2110 of the preparation device 2100. However, FIG. 41 is merely illustrative.

Referring to FIG. 41, the preparation device HW unit 2110 of the preparation device 2100 includes a pipette module 2111 and a transfer module 2112. In addition, according to an embodiment, the preparation device 2100 may further include a nucleic acid extraction module for extracting nucleic acids.

The pipette module 2111 is a module used in the above-described nucleic acid extraction operation and the reaction mixture preparation operation for nucleic acid amplification. The pipette module 2111 may include a pipette tip and an arm for moving the pipette tip. The pipette module 2111 may be referred to as a liquid handler.

Such a pipette module 2111 may automatically or programmatically aspirate and/or dispense a desired amount of reagent, analysis sample, or other liquid from a designated container for automation of a chemical or biochemical laboratory.

The transfer module 2112 transfers the reaction vessel to the transport device 2300. An analysis sample may be accommodated in the reaction vessel transported by the transfer module 2112. The analysis sample accommodated in the reaction vessel may be one in which each of the above-described nucleic acid extraction operation and the above-described preparing operation for the reaction mixture is completed.

Meanwhile, the transfer module 2112 described above may grip or place the reaction vessel, and may also move while gripping the reaction vessel. In this regard, the transfer module 2112 may be referred to as a gripper.

Although not shown in FIG. 41, the pipette module 2111, the transfer module 2112, or the nucleic acid extraction module may be disposed in an enclosure having a space therein. That is, the preparation device HW part 2110 may include the above-described enclosure as a configuration. If the above-described nucleic acid extraction operation or the above-described preparing operation for reaction mixture is performed in the enclosure of the preparation device 2100, various substances, for example, pathogens that may be included in the sample may be leaked into a space provided in the enclosure, and thus, may be floated in the space provided in the enclosure. Accordingly, according to an embodiment, a technology for reducing this risk is presented, which will be described later.

Here, each configuration illustrated in FIG. 41 is already known in the art, and a drawing of each structure thereof and a detailed description related thereto will be omitted.

Next, FIG. 42 is a block diagram conceptually illustrating the configuration of the preparation device management unit 2120 of the preparation device 2100. However, FIG. 42 is merely illustrative.

Referring to FIG. 42, the preparation device management unit 2120 of the preparation device 2100 includes a command generation unit 2121, an API unit 2122, a log output unit 2123, and a file generation unit 2124. Here, the preparation device management unit 2120 and each of the configuration 2121 to 2124 included therein may be implemented by a processor and a memory including instructions executable by the processor.

Here, the command generation unit 2121 is configured to generate a control command for controlling each of the configurations 2111 and 2112 shown in FIG. 41. The preparation device HW unit 2110 shown in FIG. 41 may be driven based on the generated control command.

The API unit 2122 is configured to provide a predetermined API. In some cases, the preparation device 2100 may monitor or control an external object by using an API provided by the API unit 2122. On the contrary, the external object may monitor or control the preparation device 2100 by using the API provided by the API unit 2122.

In an embodiment, the API provided by the API unit 2122 does not support a function of accessing the analysis device 2200 to be described below. However, the API supports a function of generating a file having a specific file name and a function of writing the generated file to a predetermined location. In this case, the file may include predetermined content. Here, the "content" includes a text, an image, a voice, or the like, which is written in a file, but is not limited thereto. In addition, an example of the predetermined position may be a file storage unit constituting the control module 2500, which will be described later, and the position may be a position which has been previously consulted with the analysis device 2200.

The log output unit 2123 is configured to output a state of the preparation device 2100 and other various information as a log. Examples of the output log may include state information indicating whether the preparation device 2100 is operating normally, operating abnormally, or stopped, a command to call the transport device 2300, a command to transport the reaction vessel to the transport device 2300, and the like, but are not limited thereto.

The file generator 2124 is configured to generate a file. The file includes an instruction that the analysis device 2200 refers to when performing a detection operation on a target nucleic acid molecule.

The instructions include, for example, at least one of information about a plate on which a plurality of wells are provided, information about a specimen included in each of the plurality of wells, information about a reagent included in each of the plurality of wells, information about an optical scan method performed during the detection operation, and a reaction condition during the detection operation, but are not limited thereto. Here, the "reaction condition" may be, for example, a PCR protocol, but is not limited thereto.

When the file generation unit 2124 generates the above-described file, a preparation operation for a target nucleic acid molecule performed by the preparation device 2100 may be used as a basis. That is, the contents of the above-described instructions may be determined according to which plate the pipette module 2111 of the preparation device 2100 performs a preparation operation, which specimen is included in each of a plurality of wells included in the corresponding plate, or which reagent is dispensed in each of the plurality of wells. Here, a subject for determining the contents of the instruction in detail may be the file generation unit 2124, and to this end, a table "when the contents of the preparation work are the same, the contents of the instruction should be different" may be prepared in advance in the file generation unit 2124. Alternatively, a subject that specifically determines the content of the instruction may be an operator of the automated analysis system 2000 according to an embodiment.

The file generated by the file generation unit 2124 may be written in a predetermined location by a function of writing the file in a predetermined location among functions of the API provided by the API unit 2122.

Also, the file generation unit 2124 may record the file name of the file at a predetermined location using the function of the API provided by the API unit 2122. Here, the predetermined location where the file name is recorded may be the file information storage unit 2522 of the control module 2500, which will be described later.

Referring back to FIG. 40, the analysis device 2200 will be described. The analysis device 2200 is used for qualitative or quantitative analysis of an analyte. In this regard, the analysis device 2200 may be referred to as a sample analysis device.

More specifically, the analysis device 2200 performs a nucleic acid amplification operation of amplifying a nucleic acid having a specific nucleotide sequence and a detection operation of detecting the amplified nucleic acid, but is not limited thereto.

Meanwhile, the above-described nucleic acid amplification operation and the above-described nucleic acid detection operation are known in the art, and thus additional description thereof will be omitted.

As shown in FIG. 40, the analysis device 2200 includes an analysis device hardware (HW) unit 2210 and an analysis device management unit 2220.

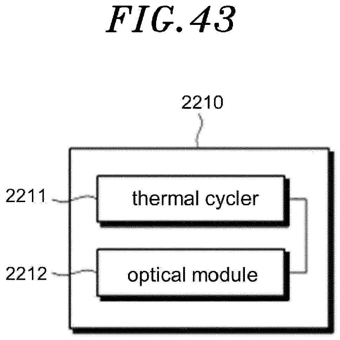

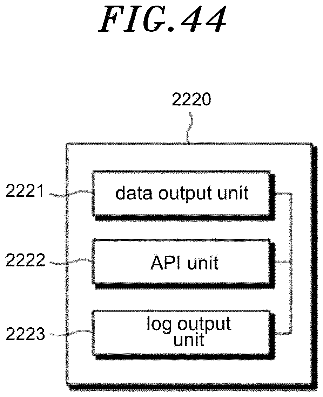

The present disclosure refers to an equipment or structure in which the analysis device HW unit 2210 is physically implemented. In addition, the analysis device management unit 2220 may drive the analysis device HW unit 2210, output a result of the driving or various other messages as a log, and provide a predetermined application programming interface (API). The analysis device HW unit 2210 and the analysis device management unit 2220 themselves will be described with reference to FIGS. 43 and 44

FIG. 43 is a block diagram conceptually illustrating a configuration of the analysis device HW unit 2210 of the analysis device 2200. However, FIG. 43 is merely illustrative.

Referring to FIG. 43, an analysis device HW part 2210 of an analysis device 2200 includes a thermal cycler 2211 and an optical module 2212.

The thermal cycler 2211 is configured to perform a nucleic acid amplification operation. Specifically, the thermal cycler 2211 is used for a polymerase chain reaction (PCR)-based nucleic acid amplification reaction. More specifically, the thermal cycler 2211 may perform a denaturing step, an annealing step, and an extension step in order to amplify DNA having a specific nucleotide sequence.

Among these, the denaturation step is a step of separating the double-stranded DNA into single-stranded DNA by heating a solution containing a sample containing the double-stranded DNA as a template nucleic acid and a reagent at a specific temperature, for example, about 95°C. The annealing step is a step of providing an oligonucleotide primer having a nucleotide sequence complementary to a nucleotide sequence of a nucleic acid to be amplified, cooling the isolated single-stranded DNA to a specific temperature, for example, 60°C, and binding the primer to a specific nucleotide sequence of the single-stranded DNA to form a partial DNA-primer complex. The extension step is a step of maintaining the solution at a specific temperature, for example, 72°C, after the annealing step to form double-stranded DNA based on primers of partial DNA-primer complexes by DNA polymerase.

In one embodiment, the thermal cycler 2211 may exponentially amplify the DNA having the particular nucleotide sequence by repeating the above-described three steps, for example, 10 to 50 times.

In another embodiment, the thermal cycler 2211 may perform the annealing step and the extension step simultaneously. In this case, the thermal cycler 2211 may complete one cycle (cycle) by performing two steps including a denaturation step and an annealing/extension step.

The optical module 2212 is implemented to perform a nucleic acid detection operation. The optical module 2212 may analyze (or monitor) the amplification reaction performed by the thermal cycler 2211 in real time. The optical module 2212 may include, for example, a plurality of light sources, an optical filter, a convex lens, a beam splitter, a photodetector, and the like.

Next, FIG. 44 is a block diagram conceptually illustrating the configuration of the analysis device management unit 2220 of the analysis device 2200. However, FIG. 44 is merely illustrative.

Referring to FIG. 44, the analysis device management unit 2220 of the analysis device 2200 includes a data output unit 2221, an API unit 2222, and a log output unit 2223. Here, the analysis device management unit 2220 and each of the configuration 2221 to 2223 included therein may be implemented by a processor and a memory including instructions executable by the processor.

Here, the data output unit 2221 outputs a result detected or analyzed by the optical module 2212 illustrated in FIG. 43 as data. The output data includes the intensity of light for each of a plurality of cycles, but is not limited thereto.

The API unit 2222 is configured to provide a predetermined API. In some cases, the analysis device 2200 may monitor or control an external object by using an API provided by the API unit 2222. On the contrary, the external object may monitor or control the analysis device 2200 by using the API provided by the API unit 2222.

For example, the API provided by the API unit 2222 may be used to externally receive a run command for driving the analysis device 2200.

In addition, the above-described API may be used to control the operation of a door (not shown) provided in the analysis device 2200. Specifically, the analysis device 2000 has a space in which the reaction vessel is accommodated. In addition, this space may be opened and closed by the above-described door. Here, the above-described API may support a control function of the door. Accordingly, an external object, for example, the control module 2500, may open or close the above-described door using an API provided by the API unit 2222.

In addition, the above-described API may support a function of reading a file itself located at a predetermined location, a file name of a file, or contents written in a file. Here, the "content" includes a text, an image, a voice, or the like, which is written in a file, but is not limited thereto. In an embodiment, the API unit 2222 reads a file name stored in the file information storage unit 2522 of the control module 2500. Thereafter, a file having the read file name may be read from among the files stored in the file storage unit 2521 of the control module 2500. The object to be read may include various objects. For example, a file generated by the preparation device management unit 2120 of the preparation device 2100, that is, a file including an instrumentation referred to when the analysis device 2200 performs a detection operation, may be included therein.

The log output unit 2223 is configured to output a state of the analysis device 2200 and other various states as a log. Examples of the output log may include state information indicating whether the analysis device 2200 is operating normally, abnormally, or stopped, and an estimated time required until the analysis device 2200 finishes a nucleic acid amplification operation and/or a nucleic acid detection operation currently in progress, but are not limited thereto.

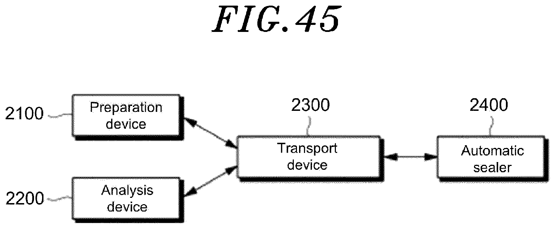

Referring again to FIG. 40, the transport device 2300 will be described. The transport device 2300 is configured to transport the reaction vessel among the preparation device 2100, the analysis device 2200, the automatic sealer 2400, and the container processing module 2800. FIG. 45 illustrates a direction in which the reaction vessel is transported by the transport device 2300. Referring to FIG. 45, the reaction vessel is transported from the preparation device 2100 to the transport device 2300, is transported from the transport device 2300 to the automatic sealer 2400, is again transported from the automatic sealer 2400 to the transport device 2300, and is then transported from the transport device 2300 to the analysis device 2200. Although not shown in FIG. 45, the reaction vessel transported to the analysis device 2200 is transported to the container processing module 2800 and then discarded. Of course, the transport path of the reaction vessel shown in FIG. 45 is merely exemplary.

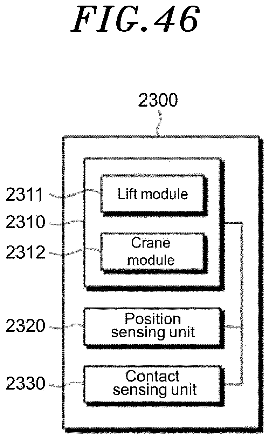

A specific configuration of the transport device 2300 is illustrated in FIG. 46. Referring to FIG. 46, the transport device 2300 includes a transport module 2310, a position sensing unit 2320, and a contact sensing unit 2330, but is not limited thereto.

The transport module 2310 is configured to transport the reaction vessel while moving among the preparation device 2100, the analysis device 2200, the automatic sealer 2400, and container processing module 2800.

The transport module 2310 may be implemented as one module. For example, when the preparation device 2100, the analysis device 2200, and the automatic sealer 2400 are disposed on the same horizontal plane, the transport module 2310 may be implemented as one module that is movable on the corresponding horizontal plane.

Unlike this, the transport module 2310 may be implemented to include at least two configurations. For example, the transport module 2310 may include a lift module 2311 movable in the longitudinal direction and a crane module 2312 movable in the transverse direction. Hereinafter, this will be described in more detail.

The preparation device 2100 may be disposed relatively at an upper portion in the automated analysis system 2000, whereas the analysis device 2200, the automatic sealer 2400, and the container processing module 2800 may be disposed relatively at a lower portion in the automated analysis system 2000. In addition, the analysis device 2200, the automatic sealer 2400, and the container processing module 2800 may be disposed on the same horizontal plane. In this case, the lift module 2311 may move in a longitudinal direction, for example, toward the upper portion, to receive the reaction vessel from the preparation device 2100. Also, the lift module 2311 may move in a longitudinal direction, for example, toward the lower portion, while having the reaction vessel received from the preparation device 2100.

According to the present disclosure, when the lift module 2311 completes the movement in the longitudinal direction, for example, toward the lower portion while having the reaction vessel received from the preparation device 2100, the crane module 2312 grips and acquires the reaction vessel from the lift module 2311 and then moves in the transverse direction. The automatic sealer 2400, the analysis device 2200, and the container processing module 2800 are disposed in the transverse direction of movement. Thus, the crane module 2312 can transport the reaction vessel to each of these modules 2200, 2400, 2800 or retrieve from each of these modules 2200, 2400, 2800 while moving with the reaction vessel transversely

However, it is only an example that the transport module 2310 is implemented or driven as described above, and the spirit of the present disclosure is not limited thereto. For example, the transport module 2310 may be implemented in any form capable of moving with a reaction vessel.

The position sensing unit 2320 is implemented as a position sensor. The position sensing unit 2320 senses the position of the transport module 2310. As a result of the sensing, the position sensing unit 2320 may sense and provide, for example, how far the transport module 2310 is spaced apart from the preparation device 2100 or a relative position of the transport module 2310 with respect to the preparation device 2100.

The contact sensing unit 2330 is implemented by a touch sensor. The contact sensing unit 2330 senses whether the transport module 2310 and the reaction vessel contact each other. As a result of the sensing, the contact sensing unit 2330 may sense and output, for example, whether the transport module 2310 is in contact with the reaction vessel or not in contact with the reaction vessel.

Referring again to FIG. 40, the automatic sealer 2400 is described. The automatic sealer 2400 is implemented to seal the inlet, such as the upper surface, of the reaction vessel in which the analysis sample is accommodated. Specifically, when the reaction vessel is delivered from the transport device 2300, the automatic sealer 2400 seals the upper surface. Here, the sealed surface may be a side surface or a lower surface rather than an upper surface, but hereinafter, it will be described on the premise that it is an upper surface.

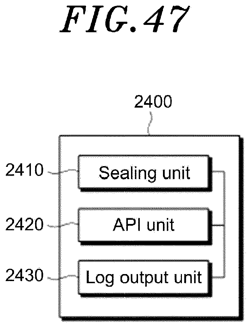

A specific configuration of the automatic sealer 2400 is illustrated in FIG. 47. Referring to FIG. 47, the automatic sealer 2400 includes a sealing unit 2410, an API unit 2420, and a log output unit 2430, but is not limited thereto.

As described above, the sealing unit 2410 may seal an inlet, for example, an upper surface, of the reaction vessel in which the analysis sample is accommodated. To this end, the sealing unit 2410 may adhere the transparent film to the inlet of the reaction vessel. In this case, heat may be used for adhesion, or an adhesive may be used.

The API unit 2420 is configured to provide a predetermined API. In some cases, the external object may monitor or control the state of the sealing unit 2410 by using the API provided by the API unit 2420.

The log output unit 2430 is configured to output a state of the automatic sealer 2400 as a log. Examples of the output log may include, but are not limited to, whether the sealing operation is currently in progress.

Referring again to FIG. 40. The fan module 2600 is disposed in the automated analysis system 2000 and operates to discharge air out from the automated analysis system 2000. At least one of the fan modules 2600 may be disposed in the automated analysis system 2000.

Where the fan module 2600 is disposed in the internal structure of the automated analysis system 2000, and operation control of the fan module 2600 will be described later.

The container processing module 2800 is configured to receive a reaction vessel, on which detection of a target nucleic acid has been completed, from the analysis device 2200. According to an implementation example, the transport device 2300 may recover the reaction vessel on which the detection operation is completed from the analysis device 2200 and transport the reaction vessel to the container processing module 2800, but is not limited thereto.

The following is a description of the control module 2500. Referring to FIG. 40, the control module 2500 includes a control unit 2510 and a storage unit 2520. However, it is not limited thereto. Here, the control module 2500 and each of the features 2510 to 2520 included therein may be implemented by a processor and a memory storing instructions executable by the processor.

The storage unit 2520 may store various types of information. For example, the storage unit 2520 may store state information of the transport device 2300, state information of the preparation device 2100 or the analysis device 2200, or state information of the automatic sealer 2400.

The control unit 2510 controls an overall operation of the automated analysis system 2000. For example, the control unit 2510 controls an operation of the transport device 2300. In addition, the control unit 2510 may control the automatic sealer 2400, the fan module 2600, or the container processing module 2800. In addition, the control unit 2510 may transport a file between the preparation device 2100 and the analysis device 2200.

Next, operation control of the transport device 2300 by the control unit 2510 will be described. Under the control of the control unit 2510, the transport device 2300 moves toward the preparation device 2100. Then, the transport device 2300 receives the sample analysis container on which the sealing operation has not been performed from the preparation device 2100.

Also, the transport device 2300 is moved from the preparation device 2100 to the automatic sealer 2400 under the control of the control unit 2510. Then, the reaction vessel is transported to the automatic sealer 2400, and the reaction vessel is sealed by the automatic sealer 2400.

Also, the transport device 2300 is moved to the analysis device 2200 with the reaction vessel of which sealing operation is completed under the control of the control unit 2510. Then, the reaction vessel is transported to the analysis device 2200, and a detection operation for the target nucleic acid is performed.

Also, the transport device 2300 is moved from the analysis device 2200 to the container processing module 2800 with the reaction vessel under the control of the control unit 2510. The reaction vessel is returned to the container processing module 2800.

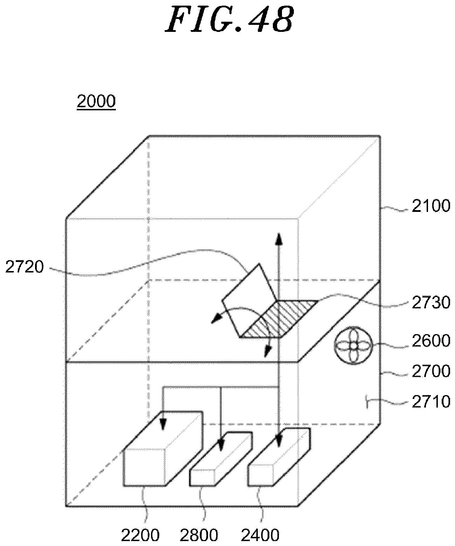

Next, control of the fan module 2600 by the control unit 2510 will be described. Where the fan module 2600 is disposed in the automated analysis system 2000, and how each feature is disposed in the automated analysis system 2000 at this time and how the transport device 2300 moves will be described with reference to FIG. 48.

FIG. 48 illustrates an appearance of the automated analysis system 2000 according to an embodiment, and a device or a module disposed therein. However, FIG. 48 is only an example, and the spirit of the inventive concept is not limited thereto.

Referring to FIG. 48, a preparation device 2100 is disposed on an upper portion of the automated analysis system 2000. In addition, an enclosure 2700 is disposed under the automated analysis system 2000. Of course, the arrangement position of the enclosure 2700 is not limited to the lower portion of the preparation device 2100. For example, according to an embodiment, the enclosure 2700 may be disposed on an upper portion or a side portion of the preparation device 2100. However, hereinafter, it is assumed that the enclosure 2700 is disposed under the preparation device 2100.

The enclosure 2700 will be described in detail. An internal space 2710 is provided inside the enclosure 2700. The analysis device 2200, the automatic sealer 2400, and the container processing module 2800 are disposed in the internal space 2710. In addition, the fan module 2600 may be disposed to pass through the enclosure 2700 so as to discharge the air in the internal space 2710 to the outside of the automated analysis system 2000.

The internal space 2710 may be sealed or isolated from the outside. To this end, the material of the enclosure 2700 may include a material through which air cannot pass. Also, the enclosure 2700 may include a transparent or translucent material to allow the inside space 2710 to be seen from the outside.

The enclosure 2700 is provided with a defined passage 2730. The defined passage 2730 serves as a passage for connecting the internal space 2710 of the enclosure 2700 to another space. Referring to FIG. 48, the internal space 2710 of the enclosure 2700 may be connected to the preparation device 2100 or the internal space of the preparation device 2100 through the defined passage 2730. That is, air may move between the internal space of the enclosure 2700 and the internal space of the preparation device 2100 through the defined passage 2730. In addition, the above-described transport device 2300 may move between the preparation device 2100 and the internal space 2710 through the defined passage 2730.

The defined passage 2730 is opened and closed by the gate part 2720. If the defined passage 2730 is closed by the gate part 2720, air cannot move between the internal space of the preparation device 2100 and the internal space 2710 of the enclosure 2700. On the other hand, when the defined passage 2730 is opened by the gate part 2720, air may move between the internal space of the preparation device 2100 and the internal space 2710 of the enclosure 2700.

The gate part 2720 may open and close the defined passage 2730 in various ways. For example, the gate part 2720 may be passively opened and closed by the movement of the transport device 2300. More specifically, while the transport device 2300 in the internal space 2710 moves to the preparation device 2100 through the defined passage 2730, the transport device 2300 moves while pushing the gate part 2720, and accordingly, the gate part 2720 is opened. Thereafter, when the transport device 2300 moves from the preparation device 2100 to the internal space 2710 of the enclosure 2700 through the defined passage 2730, the transport device 2300 does not push the gate part 2720 anymore, and accordingly, the gate part 2720 is closed. To this end, the gate part 2720 may be operated in an arrow direction as illustrated in FIG. 48, and for this purpose, may be connected to the enclosure 2700 by a hinge.

Unlike this, the gate part 2720 may be controlled by the control unit 2510 illustrated in FIG. 40. For example, when the transport device 2300 in the internal space 2710 has to pass through the defined passage 2730, the gate part 2720 is opened about the hinge by the control unit 2510. Thereafter, when the transport device 2300 moves from the preparation device 2100 to the internal space 2710 of the enclosure 2700 through the defined passage 2730, the gate part 2720 is closed around the hinge by the control unit 2510.

The control unit 2510 stops the operation of the fan module 2600 when the definitive path 2730 is opened. For example, while the transport device 2300 is passing through the defined passage 2730 or while the transport device 2300 is inside the preparation device 2100, the defined passage 2730 is opened, and during this period, the control unit 2510 stops the operation of the fan module 2600.

More specifically, the transport device 2300 is normally stationary in the internal space 2710 of the enclosure 2700. In this case, the defined passage 2730 is closed by the gate part 2720. In this case, the fan module 2600 may be stopped or in operation, and whether to operate or stop is determined by the control unit 2510.

Also, the transport device 2300 may move from the internal space 2710 toward the inside of the preparation device 2100, and may pass through the defined passage 2730 in the process. The control unit 2510 stops the operation of the fan module 2600 while the transport device 2300 passes through the defined passage 2730, that is, while passing.

Also, the transport device 2300 that has passed through the defined passage 2730 approaches the preparation device 2100 to receive the reaction vessel therefrom, and then passes through the defined passage 2730 to return to the internal space 2710. During this period, the defined passage 2730 is opened, and the control unit 2510 stops the operation of the fan module 2600 during this period.

That is, according to one embodiment, the operation of the fan module 2600 is stopped while the definitive passage 2730 is opened. Therefore, since the fan module 2600 does not operate despite the opening of the defined passage 2730, the possibility of air exchange between the internal space of the preparation device 2100 and the internal space 2710 of the enclosure 2700 of the analysis device 2200 is lowered.

In general, air should not be exchanged between the internal space of the preparation device 2100 and the internal space 2710 in which the analysis device 2200 is disposed. This is because, when air is exchanged, pathogens that may be included in the air may move from one space to another, and thus contamination or infection may occur.

However, in an embodiment, a situation may occur in which the defined passage 2730 is opened, and in this case, when the fan module 2600 is operated, there is a high possibility that air is exchanged between the internal space of the preparation device 2100 and the internal space 2710 in which the analysis device 2200 is disposed. Accordingly, in one embodiment, the control unit 2510 may stop the operation of the fan module 2600 in the above-described situation, thereby reducing the possibility of the above-described contamination or infection.

Meanwhile, the situation in which the control unit 2510 stops the operation of the fan module 2600 may include not only the case in which the defined passage 2730 is opened but also the case in which it is closed. Hereinafter, this will be described in more detail.

When the transport device 2300 is moving with the unsealed reaction vessel, the control unit 2510 stops the operation of the fan module 2600 even when the defined passage 2730 is closed. For example, while the transport device 2300 is in the internal space 2720 after receiving the unsealed reaction vessel from the preparation device 2100 and passing through the defined passage 2730, the control unit 2510 may stop the operation of the fan module 2600. That is, the control unit 2510 may stop the operation of the fan module 2600 until the transport device 2300 passes through the defined passage 2730 with the unsealed reaction vessel, reaches the automatic sealer 2400, and the sealing operation is completed. Thereafter, when the sealing operation is completed, the control unit 2510 may resume the operation of the fan module 2600 from that time.

On the other hand, the control unit 2510 may stop the operation of the fan module 2600 until the transport device 2300 passes through the defined passage 2730 with the unsealed reaction vessel and then completes the transport to the analysis device 2200 via the automatic sealer 2400. Thereafter, when the transport of the reaction vessel to the analysis device 2200 is completed, the control unit 2510 may resume the operation of the fan module 2600 from that time.

That is, according to an embodiment, the operation of the fan module 2600 may be stopped while the unsealed reaction vessel is located in the internal space 2710 or for a longer period. Accordingly, a phenomenon in which various materials, for example, pathogens, contained in an unsealed reaction vessel are spread into the air of the internal space 2710 may be reduced.

In general, pathogens or the like may be included in an unsealed reaction vessel, and these may be discharged into the air, particularly into the internal space 2710. In this situation, when the fan module 2600 is operated, air is discharged from the internal space 2710 to the outside of the automated analysis system 2000, and there is a risk that pathogens or the like are mixed with the air and discharged to the outside according to circumstances.

Accordingly, in one embodiment, when the reaction vessel that is not sealed is located in the internal space 2710, the fan module 2600 is not operated at this time, and thus the above-described pathogen or the like may not be discharged out from the inside of the automated analysis system 2000.

Hereinafter, referring to FIGS. 49 to 56 together with FIG. 48, the control of the above-described fan module will be described as an example.

In FIGS. 49 to 56, the position of the transport device 2300 is indicated by (1) to (8), respectively.

Referring to FIG. 49, the transport device 2300 is at the position of (1). This position is referred to as a "home" position. When the automated analysis system 2000 is not driven and is in an idle state, or when the automated analysis system 2000 completes an analysis of the reaction vessel once and waits for the next time, the transport device 2300 is located at this position.

In this case, the defined passage 2730 is closed by the gate part 2720. In addition, the fan module 2600 may be turned on or off according to the temperature of the internal space 2710, as illustrated in FIG. 49. Hereinafter, it will be described on the premise that the fan module 2600 is turned on.

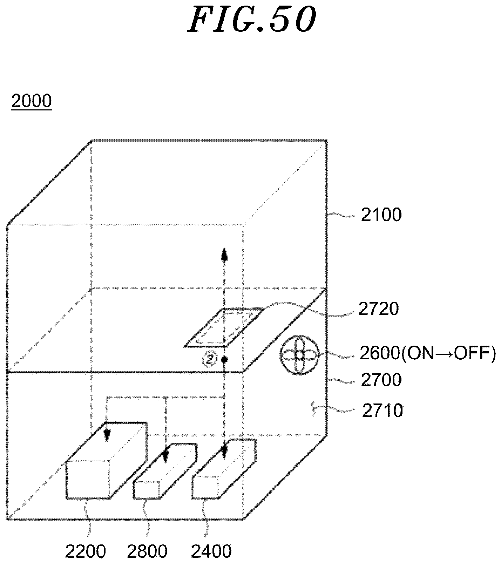

FIG. 50 illustrates a point in time when the transport device 2300 starts to move toward the preparation device 2100 from the home position of (1). (2) in FIG. 50 shows an example of a position where the transport device 2300 has moved from (1). This position (2) is before the transport device 2300 has yet to pass the defined passage 2730. In this case, the defined passage 2730 is still closed by the gate part 2720. In addition, as illustrated in FIG. 50, the fan module 2600 is ON->OFF.

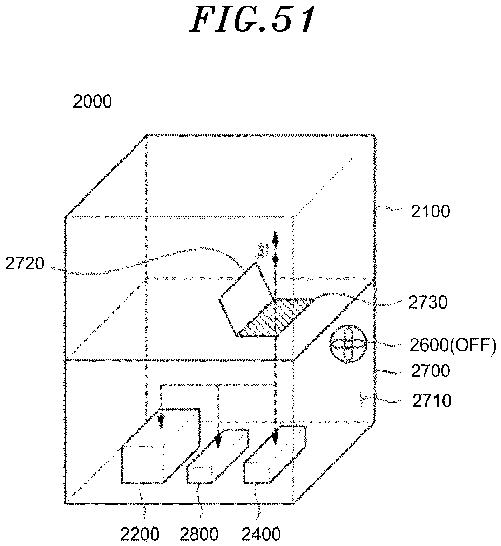

FIG. 51 shows the time when the transport device 2300 is located in the preparation device 2100 (internal space of) via the defined passage 2730. (3) in FIG. 51 shows an example of a point where the transport device 2300 is located in the preparation device 2100. Referring to FIG. 51, the defined passage 2730 is in a state of being opened by the gate part 2720. In this case, there is a possibility that air moves between the internal space of the preparation device 2100 and the internal space 2710 of the enclosure 2700 of the analysis device 2200. In this case, the operation of the fan module 2600 is stopped by the control unit 2510. Therefore, the possibility of air moving between the internal space of the preparation device 2100 and the internal space 2710 of the enclosure 2700 of the analysis device 2200 may be reduced compared to when the fan module 2600 is operated, and even if the air moves, the amount of air may be reduced compared to when the fan module 2600 is operated.

In FIG. 51, the transport device 2300 may receive an unsealed reaction vessel from the preparation device 2100.

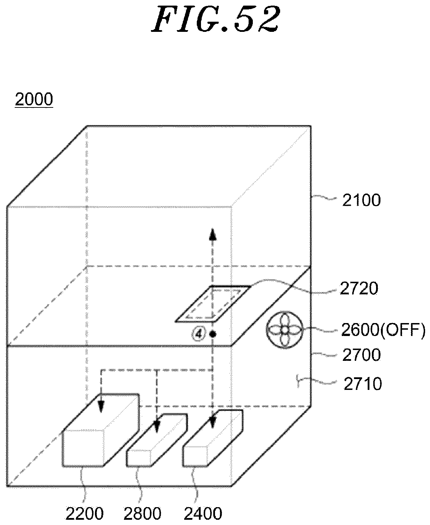

FIG. 52 shows the time when the transport device 2300 passes through the defined passage 2730 in the internal space of the preparation device 2100 and returns to the internal space 2710 of the enclosure 2700 of the analysis device 2200. (4) in FIG. 52 illustrates an example of a location where the transport device 2300 is located in the internal space 2710

Referring to FIG. 52, the defined passage 2730 is in a state of being closed by the gate part 2720. In this case, there is no possibility that air moves between the internal space of the preparation device 2100 and the internal space 2710 of the enclosure 2700 of the analysis device 2200.

However, the transport device 2300 may have an unsealed reaction vessel received from the preparation device 2100 and may be in the internal space 2710. In this case, the operation of the fan module 2600 is stopped by the control unit 2510.

Therefore, the possibility of the pathogens that may be contained in the reaction vessel leaking into the internal space 2710 may be reduced compared to the case in which the fan module 2600 operates.

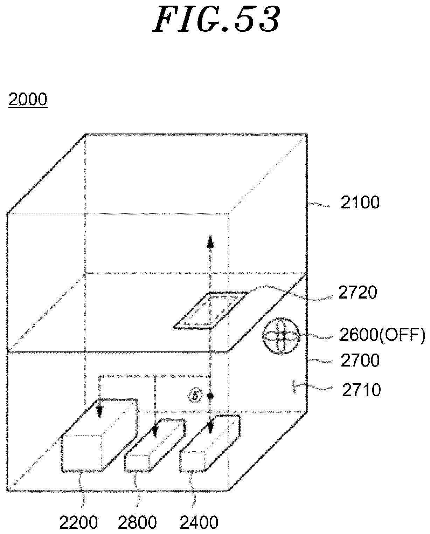

FIG. 53 shows a time point when the transport device 2300 has completely moved to the automatic sealer 2400. (5) in FIG. 53 illustrates the position of the transport device 2300 at this time. Referring to FIG. 53, the defined passage 2730 is in a state of being closed by the gate part 2720. In this case, there is no possibility that air moves between the internal space of the preparation device 2100 and the internal space 2710 of the enclosure 2700 of the analysis device 2200. Further, the fan module 2600 is also stopped.

In FIG. 53, the automatic sealer 2400 receives a reaction vessel that is not sealed from the transport device 2300, and performs a sealing operation for the reaction vessel.

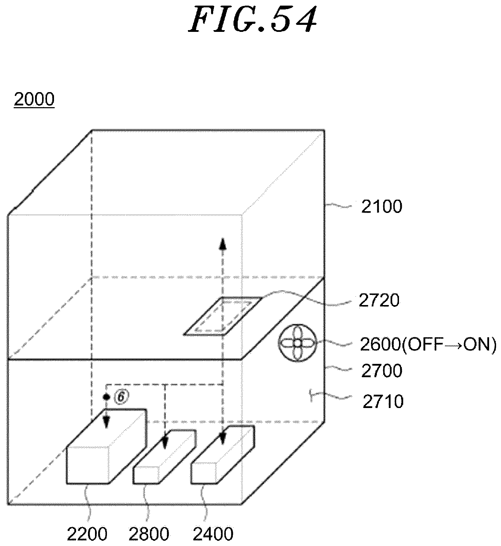

FIG. 54 shows a time point when the transport device 2300 completes the transport from the automatic sealer 2400 to the analysis device 2200. (6) in FIG. 54 illustrates the position of the transport device 2300 at this time.

Referring to FIG. 54, the defined passage 2730 is in a state of being closed by the gate part 2720. In this case, there is no possibility that air moves between the internal space of the preparation device 2100 and the internal space 2710 of the enclosure 2700 of the analysis device 2200.

In FIG. 54, the analysis device 2200 receives a reaction vessel in which a sealing operation is completed from the transport device 2300, and detects a target nucleic acid from the reaction vessel. In this process, heat is generated in the analysis device 2200.

Accordingly, as illustrated in FIG. 54, when the reaction vessel is delivered to the analysis device 2200, the control unit 2510 restarts the operation of the fan module 2600 from that time. Then, the heat generated by the operation of the analysis device 2200 is discharged out from the internal space 2710 of the automated analysis system 2000.

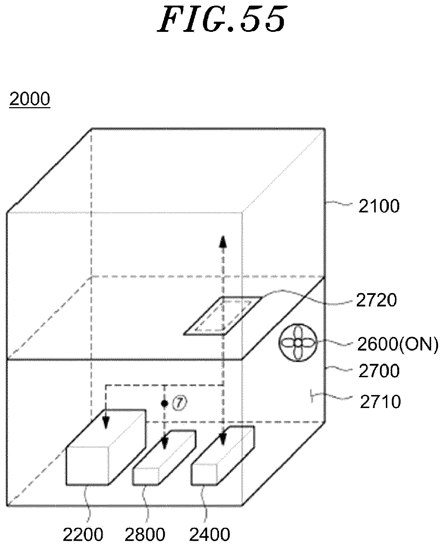

FIG. 55 illustrates a point in time when the transport device 2300 completes the transport from the analysis device 2200 to the container processing module 2800. (7) in FIG. 55 illustrates the position of the transport device 2300 at this time.

Referring to FIG. 55, the defined passage 2730 is in a state of being closed by the gate part 2720. In this case, there is no possibility that air moves between the internal space of the preparation device 2100 and the internal space 2710 of the enclosure 2700 of the analysis device 2200.

In FIG. 55, the transport device 2300 transports the reaction vessel received from the analysis device 2200 to the container processing module 2800. Then, the reaction vessel is recovered.

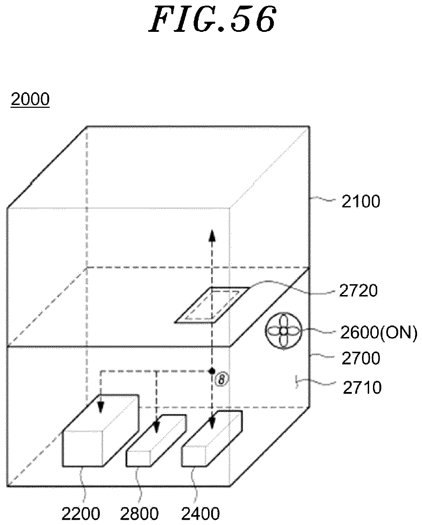

FIG. 56 illustrates a state in which the transport device 2300 completes the movement from the container processing module 2800 to the position of the home. (8) in FIG. 56 illustrates the position of the transport device 2300 at this time.

Referring to FIG. 56, the defined passage 2730 is in a state of being closed by the gate part 2720. In this case, there is no possibility that air moves between the internal space of the preparation device 2100 and the internal space 2710 of the enclosure 2700 of the analysis device 2200. In addition, the fan module 2600 is in an ON state.

Meanwhile, the control unit 2510 may control a rotation speed of the fan in the fan module 2600. For example, the control unit 2510 may control the rotation speed of the fan in the fan module 2600 to be fast or slow according to the internal temperature of the automated analysis system 2000. To this end, although not illustrated in the automated analysis system 2000, a temperature measuring unit for measuring a temperature of the internal space 2710 may be included.

Meanwhile, at least one of the above-described fan modules 2600 may be provided in the automated analysis system 2000. Some of the fan modules 2600 may be driven regardless of whether the temperature of the internal space 2710 is below or exceeds the reference value while the analysis device 2200 is being driven and the defined passage 2730 is closed, and the remaining fan modules 2600 may be driven only when the temperature of the internal space 2710 exceeds the reference value while the analysis device 2200 is being driven and the defined passage 2730 is closed. Control of the fan module 2600 may be performed by the control unit 2510.

FIG. 57 is a flowchart of a fan module control method that may be performed by the automated analysis system 2000 according to an embodiment. However, since FIG. 57 is only an example, the spirit of the present disclosure is not limited to that illustrated in FIG. 57.

Referring to FIG. 57, in the fan module control method, a step of opening the defined passage 2730 of the enclosure 2700 of the analysis device 2200 by the gate part 2720 is performed (S100).

Then, while the defined passage 2730 is opened by the gate part 2720 in S100, the control unit 2510 stops the operation of the fan module 2600 (S200).

Hereinafter, the fan module control method is performed by the automated analysis system 2000, and the detailed description of the corresponding method will be referred to the description of the automated analysis system 2000.

Meanwhile, the method illustrated in FIG. 57 may be implemented in a computer-readable recording medium storing a computer program programmed to perform the steps included in each of these methods, or may be implemented in the form of a computer program stored in a computer-readable recording medium as a computer program programmed to perform each step included in these methods.

As described above, according to an embodiment, the risk that pathogens that may be included in the reaction vessel are leaked and dispersed into the air by the operation of the fan module may be reduced.

Also, the possibility of mixing the air inside a preparation device for carrying out preparation for detecting the target nucleic acid and the air around an analysis device for carrying out detection of the target nucleic acid can be reduced.

### Assembly method

Next, an assembly method of the automated analysis system will be described.

The present disclosure relates to a stand-alone analysis device used for sample preparation and analysis for molecular diagnosis, a stand-alone preparation device, and a method for assembling the three types of devices which assemble a transport unit for transporting a reaction vessel between the two devices to perform a series of processes from sample preparation to analysis in a lump.

The present inventors have made extensive efforts to develop a method of securing user convenience and safety while maintaining independence of each device with respect to existing stand-alone devices licensed for molecular diagnosis. As a result, the present inventors have developed a method for assembling molecular diagnostic devices which uses a transport unit to form a movement pathway of a reaction vessel between the stand-alone analysis device and the stand-alone preparation device without impairing the independence of the stand-alone analysis device and the stand-alone preparation device.

The present disclosure provides a method for manufacturing an assembly of a molecular diagnostic device.

According to an aspect of the disclosure, there is provided a method of manufacturing an assembly of a molecular diagnostic device, the assembly including a stand-alone analysis device, a transport device, and a stand-alone preparation device, wherein the transport device transports a reaction vessel; wherein the stand-alone preparation device provides a reaction vessel including a sample analyzable by the stand-alone analysis device; wherein the stand-alone analysis device analyzes the sample contained in the reaction vessel; and wherein the method includes the following steps: (a) providing the stand-alone analysis device and the stand-alone preparation device to the transport device; and (b) aligning the stand-alone analysis device, the transport device, and the stand-alone preparation device, wherein the aligning forms a movement pathway for the reaction vessel between the stand-alone preparation device and the stand-alone analysis device.

Molecular diagnosis refers to acquiring desired information by applying molecular biological technology for analyzing genetic information included in a sample or biological markers included in a protein to a medical test. The biological label refers to a target analyte, and may be, for example, a target nucleic acid sequence or an amino acid sequence. Desired information may be information on the presence, absence, or amount of the biological label.

The molecular diagnostic device refers to a device that may be used in such molecular diagnosis. The molecular diagnostic device may include an analysis device for identifying genetic information (for example, a nucleic acid amplification device, a sequencing device, and a DNA chip system) and an analysis device for identifying amino acid sequence information (for example, an antibody-based analysis device). In addition, the molecular diagnostic device may include a preparation device (for example, an extraction device or a PCR setup device) for preparing a sample in a state in which an analysis device for identifying the genetic information or the amino acid information may perform a process for identification. The target analyte is referred to as an analyte or a target analyte.

According to an embodiment of the present disclosure, the molecular diagnostic device may be a stand-alone device. A stand-alone device refers to a device that is configured to independently perform its own functions without involving devices having other functions.

The analysis device of the present disclosure is a stand-alone analysis device, and the preparation device of the present disclosure is a stand-alone preparation device. Therefore, according to an embodiment of the present disclosure, the stand-alone analysis device and/or the stand-alone preparation device of the present disclosure may be a molecular diagnostic device.

According to an embodiment of the present disclosure, the stand-alone analysis device may be a previously licensed analysis device, and the stand-alone preparation device may be a previously licensed preparation device. The license may be a license to use a corresponding device for molecular diagnosis. Specifically, the license may be a license for using a corresponding device in the use of an In-vitro Diagnostics (IVD). The term "in vitro diagnostic medical device" means a device including a reagent used as a medical device used in vitro for the purpose of providing information such as diagnosis, prognosis, observation, blood or tissue suitability determination, etc. of a disease by testing substances in a sample derived from a human body as a sample.

Therefore, the preparation device or the analysis device previously licensed in the present disclosure includes the preparation device or the analysis device itself that is licensed as an in vitro diagnostic medical device, and the preparation device or the analysis device is included in the previously licensed preparation device or analysis device even when the kit for determining a specific disease or infection is licensed as an in vitro diagnostic medical device to use the preparation device or the analysis device.

The license may be a license approved by the health authorities of each country. Specifically, the country may be Korea, the United States, or Europe.

In the present disclosure, an assembly refers to a structure in which two or more devices are combined. At least one of the two or more devices constituting the assembly may be a stand-alone device. According to an embodiment of the present disclosure, at least one of the devices constituting the assembly may be a pre-licensed device as an in vitro diagnostic medical device, and the pre-licensed device may be independently used as an in vitro diagnostic medical device without separate license even though the device is included in the assembly of the present disclosure.

In other words, in the conventional full auto molecular diagnostic system, several modules included therein are manufactured for the full auto molecular diagnostic system, and the full auto molecular diagnostic system itself is one device performing various functions, whereas the assembly of the present disclosure is not one device itself, but a set of a plurality of devices in which a plurality of devices capable of performing their own functions are elaborately connected.

According to one embodiment of the present disclosure, the assembly may be the above-described automated analysis system 1000.

The assembly of the present disclosure includes a preparation device. The preparation device prepares an analysis sample including or presumed to include an analyte. In this specification, the terms "preparation device" and "sample preparation device" have the same meaning and may be used interchangeably.

A preparation device is a device that prepares or preprocesses a sample in a state in which an analysis device may proceed with a process for identification. For example, the preparation device may be, for example, an extraction device for separating a nucleic acid or a polypeptide from a sample or a setup device for mixing the separated nucleic acid or the polypeptide with a reagent required for the analysis so that the analysis device may perform a desired analysis.

The assembly of the present disclosure also includes an analysis device. The analysis device analyzes a sample contained in the reaction vessel. Specifically, the analysis device analyzes a sample and provides information on the presence or amount of an analyte in the sample. The analysis device may include an analysis device for identifying genetic information (for example, a nucleic acid amplification device, a sequencing device, a DNA chip system) and an analysis device for identifying amino acid sequence information (for example, an antibody-based analysis device).

As used herein, the terms "analysis device" and "sample analysis device" may be used interchangeably with each other, and refer to a device used for qualitative or quantitative analysis of an analyte.

Sample analysis includes detecting the presence of an analyte and measuring the content.

According to one embodiment, the sample analysis device is a real-time detection device. According to one embodiment, the sample analysis device is a real-time nucleic acid detection device. According to one embodiment, the sample analysis device is a real-time PCR device.

The assembly of the present disclosure also includes a transport unit. The transport unit transports the reaction vessel. The transport unit includes a transport device and an enclosure. The transport device includes a lift module and a crane module.

According to an embodiment of the present disclosure, the transport unit may include at least one enclosure, and the enclosure may include a second defined passage through which the reaction vessel passes.

The enclosure may accommodate the stand-alone analysis device or the stand-alone preparation device.

According to one embodiment of the present disclosure, the enclosure may include positioning means for alignment. In the present specification, the terms "positioning means" and "position determining means" have the same meaning and may be used together. The positioning means allows the analysis device or preparation device accommodated in the enclosure to be located at a predetermined point. The positioning means may be, for example, a binding means fixing a lower portion of the analysis device or the preparation device or a concave groove, a protruding structure, or a fastening portion formed on an inner surface of the enclosure in contact with the analysis device or the preparation device. The analysis device or the preparation device, by means of the positioning means, is positioned at a predetermined site on the inside of the enclosure and can form a movement pathway for the reaction vessel.

The enclosure includes a second defined passage through which the reaction vessel passes. The reaction vessel enters and exits through the second defined passage. Therefore, the formed movement pathway of the reaction vessel may be formed to pass through the second defined passage.

According to one embodiment of the present disclosure, the second defined passage may include a door device. The door device may be opened when the reaction vessel is moved through the second defined passage, and may be operated to be closed after the movement is completed.

The position of the second defined passage is not particularly limited, and may be, for example, positioned at an upper portion, a lower portion, or a side surface of the enclosure. According to one embodiment of the present disclosure, the second defined passage may be located above the enclosure.

According to one embodiment of the present disclosure, the transport unit of the present disclosure may include an environmental control means. The environment control means is an environment control means for controlling the internal environment of the enclosure of the transport unit.

The environment adjusting means is for adjusting the internal environment of the enclosure of the transport unit to the same environment as when a stand-alone device (for example, a stand-alone analysis device or a stand-alone preparation device) positioned inside the enclosure is not included in the assembly of the present disclosure and is used alone. Consequently, the same performance as when the stand-alone device located in the enclosure is used alone can be exhibited. Modules of conventional full auto molecular diagnostic systems are not used alone, and have been originally designed to operate in an environment provided by the corresponding system. Therefore, the conventional fully automatic molecular diagnostic system does not need to have a means for controlling the environment to be the same as the environment in which each module is used alone.

The environment control means of the present disclosure may include a cooling and heating device such as a ventilation fan, an air conditioner, a heating wire, a lamp, and the like, and a control unit for controlling the same.

Also, the environment control means of the transport unit according to the present disclosure may be controlled in connection with the operation of another part of the transport unit. According to an embodiment of the present disclosure, the environmental control means of the present disclosure may be an operation-linked environmental control means. The motion-linked environment control means may operate in conjunction with the operation of other components of the transport unit.

Both the stand-alone analysis device and the stand-alone preparation device may be located in the enclosure of the transport unit of the present disclosure. However, in general, the stand-alone preparation device has a shielding means for separating the inside and the outside by itself. This is because, even when the preparation device is used alone, a means for separating the inside and the outside of the preparation device is required in order to prevent contamination of samples, reaction vessels, and various reagents accommodated in the preparation device. However, the present disclosure does not include a means for shielding the stand-alone analysis device. Therefore, according to an embodiment of the present disclosure, the stand-alone analysis device may be located in the enclosure of the transport unit.

The transport unit of the present disclosure transports the reaction vessel. Therefore, according to one embodiment of the present disclosure, the transport unit may include one or more transport modules.

According to one embodiment of the present disclosure, the transport unit may include a lift module and a crane module.

According to an embodiment of the present disclosure, the lift module may be configured to transport the reaction vessel through the second defined passage of the transport unit, and the crane module may be configured to transport the reaction vessel inside the enclosure of the transport unit. As a result, the size of the second defined passage of the enclosure can be minimized so that the movement pathway of the reaction vessel can be established between the two devices while maintaining the mutual isolation effects of the preparation device and the analysis device. In order to implement both the three-dimensional movement of the transport unit inside the enclosure and the transport of the reaction vessel through the second defined passage with one transport module, an expensive multi joint device is required.

According to an embodiment of the present disclosure, the stand-alone analysis device may be located at the enclosure of the transport unit, in which case the lift module transports the reaction vessel from the inside of the stand-alone preparation device to the inside of the enclosure of the transport unit, and the crane module transports the reaction vessel inside the enclosure of the transport unit.

According to an embodiment of the present disclosure, the transport unit of the present disclosure may include two transport modules, and the stand-alone preparation device and the stand-alone analysis device are respectively aligned with different transport modules. The combination of the present disclosure can construct a one-step molecular diagnosis process by using a conventional preparation device or analysis device used independently in the combination. Thus, the molecular diagnosis device of the present disclosure eliminates the necessity of discarding existing molecular diagnosis devices and constructing an expensive automatic molecular diagnosis system. The transport unit should be able to form a reaction vessel movement pathway between the preparation device and the analysis device designed and manufactured without considering the transport of the reaction vessels to each other. To this end, the transport unit of the present disclosure includes a lift module and a crane module, and the stand-alone preparation device and the stand-alone analysis device are respectively aligned with different transport modules. Accordingly, in the configuration of the assembly of the present disclosure, the stand-alone preparation device and the stand-alone analysis device may be selected independently of each other. In other words, according to the assembly and the manufacturing method thereof of the present disclosure, the selection of the stand-alone preparation device is not limited by the use of a specific stand-alone analysis device, and the selection of the stand-alone analysis device is not limited by the use of a specific stand-alone preparation device.

The method of manufacturing the assembly of the molecular diagnostic device of the present disclosure includes the following steps.

The method may further include: (a) providing the stand-alone analysis device and the stand-alone preparation device to the transport unit; and (b) aligning the stand-alone analysis device, the transport unit, and the stand-alone secretion device, wherein the aligning forms a movement pathway of the reaction vessel between the stand-alone preparation device and the stand-alone analysis device.

In step (a), the stand-alone analysis device and the stand-alone preparation device are provided to the transport unit.

As described above, the transport unit may include at least one enclosure, and the enclosure may include a second defined passage through which the reaction vessel passes. According to an embodiment of the present disclosure, the providing of the stand-alone analysis device to the transport unit may include placing the stand-alone analysis device on the enclosure of the transport unit.

In addition, the enclosure includes positioning means for alignment, and the step (a) of providing the stand-alone analysis device and the transport unit may include a step of positioning the stand-alone analysis device on the positioning means.

FIG. 34 is a view for describing providing the stand-alone analysis device 1200 to the enclosure 1300 of the transport unit 1050 according to an embodiment of the present disclosure. As shown in FIG. 6, in the providing of the present disclosure, the stand-alone analysis device 1200 is positioned on the enclosure 1300 of the transport unit 1050. The enclosure 1300 may dispose the second positioning means 1390 at a predetermined position for the stand-alone analysis device 1200. The second positioning means 1390 may be, for example, a binding unit fixing a lower portion of the analysis device or the preparation device or a concave groove, a protruding structure, or a fastening unit formed on an inner surface of the enclosure in contact with the analysis device or the preparation device.

In step (a), the stand-alone preparation device is also provided to the transport unit.

The providing of the stand-alone preparation device refers to positioning the stand-alone preparation device with respect to the transport unit so as to complete a movement pathway for the analysis device and the reaction vessel.

According to an embodiment of the present disclosure, the stand-alone preparation device may include a first defined passage through which the reaction vessel may pass, and the providing may include positioning the second defined passage of the transport unit and the first defined passage to face each other.

In the stand-alone preparation device of the present disclosure, the opening (the first defined passage) of the stand-alone preparation device and the opening (the second defined passage) of the enclosure can be positioned so as to closely face each other. The entry and exit of the reaction vessel in the stand-alone preparation device are carried out by means of the first defined passage, and the entry and exit of the reaction vessel in the enclosure are carried out by means of the second defined passage.

Therefore, when the stand-alone preparation device is positioned so that the first defined passage is adjacent to and faces the second defined passage, the exposure of the reaction vessel to the outside while the reaction vessel moves may be blocked or minimized.

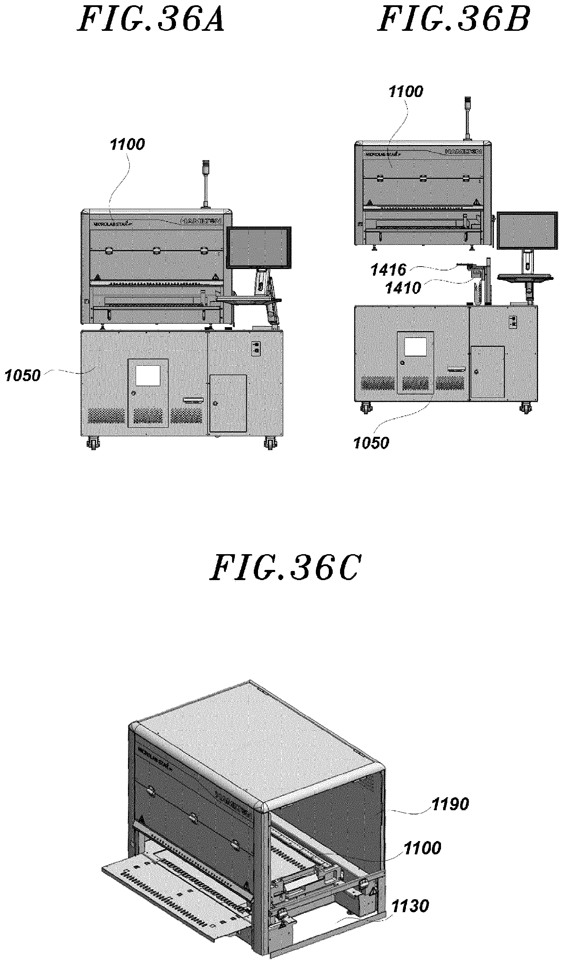

Referring to FIG. 6, a second defined passage 1310 is formed on an upper surface of the enclosure 1300 of the transport unit 1050. In addition, a third positioning means 1395 is provided to position the stand-alone preparation device 1100 provided to the transport unit 1050. FIG. 17 illustrates the transport unit 1050 in a state in which the lift module 1410 is exposed. FIGS. 36A to 36C are diagrams for describing providing the preparation device 1100 to the transport unit 1050.

The first defined passage 1130 and the second defined passage 1310 of the enclosure 1300 are positioned to face each other so that the lift module 1410 may move into the preparation device 1100 through the first defined passage 1130 of the preparation device 1100 (see FIGS. 36C and 6).

In order to facilitate such provision, a third positioning means 1395 for positioning the stand-alone preparation device 1100 provided to the transport unit 1050 is provided on the upper surface of the enclosure 1300 (see FIG. 6).

Accordingly, a movement pathway of the reaction vessel from the preparation device 1100 to the analysis device 1200 may be formed. According to one embodiment of the present disclosure, the movement pathway of the reaction vessel may be formed through the second defined passage and the first defined passage.

In step (b), the stand-alone analysis device, the transport unit, and the stand-alone preparation device are aligned.

Through the alignment step, a movement pathway of the reaction vessel is formed between the stand-alone preparation device and the stand-alone analysis device.

The alignment may specifically include a first alignment step of forming a movement pathway of the reaction vessel between the stand-alone preparation device and the transport unit, and a second alignment step of forming a movement pathway of the reaction vessel between the stand-alone analysis device and the transport unit.

The production method of the present disclosure is a method for configuring an assembly capable of implementing a one-step molecular diagnostic process by using stand-alone preparation devices and stand-alone analysis devices which have been independently produced without considering the forming of a travel path for reaction vessels. Therefore, the stand-alone preparation device and the stand-alone analysis device used in the present disclosure do not always have a structure capable of forming a movement pathway of a reaction vessel by directly aligning with each other. Therefore, the present disclosure forms the movement pathway of the reaction vessel between the stand-alone preparation device and the stand-alone analysis device using the transport unit. Specifically, according to the method of the present disclosure, the method may include: a first alignment step of forming a movement pathway of the reaction vessel between the stand-alone preparation device and the transport unit; and a second alignment step of forming a movement pathway of the reaction vessel between the stand-alone analysis device and the transport unit. Accordingly, the manufacturing method of the present disclosure can assemble an assembly capable of implementing a one-step molecular diagnosis process by using an independently manufactured stand-alone preparation device and a stand-alone analysis device without considering the formation of a movement pathway of a reaction vessel.

Further, the transport unit of the present disclosure may include at least two transport modules, and the stand-alone preparation device and the stand-alone analysis device may be aligned with different transport modules, respectively. Thus, a reaction vessel movement pathway may be significantly easily formed as compared to the case where one transport module aligns each of the preparation device and the analysis device.

Specifically, the transport unit may include a lift module and a crane module. According to an embodiment of the present disclosure, the aligning may include: (c1) aligning the lift module and the stand-alone preparation device; and (c2) aligning the crane module and the stand-alone analysis device.

As described above, the transport unit of the present disclosure may include a lift module and a crane module, the lift module may be configured to transport the reaction vessel through the second defined passage of the transport unit, and the crane module may be configured to transport the reaction vessel inside the enclosure of the transport unit. The embodiment relates to a case in which the stand-alone analysis device is positioned inside the enclosure of the transport unit and the stand-alone preparation device is positioned outside the enclosure.

The lift module transporting the reaction vessel through the second defined passage of the transport unit is aligned with the stand-alone preparation device to transport the reaction vessel prepared in the stand-alone preparation device into the enclosure through the lift module. Inside the enclosure, the lift module transports the reaction vessel to the crane module. A crane module, which transports the reaction vessel inside the enclosure of the transport unit, is aligned with an analysis device positioned inside the enclosure and positions the transported reaction vessel at a predetermined position of the analysis device. Thus, a movement pathway of the reaction vessel is formed between the stand-alone preparation device and the stand-alone analysis device.

Referring to FIGS. 36A to 36C, the lift module 1410 and the preparation device 1100 are aligned so that the lift module 1410 may pass through the first defined passage 1130 of the preparation device 1100. In detail, the lift module 1410 may be an alignment for adjusting a protruding height (see FIG. 36B) so that the preparation device 1100 may hand over the reaction vessel to the lift module, and the preparation device 1100 may be an alignment for adjusting a position of the preparation device so that the lift module 1410 may pass through the first defined passage 1130 and an alignment for adjusting a position of the preparation device 1100 so that a third transport module (not shown) of the preparation device may hand over the reaction vessel to the analytical sample vessel rack 1416 of the lift module 1410.

According to an embodiment of the present disclosure, the method may include learning, by the transport unit, a position of the stand-alone analysis device.

According to an embodiment of the present disclosure, the method may include learning, by the stand-alone preparation device, a position of the transport unit.

When the stand-alone preparation device and the stand-alone analysis device are positioned on the transport unit through the alignment step, their relative positions are mechanically adjusted to form the movement pathway of the reaction vessel.

The method for manufacturing the reaction vessel of the present disclosure includes a step of accurately transporting the reaction vessel between devices by finely controlling the movement of a lift module, a crane module, or a pipette module of a preparation device, which are transport modules of a transport unit, in a software manner separately from the alignment step. This step is referred to as a teaching step, and is performed in the last step after mechanical positioning of each device is completed. The teaching may be performed in such a manner that a moving module of one device learns a specific location of another device.

The manufacturing method of the present disclosure may include a step in which the transport unit learns the position of the stand-alone analysis device. This may be performed in such a manner that the transport module of the transport unit touches a reaction vessel previously accommodated in the stand-alone analysis device to store the position thereof.

The manufacturing method of the present disclosure may further include learning, by the preparation device, the position of the transport unit. This may be performed in a manner in which the pipette module of the preparation device touches a reaction vessel previously accommodated in the transport module to memorize the position.

As described above, the manufacturing method of the present disclosure can transport the reaction vessel to the reaction vessel accommodating part of the analysis device of the transport module in the case of the analysis device without a module for transporting the reaction vessel, and can transport the reaction vessel to the transport module by learning the position of the transport module in the case of the preparation device to which the pipette module can transport the reaction vessel. In order for the transport module to pick up the reaction vessel in the preparation device, it must be designed so that the transport module can enter the working space inside the preparation device. In this case, the mechanical structure of the transport module may limit the movement of the pipette module of the preparation device, and in order to solve this problem, it may be necessary to change the structure of a main part of the preparation device. However, when the pipette module of the preparation device transports the reaction vessel to the place where the transport module is located, the space occupied by the mechanical structure of the transport module in the preparation device can be minimized, and the pipette module can perform the sample preparation process without being disturbed by the transport module without structural changes to the main parts of the preparation device.

Referring to FIGS. 21 and 22, it is described that the transport unit 1050 learns a position of the stand-alone analysis device 1200. Specifically, the position of the analysis device 1200 may be performed by the crane module 1430 of the transport unit 1050. As shown in FIG. 22, the reaction vessel 1500 may be placed in the sample holder of the analysis device 1200, and the gripper 1437 of the crane module 1430 may be brought into contact with the sample holder 1500 to learn the position of the analysis device 1200. Accordingly, the transport unit 1050 stores the position of the analysis device 1200 to more accurately adjust the movement of the crane module 1430.

According to an embodiment of the present disclosure, the learning of the crane module 1430 may be performed not only for the analysis device 1200 but also for other components within the enclosure 1300. Referring to FIG. 21, the automatic sealer 1700 may be positioned inside the enclosure 1300, and the crane module 1430 may learn about the position.

According to an embodiment of the present disclosure, the stand-alone preparation device 1100 may include a step of learning the position of the transport unit 1050. The learning is to learn the position of the lift module of the transport unit by the stand-alone preparation device 1100, and specifically, may learn the position of the reaction vessel 1500 accommodated in the analytical sample vessel rack 1416 when the analytical sample vessel rack 1416 of the lift module 1410 is maximally extended in the horizontal direction to receive the reaction vessel 1500.

After the mechanical alignment between the lift module 1410 and the stand-alone analysis device 1200 is completed, as shown in FIG. 24A, while the analytical sample vessel rack 1416 of the lift module 1410 is maximally extended, a gripper of a third transport module (not shown) of the preparation device 1100 may learn the position of the reaction vessel 1500 accommodated in the analytical sample vessel rack 1416.

FIG. 6 shows an enclosure according to an embodiment of the present disclosure.

The transport unit 1050 includes an enclosure 1300 and a transport device 1400. The transport device 1400 includes a lift module 1410 and a crane module 1430.

The enclosure 1300 is used to spatially isolate molecular diagnostic devices provided to the transport unit 1050, and may be in the form of a cabinet, a locker, a box, or the like. The enclosure 1300 may be configured as a table in which front, rear, left, right, and lower portions thereof are closed and at least one door is provided. The closing may have a form in which the air or matter is completely blocked from enteringin the alternative, and may be a closing in which the air is allowed to enter in a controlled state through the ventilation hole.

The enclosure 1300 may include components for sample analysis therein. The enclosure 1300 has at least one door installed for maintenance of the components. The door is installed on the front/rear, left/right, etc. so that the user may access each component.

The enclosure 1300 includes a second defined passage 1310 through which a reaction vessel passes. The lift module 1410 transports the reaction vessel through the second defined passage 1310.

The enclosure 1300 has a second defined passage (1st passthrough cavity) 1310 formed on its upper surface through which the lift module 1410 moving to the analysis device 1200 to receive the reaction vessel 1500 passes.

FIG. 7(b) is a perspective view illustrating a door device of the second defined passage 1310 according to an embodiment of the present disclosure. FIG. 6 illustrates the closed form of the gate part 1311 of the second defined passage 1310.

The second defined passage 1310 may be formed in an upper surface of the enclosure 1300.

The second defined passage 1310 is formed to have a size through which a vertical motion guide 1413 included in the lift module 1410 and an analytical sample vessel rack 1416 capable of accommodating the reaction vessel 1500 pass.

In the present disclosure, the second defined passage 1310 may be formed to be vertically connected to the first defined passage 1130 formed in the stand-alone preparation device 1100 located above the enclosure 1300. (see FIGS. 36A to 36C)
In an embodiment of the present disclosure, the second defined passage 1310 is a passage for moving the lift module 1410 into the preparation device 1100.

In another embodiment of the present disclosure, the second defined passage 1310 is a passage opened to move the lift module 1410 into the preparation device 1100, and when the lift module 1410 does not move to the preparation device 1100, the opened second defined passage 1310 may be closed through the gate part 1311 provided in the second defined passage 1310.

The gate part 1311 is provided to maximally prevent uncontrolled material from moving between the enclosure 1300 and the preparation device 1100. The analysis device 1200 may be located inside the enclosure 1300, and the analysis device 1200 may produce a high concentration of an analyte in an analysis process. When such an analyte is diffused into the preparation device 1100 through the second defined passage 1310 and the first defined passage 1130, an error in the diagnosis result may occur. The gate part 1311 provided in the second defined passage 1310 may block the occurrence of an error due to such contamination.

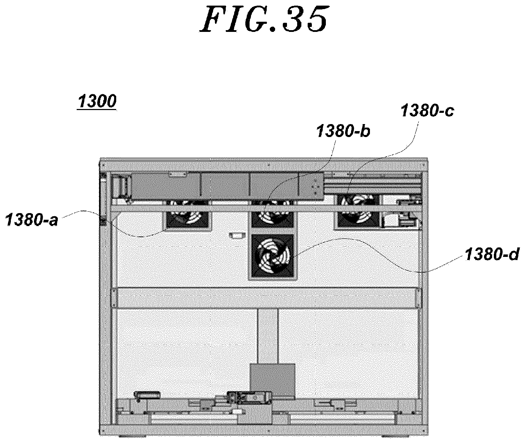

FIG. 35 is a view illustrating the inside of an enclosure 1300 in which a fan 1380, which is an example of an environment control means according to an embodiment of the present disclosure, is disposed. The environment control means 1380 controls the internal environment of the enclosure 1300 of the transport unit. The environment control means 1380 is for controlling the internal environment of the enclosure 1300 of the transport unit to be the same environment as when the stand-alone analysis device positioned inside the enclosure 1300 is not included in the assembly of the present disclosure and is used alone. Accordingly, the same performance as when the stand-alone device located on the enclosure 1300 is used alone may be exhibited. According to an embodiment, the environment control means may include a cooling and heating device such as a fan, an air conditioner, a heating wire, and a lamp, and a control unit for controlling the same. FIG. 35 illustrates an enclosure 1300 in which four fans 1380-a, 1380-b, 1380-c, and 1380-d are disposed as environmental control means. The four fans may be configured to be entirely turned on or off by one power source. The four in the alternative fans may be configured to selectively operate only some of the fans according to a change in the internal environment.

According to one embodiment, a duct for guiding air discharged by the fan may be additionally configured. Accordingly, it is possible to prevent the air discharged by the fan from accessing the preparation device located on the upper portion of the enclosure.

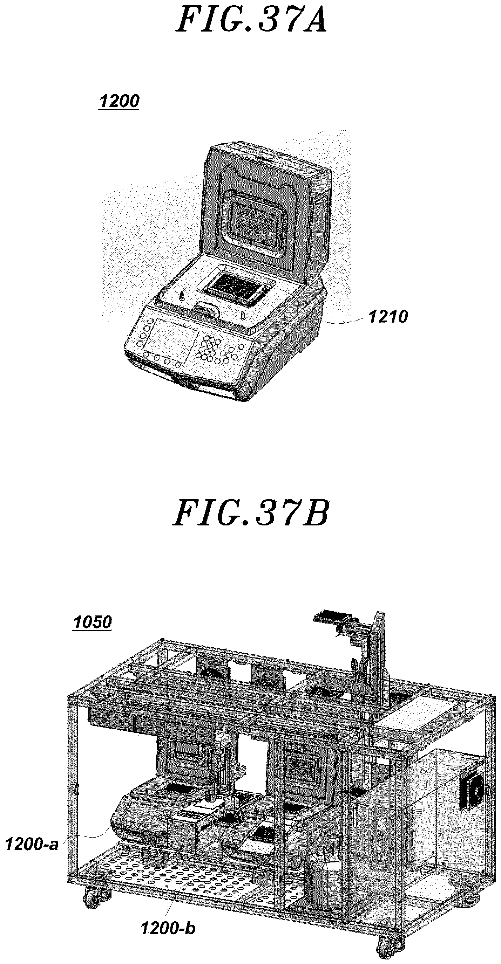

FIGS. 37A and 37B are perspective views of an analysis device 1200 according to an embodiment of the present disclosure. As shown in FIG. 37B, at least one analysis device 1200 for receiving a sealed reaction vessel 1500 in an automatic sealer 1700 may be provided in an enclosure 1300. That is, referring to FIG. 37B, the inside of the enclosure 1300 of the transport unit 1050 is shown with two analysis devices 1200-a. 1200-b may be provided.

In an embodiment of the present disclosure, when two analysis devices are provided in the enclosure 1300, the preparation device 1100 sequentially prepares analysis samples for analysis. When the first reaction vessel is prepared in the preparation device 1100, the first reaction vessel may be moved to any one analysis device in the enclosure 1300 to perform analysis. Thereafter, when the second reaction vessel is prepared in the preparation device 1100, the second reaction vessel may be moved to another analysis device in the enclosure 1300 to perform analysis.

Therefore, according to an embodiment of the present disclosure, the method of the present disclosure may include a step of learning, by the transport unit 1050, the position of each of the analysis devices 1200 when a plurality of analysis devices 1200 are provided.

The preparation device 1100 is a sample preparation device for preparing an analysis sample. FIGS. 36A to 36C illustrate the stand-alone preparation device 1100 of the present disclosure and the alignment between the preparation device 1100 and the transport unit 1050.

The preparation device 1100 includes a deck 1110 that can hold various kinds of instruments and containers for preparing the analytical sample. The deck 1110 is formed in a form in which components included in the preparation device 1100 may be mounted and fixed.

In one embodiment of the present disclosure, the deck 1110 provides a guide for the components of the preparation device 1100 to be inserted into the preparation device 1100 in a sliding manner and positioned on top of the deck 1110.

The preparation device 1100 includes a housing 1190. The housing 1190 isolates the inside of the preparation device 1100 from the outside. The housing 1190 is disposed on the upper surface, the lower surface, and the rear surface of the preparation device. The housing 1190 may be disposed on the front surface of the preparation device 1100. An openable door in which a sample or the like can be put may be formed in the housing on the front. The housing 1190 may be disposed on a left surface and a right surface of the preparation device 1100.

According to one embodiment, a first defined passage 1130 is disposed on a lower surface of the housing 1190.

The first defined passage 1130 is a space in which the lift module 1410 moving from the enclosure 1300 is moved. The first defined passage 1130 is formed to vertically correspond to the second defined passage 1310 of the enclosure 1300. The first defined passage 1130 is formed to have a size in which the vertical motion guide 1413 and the analytical sample vessel rack 1416 of the lift module 1410 may be moved.

FIG. 4 shows an assembly according to an embodiment of the present disclosure. As described above, in the assembly manufactured by the manufacturing method of providing and aligning the stand-alone preparation device 1100 and the stand-alone analysis device 1200 in the transport unit 1050 according to the present disclosure, a movement pathway of the reaction vessel may be formed between the stand-alone preparation device 1100 and the stand-alone analysis device 1200.

Specifically, the movement pathway of the reaction vessel may include the following steps.

(1) Transport of the reaction vessel 1500 from the preparation device 1100 to the lift module 1410; (2) transport of the reaction vessel 1500 from the inside of the preparation device 1100 to the inside of the enclosure 1300 by the lift module 1410; and (3) transport of the reaction vessel 1500 from the lift module 1410 to the stand-alone analysis device 1200 by the crane module.

According to an embodiment, in the transport process of (3), the reaction vessel 1500 may be transported to the analysis device 1200 through the automatic sealer 1700.

Further, the manufacturing method of the present disclosure can isolate the stand-alone analysis device 1200 and the stand-alone preparation device 1100 through the step of positioning the stand-alone analysis device 1200 on the enclosure 1300 of the transport unit 1050, thereby preventing the risk of analysis errors due to contamination.

## Claims

1. An automated analysis system comprising:
a preparation device for preparing an analysis sample in a reaction vessel; the preparation device being a stand-alone device;
an analysis device for analyzing the analysis sample prepared in the reaction vessel;
the analysis device being a stand-alone device;
a transport device for transporting the reaction vessel; and
an enclosure,
wherein at least one device selected from the group consisting of the analysis device and the transport device is located within the enclosure;
wherein the preparation device and the enclosure each comprise a defined passage through which the reaction vessel is transported, and the transport device transports the reaction vessel through the defined passage;
wherein the stand-alone device is capable of operating independently when separated from the automated analysis system.

2. The automated analysis system of claim 1, wherein the enclosure is encloses a single space, and the analysis device and the transport device are located in the single space.

3. The automated analysis system of claim 1, wherein the transport device is positioned inside the enclosure.

4. The automated analysis system of claim 1, wherein the preparation device and/or the analysis device is operatively connected to the enclosure.

5. The automated analysis system of claim 4, wherein the enclosure comprises positioning means capable of determining a position of the preparation device and/or the analysis device to be operatively connected.

6. The automated analysis system of claim 1, further comprising a control module capable of controlling the transport device.

7. The automated analysis system of claim 6, wherein the control module is connected to the preparation device, the analysis device, and the transport device through a communication channel.

8. The automated analysis system of claim 7, wherein the control module controls the preparation device, the analysis device, and the transport device to be operated in a timely manner.

9. The automated analysis system of claim 7, wherein the control module provides an external signal necessary for the operation of the stand-alone device.

10. The automated analysis system of claim 7, wherein the control module is configured to perform:
receiving a signal in which the preparation of the analysis sample is completed in the preparation device via the communication channel; and
providing a control signal to the transport device for transporting the reaction vessel of the preparation device to the analysis device via the communication channel, such that the reaction vessel is transported from the preparation device to the analysis device.

11. The automated analysis system of claim 1, wherein the transport device comprises at least one robotic module configured to transport the reaction vessel from the preparation device to the analysis device.

12. The automated analysis system of claim 1, wherein the preparation device is configured to provide the defined passage by a pre-formed passthrough cavity.

13. The automated analysis system of claim 1, wherein the defined passage is formed with a gate part which is opened when the reaction vessel is transported.

14. The automated analysis system of claim 1, wherein the preparation device is positioned above or below the analysis device.

15. The automated analysis system of claim 1, wherein the preparation device and the analysis device are powered by separate power sources.

16. The automated analysis system of claim 1, the preparation device and the analysis device are commercially available and/or approved as stand-alone devices.

17. The automated analysis system of claim 1, wherein the preparation device uses reagents approved for being used in the stand-alone device.

18. The automated analysis system of claim 1, further comprising an automatic sealer for sealing the upper surface of the reaction vessel.

19. The automated analysis system of claim 18, wherein the automated sealer is positioned on the enclosure.

20. The automated analysis system of claim 1, wherein the transport device comprises a lift module and a crane module, and the lift module is moved upward to the preparation device to move the reaction vessel to the analysis device.

21. The automated analysis system of claim 20, wherein the lift module is extended in a horizontal direction for reception of the reaction vessel within the preparation device.

22. The automated analysis system of claim 20, wherein the crane module further performs an operation of horizontally rotating the reaction vessel.

23. The automated analysis system of claim 6, further comprising:
an gate part which opens and closes the defined passage; and
a fan module that operates to discharge air out of an internal space of the enclosure.

24. The automated analysis system of claim 23, further comprising an automatic sealer for sealing an unsealed reaction vessel,
wherein the transport device receives the unsealed reaction vessel from the preparation device, transports the unsealed reaction vessel to the automatic sealer, receives the sealed reaction vessel from the automatic sealer and transports the sealed reaction vessel to the analysis device, and
the control module stops the operation of the fan module from the time when the transport device receives the unsealed reaction vessel from the preparation device to the time when the transport device completes the transport of the sealed reaction vessel to the analysis device.

25. The automated analysis system of claim 23, wherein the automated analysis system comprises a plurality of fan modules, and
the automated analysis system further comprises a temperature measurement unit configured to measure a temperature of the internal space, and
wherein the control module operates only some of the plurality of fan modules and stops the remaining fan modules when the temperature measured by the temperature measurement unit is equal to or lower than a predetermined temperature, and operates all of the plurality of fan modules when the measured temperature is higher than the predetermined temperature.

26. An analysis method using an automated analysis system, the automated analysis system comprising a preparation device, an analysis device, a transport device, a control module, and an enclosure,
wherein the analysis method comprises:
controlling, by the control module, the transport device such that a reaction vessel containing an analysis sample is transported from the preparation device to the analysis device; the preparation device and the analysis device being stand-alone devices;
controlling, by the control module, the analysis device such that the analysis sample is analyzed in the analysis device; and
controlling, by the control module, the transport device such that the reaction vessel in which analysis of the analysis sample is completed is removed from the analysis device,
wherein at least one device selected from the group consisting of the analysis device and the transport device is located inside an enclosure,
the preparation device and the enclosure each comprises a defined passage through which the reaction vessel is transported, and
the transport device transports the reaction vessel through the defined passage.

27. The analysis method using an automated analysis system of claim 26, further comprising, before the controlling of the transport device to transport the reaction vessel to the analysis device, receiving, by the control module, a signal in which preparation of the analysis sample is completed in the preparation device.

28. The analysis method using an automated analysis system of claim 26, further comprising, before the controlling of the transport device to remove the reaction vessel, receiving, by the control module, a signal in which the analysis of the analysis sample is completed in the analysis device.

29. The analysis method using an automated analysis system of claim 26, wherein the enclosure encloses a single space, and in the step of controlling, by the control module, the transport device to transport the reaction vessel from the preparation device to the analysis device, the transport device located inside the enclosure transports the reaction vessel to the analysis device located in the same space.

30. The analysis method using an automated analysis system of claim 26, wherein the preparation and/or analysis device for performing the steps is operatively connected to the enclosure.

31. The analysis method using an automated analysis system of claim 26, wherein the control module is connected to the preparation device, the analysis device, and the transport device through a communication channel and
in the controlling of the transport device to transport the reaction vessel to the analysis device, the control module performs the steps of:
receiving a signal in which preparation of an analysis sample is completed, from the preparation device, via the communication channel; and
providing a control signal to the transport device for transporting the reaction vessel of the preparation device to the analysis device via using the communication channel,
thereby transporting the reaction vessel from the preparation device to the analysis device.

32. The analysis method using an automated analysis system of claim 26, wherein the transport device comprises a lift module, and wherein controlling the transport device to transport the reaction vessel from a preparation device to the analysis device comprises:
when the lift module is moved into the preparation device, controlling the lift module such that the lift module extends in a horizontal direction to receive the reaction vessel.

33. The analysis method using an automated analysis system of claim 26, wherein the transport device comprises a crane module, and the controlling of the transport device to transport the reaction vessel from the preparation device to the analysis device further comprises,
controlling the crane module, such that the crane module horizontally rotates the reaction vessel.

34. An automated analysis system comprising a memory, at least one processor configured to access the memory, and at least one program stored in the memory and configured to be executed by the processor, the automated analysis system comprising a preparation device, an analysis device, a transport device, a control module, and an enclosure, the at least one program comprising instructions that, when executed by the at least one processor, cause the following steps to be performed:
controlling, by the control module, the transport device such that a reaction vessel containing an analysis sample is transported from the preparation device to the analysis device; the preparation device and the analysis device being stand-alone devices;
controlling, by the control module, the analysis device such that the analysis sample is analyzed in the analysis device; and
controlling, by the control module, the transport device such that the reaction vessel in which the analysis of the analysis sample is completed is removed from the analysis device;
wherein at least one device selected from the group consisting of the analysis device and the transport device is located inside an enclosure,
the preparation device and the enclosure each comprising a defined passage through which the reaction vessel is transported; and
the at least one program comprising instructions that cause the transport device to transport the reaction vessel through the defined passage.

35. A non-transitory computer-readable storage medium containing instructions that, when executed by one or more processors, cause an analysis method using an automated analysis system to be performed,
the automated analysis system comprising a preparation device, an analysis device, a transport device, a control module, and an enclosure, and
the method comprising,
controlling, by the control module, the transport device such that a reaction vessel containing an analysis sample is transported from the preparation device to the analysis device; the preparation device and the analysis device being stand-alone devices;
controlling, by the control module, the analysis device such that the analysis sample is analyzed in the analysis device; and
controlling, by the control module, the transport device such that the reaction vessel in which analysis of the analysis sample is completed is removed from the analysis device,
wherein at least one device selected from the group consisting of the analysis device and the transport device is located inside an enclosure,
the preparation device and the enclosure each comprise a defined passage through which the reaction vessel is transported, and
the instructions comprise instructions that cause the transport device to perform the transport of the reaction vessel through the defined passage.

36. A method of controlling a fan module in an automated analysis system, wherein the automated analysis system comprises:
a preparation device for preparing an analysis sample in a reaction vessel,
an analysis device for analyzing the analysis sample prepared in the reaction vessel,
a transport device for transporting the reaction vessel,
an enclosure,
a gate part for opening and closing a defined passage, and
a fan module that operates to discharge air out from an internal space of the enclosure;
wherein at least one device selected from the group consisting of the analysis device and the transport device is located inside the enclosure;
wherein the preparation device and the enclosure each comprises a defined passage through which the reaction vessel is transported; and
wherein the method of controlling the fan module comprises:
opening the defined passage by the gate part; and
stopping an operation of the fan module while the defined passage is opened by the gate part.

37. The fan module control method of claim 36, wherein the stopping of the fan module is performed according to a position or a movement direction of the transport device.

38. The fan module control method of claim 37, wherein the stopping comprises stopping the operation of the fan module while the transport device is located in the preparation device.

39. The fan module control method of claim 37, wherein the automated analysis system further comprises an automatic sealer for sealing an unsealed reaction vessel,
the transport device receives the unsealed reaction vessel from the preparation device and transports the unsealed reaction vessel to the automatic sealer, and the transport device receives the sealed reaction vessel from the automatic sealer and transports the sealed reaction vessel to the analysis device, and
the operation of the fan module is stopped from the time when the transport device receives the unsealed reaction vessel from the preparation device to the time when the transport device completes the transport of the sealed reaction vessel to the analysis device.

40. The fan module control method of claim 36, wherein the automated analysis system comprises a plurality of fan modules, and
the automated analysis system further comprises a temperature measurement unit measuring a temperature of the internal space, and
the fan module control method causes only some of the plurality of fan modules to operate and stops the remaining fan modules when the temperature measured by the temperature measurement unit is equal to or lower than a predetermined temperature, and causes all of the plurality of fan modules to operate when the measured temperature is higher than the predetermined temperature.

41. A method of manufacturing an assembly of a molecular diagnostic device, the assembly comprising a stand-alone analysis device, a transport device, and a stand-alone preparation device;
wherein the transport device transports a reaction vessel;
the stand-alone preparation device provides a reaction vessel containing a sample analyzable by the stand-alone analysis device;
the stand-alone analysis device analyzes the sample contained in the reaction vessel;
the method comprising the following steps:
(a) providing the stand-alone analysis device and the stand-alone preparation device to the transport device; and
(b) aligning the stand-alone analysis device, the transport device, and the stand-alone preparation device,
wherein the aligning forms a movement pathway for the reaction vessel between the stand-alone preparation device and the stand-alone analysis device.

42. The method of claim 41, wherein the transport device comprises at least one enclosure, the enclosure comprising a second defined passage through which the reaction vessel passes.

43. The method of claim 42, wherein the stand-alone preparation device comprises a first defined passage through which the reaction vessel can pass, and the providing comprises positioning the first defined passage and the second defined passage of the transport device to face each other.

44. The method of claim 41, wherein the method comprises learning, by the transport device, a position of the stand-alone analysis device.

45. The method of claim 41, wherein the method comprises learning, by the stand-alone preparation device, a position of the transport device.
